# EUROPEAN PATENT SPECIFICATION

(11) **EP 3 908 213 B1**
(45) Date of publication and mention of the grant of the patent: **13.03.2024**
(21) Application number: 20702560.2
(22) Date of filing: 07.01.2020
(51) Int. Cl.: A61B 17/221, A61B 17/00, A61B 90/00

(54) **CLOT RETRIEVAL SYSTEM**
GERINNSELBESEITIGUNGSSYSTEM
SYSTÈME DE RETRAIT DE CAILLOT

(30) Priority: 07.01.2019 US 201916241616
(43) Date of publication of application: 17.11.2021
(73) Proprietor: Legacy Ventures LLC, Nashville, Tennessee 37203 (US)
(72) Inventor: ULM, III, Arthur, John, Nashville, TN 37215 (US); DE LA ROSA, Maurice, Santa Rosa, CA 95403 (US)
(74) Representative: Dehns
(86) International application number: PCT/US2020/012554
(87) International publication number: WO 2020/146364

(56) References cited:
- US-A1- 2005 216 031
- US-A1- 2017 105 743
- US-A1- 2018 116 685
- US-A1- 2018 125 513
- US-A1- 2018 221 035

## Description

### BACKGROUND

### TECHNICAL FIELD

The present invention relates to a deployable system for removing a blood clot or other object from a lumen of an animal.

### BACKGROUND OF THE INVENTION

Acute ischemic strokes develop when a blood clot (thrombus) blocks an artery supplying blood to the brain. Needless to say, when a blood clot creates such a blockage, time in removing the clot is critical.

The removal of intracranial obstructions is limited by several factors, such as the distance of the intracranial obstruction from the femoral access site, the tortuosity (twists and turns in the artery as it enters the base of the skull) of the cervical and proximal intracranial vasculature, the small size of the vessels and the extremely thin walls of intracranial vessels, which lack a significant muscular layer. These limitations require a device to be small and flexible enough to navigate through tortuous vessels within a guide catheter and microcatheter, expand after delivery at the site of occlusion and be retrievable into the microcatheter and yet be strong enough to dislodge strongly adherent thrombus from the vessel wall. In addition, the device should distally entrap or encase the thrombus to prevent embolization to other vessels and to completely remove the occlusion. The device should be retrievable without the need for proximal occlusion of the vessel, which carries risk of further ischemia and risk of vessel injury. The device should be simple to use and be capable of multi-use within the same patient treatment. The device should not be abrasive and should not have sharp corners exposed to the endothelial layer of the vessel wall.

Currently available intravascular thrombus and foreign body removal devices lack several of these features. Currently available devices include the MERCI^{™} RETRIEVER clot retriever device marketed by Concentric Medical, Inc. (Mountainview, CA), the PENUMBRA^{™} system marketed by Penumbra Inc. (Alameda, CA) to retrieve clots, and the newer stent retrieval devices TREVO^{™} (Stryker, Kalamazoo, MI) and SOLITAIRE^{™} (eV3 Endovascular Inc., Plymouth, MA, which is a subsidiary of Covidien). All the devices are ineffectual at removing organized hard thrombus that embolize to the brain from the heart and from atherosclerotic proximal vessels. These "hard" thrombi constitute the majority of strokes which are refractory to medical treatment and are therefore referred for removal by mechanical means through an endovascular approach. The MERCI retrieval system is comprised of coiled spring-like metal and associated suture material. The method of use is deployment distal to the thrombus and by withdrawing the device through the thrombus, the thrombus becomes entangled in the coil and mesh and then is retrieved. The MERCI system requires occlusion of the proximal vessel with a balloon catheter and simultaneous aspiration of blood while the thrombus is being removed. Most of the time, the device fails to dislodge the thrombus from the wall of the vessel and often, even when successfully dislodging the thrombus, the thrombus embolizes into another or the same vessel due to the open ended nature of the device.

The next attempt at a thrombus removal system was the PENUMBRA. The PENUMBRA is a suction catheter with a separator that macerates the thrombus which is then removed by suction. The device is ineffective at removing hard, organized thrombus which has embolized from the heart, cholesterol plaque from proximal feeding arteries and other foreign bodies.

The SOLITAIRE and TREVO systems are self-expanding non-detachable stents. The devices are delivered across the thrombus which is then supposed to become entwined in the mesh of the stent and which is then removed in a manner similar to the MERCI system. Again, these devices are ineffectual at treating hard thrombus. In fact, the thrombus is often compressed against the vessel wall by the stent which temporarily opens the vessel by outwardly pressing the clot against the vessel wall. Upon retrieval of the devices, the clot remains or is broken up into several pieces which embolize to vessels further along the vessel.

US 2018/125513 A1 discloses an endoscopic stone-extraction device with a support filament having an end portion having multiple stacked loops, a sheath including a lumen, and a handle including an actuator. The support filament is disposed in the lumen such that the sheath is slideable with respect to the support filament. Movement of the actuator in one direction retracts the sheath and causes the stacked loops to expand outside the lumen in an arc-like shape. Movement of the actuator in another direction advances the sheath and causes the stacked loops to at least partially collapse inside the lumen. U.S. Patent No. 4,890,611 teaches a device for shearing arteriosclerotic deposits. However, the device does not include a clamp for releasably engaging the pull wire.

Thus, there is a need for new, easy-to-use, easy-to-manufacture, safe surgical devices for removing obstructions, such as blood clots, from internal lumens of humans and other animals in a timely manner.

### BRIEF SUMMARY

The system for removing a blood clot from a human blood vessel according to the invention is defined in claim 1. Some embodiments are recited in the dependent claims. The methods disclosed do not form part of the claimed invention.

The present disclosure relates to several systems for removing obstructions and other objects within a blood vessel or other lumen of an animal. The system may be deployed in the lumen from a distal end of a catheter and, in some examples, includes a pull wire having a proximal end and a distal end; a distal body attached to the pull wire, the distal body comprising an interior, an exterior, a proximal end, a distal end, a plurality of proximal memory metal strips located at the proximal end, a proximal hub located in the distal body interior, and a distal hub located distal relative to the proximal hub. The distal body has a relaxed state wherein the distal body has a first height and width and a collapsed state wherein the distal body has a second height and width, the second height less than said first height, the second width less than the first width. The system further includes a catheter having an interior, a proximal end leading to the interior and a distal end leading to the interior, the catheter comprised of a biocompatible material and configured to envelope the distal body when the distal body is in the collapsed state. Each of the proximal memory metal strips has a proximal end and a distal end and preferably, in the relaxed state, each of the proximal ends of the proximal memory metal strips is located proximal relative to the proximal hub. Preferably, in the relaxed state, the proximal ends of the proximal memory metal strips are configured to move towards each other and towards the pull wire when an operator moves the proximal hub distally and closer to the stationary distal hub (i.e., when the operator decreases the distance between the hubs). Preferably, in the relaxed state, the proximal ends of the proximal memory metal strips are configured to move away from each other and away from the pull wire by moving the proximal hub proximally away from the stationary distal hub (i.e., when the operator increases the distance between the hubs).

Optionally, the system further includes a plurality of memory metal connector strips, the plurality of memory metal connector strips each having a proximal end attached to a proximal memory metal strip and a distal end attached to the proximal hub. Optionally, the connector strips are integral with the proximal hub (i.e., optionally, the connector strips and the proximal hub are formed from the same piece of memory metal). Optionally, the proximal hub is a tube having an aperture and the pull wire passes through the aperture. Optionally, in the relaxed state, the proximal hub is slideable along the pull wire (i.e., at least a segment of the pull wire). Optionally, in the relaxed state, the proximal memory metal strips are distributed substantially evenly about a perimeter of the distal body. Optionally, the distal hub is a tube having an aperture. Optionally, the distal hub is attached to the pull wire such that the distal hub is not slideable along the pull wire. Optionally, the distal body further comprises a lead wire extending distally from the distal hub. Optionally, the distal body comprises a basket comprised of a plurality of memory metal strips distal relative to the proximal memory metal strips. Optionally, the distal hub, the proximal hub, and the distal basket are comprised of a nitinol having the same material composition. Optionally, the distal body further comprises an x-ray marker. Optionally, the proximal memory metal strips form a claw, the claw having a closeable proximal end formed by the proximal ends of the proximal memory metal strips. Optionally, between 2 and 4 proximal memory metal strips form the claw. Optionally, the distal body, in the relaxed state, has a tapered shape in which the distal body height and width decrease from the proximal end to the distal end. Optionally, the distal body, in the relaxed state, has a bullet shape. Optionally, the proximal hub and the distal hub are generally cylindrical in shape and each has an outer diameter and an inner diameter that forms the apertures of the proximal and distal hubs, the outer diameters of the proximal and distal hubs are substantially the same size, and the inner diameters of the proximal and distal hubs are substantially the same size. One inch corresponds to 2.54 centimetres.

Optionally, the outer diameters of the proximal and distal hubs are from about 0.011 inches to about 0.054 inches, and the inner diameters of the proximal and distal hubs are from about 0.008 inches to about 0.051 inches. Optionally, the pull wire is generally cylindrical and the diameter of the pull wire is between about 0.008 inches and about 0.051 inches. Optionally, the proximal memory metal strips have a length of between about 10 and about 60 millimeters. Optionally, the first height and first width of the distal body are between about 2 millimeters (mm) and about 6 millimeters. Optionally, the proximal memory metal strips are configured to a separate a clot from a blood vessel wall.

There is also described herein a method of removing an object from an interior lumen of an animal, the lumen having an interior wall forming the lumen. The method may include:
a) providing a system comprising: i) a pull wire having a proximal end and a distal end; ii) a distal body attached to the pull wire, the distal body comprising a proximal end, a distal end, and a claw, the claw comprised of a plurality of memory metal strips, the distal body having a relaxed state wherein the distal body has a first height and width and a collapsed state wherein the distal body has a second height and width, the second height less than said first height, the second width less than said first width; and iii) a catheter having an interior, a proximal end leading to the interior and a distal end leading to the interior, the catheter comprised of a biocompatible material and configured to envelope the distal body when said distal body is in said collapsed state;
b) positioning the system in the lumen;
c) deploying the distal body from the distal end of the catheter;
d) allowing the height and width of said distal body to increase; and
e) moving the memory metal strips towards each other and the pull wire so as to capture the obstruction. Optionally, the claw and the memory metal strips are located at the proximal end of said distal body and the distal body is deployed distal to said object. Optionally, the proximal memory metal strips have a proximal end forming the proximal end of the claw and a distal end, and the method includes moving the proximal ends of the memory metal strips towards each other and the pull wire so as to capture the obstruction. Optionally, the distal body further comprises a proximal hub located in the distal body interior, and a distal hub located distal relative to the proximal hub, each of the memory metal strips has a proximal end and a distal end, each of the proximal ends of the memory metal strips is located proximal relative to the proximal hub, and the proximal ends of the memory metal strips are configured to move towards each other and towards the pull wire by moving the proximal hub distally and closer to the distal hub, and the proximal ends of the memory metal strips are configured to move away from each other and away from the pull wire by moving the proximal hub proximally and away from the distal hub, and the method further comprises moving the proximal hub distally and closer to the distal hub so as to capture the obstruction in the claw. Optionally, the interior lumen is an intracranial artery and the obstruction is a blood clot. Optionally, the method further comprises using the clot to move the proximal hub toward the distal hub and exert tension on the proximal memory metal strips. Optionally, the method further comprises using a tube to move the proximal hub toward the distal hub and exert tension on the proximal memory metal strips.

There is also described herein a method of manufacturing a system for removing objects within an interior lumen of an animal. The method may include:
a) providing a single tube comprised of a memory metal, the single tube having an exterior, a hollow interior, a wall separating the exterior from the hollow interior, a proximal portion comprising an aperture leading to the hollow interior, a distal portion comprising an aperture leading to the hollow interior, and a middle portion between the proximal portion and the distal portion;
b) cutting the wall of the middle portion with a laser;
c) removing the pieces of the middle portion cut by the laser to form a proximal tube, a middle portion comprising a plurality of memory metal strips attached to the proximal tube and a distal tube;
d) altering the shape of the middle portion;
e) allowing the middle portion to expand relative to the distal tube and the proximal tube;
f) cutting the memory metal strips to form a first segment comprising the proximal tube and a proximal segment of the memory metal strips, and a second segment comprising the distal tube and a distal segment of the memory metal strips; and
g) joining the proximal segments to the distal segments such that the distal segments form the proximal end of a distal body, such that the proximal tube is located inside an interior of said distal body, and such that the proximal tube is located distal relative to the proximal end.

Optionally, the method further includes placing a pull wire through the proximal tube such that the proximal tube is slideable along at least a segment of the pull wire. Optionally, the method further includes attaching the pull wire to the distal tube. Optionally, the step of joining the proximal segments to the distal segments comprises welding the proximal segments to the distal segments. Optionally, after the step of joining the proximal segments to the distal segments, the proximal end forms a claw comprised of between 2 and 4 memory metal strips, the claw memory metal strips configured to move towards each by moving said proximal tube distally and closer to the distal tube, and the claw memory metal strips configured to move away from each other by moving the proximal tube proximally and away from said distal tube. Optionally, the method further includes not altering the shape of the proximal and distal portions while altering the shape of the middle portion. Optionally, the method further includes cooling the proximal portion, the middle portion, and the distal portion after step D) and, after cooling, the proximal and distal portions have substantially the same size as the proximal and distal portions had prior to step A). Optionally, the method of allowing said middle portion to expand comprises heating the middle portion. Optionally, the method of altering the shape of the middle portion comprises using a mandrel. Optionally, the mandrel is tapered. Optionally, the proximal portion and the distal portion are not cut by the laser. Optionally, prior to cutting the memory metal tube, the memory metal tube has an outer diameter that is from about 0.011 inches to about 0.054 inches and an inner diameter that is from about 0.008 inches to about 0.051 inches.

The present disclosure provides a system for removing objects from an interior lumen of an animal that includes: a pull wire having a proximal end and a distal end;
a distal body attached to the pull wire, the distal body comprising an interior, a proximal end, a distal end, a distal body length extending from the proximal end to the distal end, a proximal hub (preferably in the form of a tube) forming the proximal end of the distal body, a basket comprised of a plurality of cells formed by a plurality of basket strips, a plurality of proximal strips, and, optionally a distal hub (preferably in the form of a tube) forming a distal end of the basket, the basket comprising a basket interior, each proximal strip having a proximal end attached to the proximal hub, and a distal end attached to a cell, the distal body having a relaxed state wherein the distal body has a first height and a first width, and a collapsed state wherein the distal body has a second height and a second width, the second height less than the first height, the second width less than the first width; and
a catheter having an interior, a proximal end leading to the interior and a distal end leading to the interior, the catheter comprised of a biocompatible material and configured to envelope the distal body when the distal body is in the collapsed state,
wherein, in the relaxed state, the basket comprises a first pair of distal crowns not attached to another cell of the basket and pointing generally in the distal direction, the first pair of distal crowns located approximately the same distance from the proximal hub and approximately 180 degrees relative to each other (e.g., between about 150 degrees and about 180 degrees relative to each other), and further wherein the basket further comprises a second pair of distal crowns not attached to another cell of the basket and pointing generally in the distal direction, the second pair of distal crowns located distally relative to, and approximately 90 degrees relative to, the first pair of distal crowns (e.g., each distal crown of the second pair of distal crowns is located approximately 60 degrees to 90 degrees relative to a distal crown of the first pair of distal crowns), the distal crowns in the second pair of distal crowns located approximately the same distance from the proximal hub and further wherein each of the distal crowns in the first and second pair of distal crowns comprises an x-ray marker, the x-ray maker more visible under x- ray as compared to the basket strips when the distal body is located in a cranial blood vessel inside the body of a human and the x-ray is taken from outside the human's body. When it is said that the first pair of distal crowns are located approximately the same distance from the proximal hub, it will be understood that if one of the first pair of distal crowns is located X distance from the proximal hub, the other of the first pair of distal crowns is located X distance plus or minus (+/-) 3 mm from the proximal hub, more preferably X distance plus or minus (+/-) 0.5 mm from the proximal hub. Similarly, when it is said that the second pair of distal crowns are located approximately the same distance from the proximal hub, it will be understood that if one of the second pair of distal crowns is located Y distance from the proximal hub, the other of the first pair of distal crowns is located Y distance plus or minus (+/-) 3 mm from the proximal hub, more preferably Y distance plus or minus (+/-) 0.5 mm from the proximal hub. Optionally, instead of a distal hub, the basket includes an open distal end.

Optionally, the x-ray markers are comprised of a material different than the material forming the basket strips. Optionally, in the relaxed state, the basket interior is substantially hollow. Optionally, in the relaxed state, the distal body does not have another x-ray marker that is located approximately the same distance from the proximal hub as the first pair of x-ray markers and the distal body does not have another x-ray marker that is located approximately the same distance from the proximal hub as the second pair of x-ray markers. In other words, the first and second pair of x-ray markers are the only markers their respective distances from the proximal hub. Optionally, each distal crown in the first and second pair of distal crowns forms part of an enlarged cell and further wherein the surface area of each enlarged cell in the relaxed state is greater than the surface area of each of the other individual cells of the basket and further wherein the enlarged cells are configured to allow a thrombus to pass therethrough and into the basket interior. Optionally, in the relaxed state, the distal body does not have another free distal- pointing crown that is located approximately the same distance from the proximal hub as the first pair of distal crowns and the distal body does not have another free distal-pointing crown that is located approximately the same distance from the proximal hub as the second pair of distal crowns. Optionally, the basket strips are comprised of a memory metal. Optionally, each of the distal crowns in the first pair and second pair of distal crowns curve radially inward toward the basket interior in the relaxed state, wherein the distal crowns of the first pair of distal crowns are configured to contact each other when an exterior, external compressive force (such as a thrombus) is exerted on a distal crown of the first pair of distal crowns when the distal body is in the relaxed state, and further wherein the distal crowns of the second pair of distal crowns are configured to contact each other when an exterior, external compressive force (such as a thrombus) is exerted on a distal crown of the second pair of distal crowns when the distal body is in the relaxed state. Optionally, the proximal hub is located approximately in the center of the first height and first width in the relaxed state. For example, preferably the proximal hub is located within 0.5 mm of the center of first width and the first height. Optionally, the catheter is comprised of a polymeric material (i.e., one or more polymeric materials such as silicone, PVC, latex rubber or braided nylon). Optionally, the pull wire is comprised of a biocompatible metallic material (e.g., a biocompatible metal or a biocompatible metal alloy). Optionally, the proximal end of a first proximal strip is located at least about 65 degrees (e.g., between about 65 and about 180 degrees) relative to the distal end of the first proximal strip, wherein the proximal end of a second proximal strip is located at least about 65 degrees (e.g., between about 65 and about 180 degrees) relative to the distal end of the second proximal strip, and further wherein the first and second proximal strips intersect adjacent and distal to the proximal hub (e.g., within about 0 and about 4 mm of the proximal hub). Optionally, each distal crown forms part of a cell that further comprises a proximal crown pointing generally in the proximal direction and connected to a memory metal strip (e.g., a proximal strip comprised of a memory metal or a basket strip comprised of a memory metal). In other words, the proximal crowns are not free. Optionally, the basket, the proximal hub and the proximal strips are comprised of a memory metal, wherein the proximal hub comprises a proximal end and a distal end, and further wherein the proximal strips are integral with the distal end of the proximal hub. Optionally, the length of the distal body from the proximal hub to the distal hub (not including any lead wire) is from about 20 mm to about 65 mm. Optionally, the system is used in a method of removing a blood clot from a blood vessel of an animal the method comprising the steps of
a) providing the system;
b) positioning the system in the lumen;
c) deploying the distal body from the distal end of the catheter;
d) allowing the height and width of the distal body to increase;
e) irradiating the distal body with x-rays;
f) moving the clot into the distal basket interior; and
g) moving the distal body proximally out of the blood vessel.

Optionally, the method further comprises irradiating the distal body with x-rays at at least two different angles. Optionally, at least one x-ray marker attached to the distal crowns is distal to the clot when the distal body is deployed from the distal end of the catheter. Optionally, the method further comprises applying contrast dye proximally and distally to the clot. Optionally, the method further comprises providing a suction catheter having a proximal end and a distal end, and attaching the distal end of the suction catheter to the clot by applying suction to the suction catheter. Optionally, the method further comprises aspirating by hand a pre-determined volume of fluid from the suction catheter using a syringe and then locking the syringe at the predetermined volume. Optionally, the method further comprises delivering the suction catheter adjacent to the clot by advancing the catheter over the pull wire.

The system may include:
a pull wire having a proximal end and a distal end;
a distal body attached to the pull wire, the distal body comprising an interior, a proximal end, a distal end, a distal body length extending from the proximal end to the distal end, a proximal hub (preferably in the form of a tube) forming the proximal end of the distal body, a basket comprised of a plurality of cells formed by a plurality of basket strips, a plurality of proximal strips, and optionally a distal hub (preferably in the form of a tube) forming a distal end of the basket, the basket comprising a basket interior, each proximal strip having a proximal end attached to the proximal hub, and a distal end attached to a cell, the distal body having a relaxed state wherein the distal body has a first height and a first width, and a collapsed state wherein the distal body has a second height and a second width, the second height less than the first height, the second width less than the first width; and
a catheter having an interior, a proximal end leading to the interior and a distal end leading to the interior, the catheter comprised of a biocompatible material and configured to envelope the distal body when the distal body is in the collapsed state,
wherein, in the relaxed state, the basket comprises a first pair of distal crowns not attached to another cell of the basket and pointing generally in the distal direction, the first pair of distal crowns located approximately the same distance from the proximal hub and approximately 180 degrees relative to each other (e.g., between about 150 degrees and about 180 degrees relative to each other), and further wherein the basket further comprises a second pair of distal crowns not attached to another cell of the basket and pointing generally in the distal direction, the second pair of distal crowns located distally relative to, and approximately 90 degrees relative to, the first pair of distal crowns (e.g., each distal crown of the second pair of distal crowns is located approximately 60 degrees to 90 degrees relative to a distal crown of the first pair of distal crowns), the distal crowns in the second pair of distal crowns located approximately the same distance from the proximal hub, wherein each distal crown of the first and second pair of distal crowns form a cell, each cell further comprising a proximal crown pointing generally in the proximal direction and connected to a memory metal strip, wherein each of the distal crowns in the first pair and second pair of distal crowns curve radially inward toward the basket interior in the relaxed state, wherein the distal crowns of the first pair of distal crowns are configured to contact each other when an exterior, external compressive force (e.g., a thrombus) is exerted on a distal crown of the first pair of distal crowns when the distal body is in the relaxed state, and further wherein the distal crowns of the second pair of distal crowns are configured to contact each other when an exterior, external compressive force (e.g., a thrombus) is exerted on a distal crown of the second pair of distal crowns when the distal body is in the relaxed state. When it is said that a proximal crown pointing generally in the proximal direction and is connected to a memory metal strip, it is meant that the proximal crown is either connected to a basket strip or a proximal strip comprised of a memory metal (e.g., nitinol). When it is said that the first pair of distal crowns are located approximately the same distance from the proximal hub, it will be understood that if one of the first pair of distal crowns is located X distance from the proximal hub, the other of the first pair of distal crowns is located X distance plus or minus (+/-) 0.5 mm from the proximal hub. Similarly, when it is said that the second pair of distal crowns are located approximately the same distance from the proximal hub, it will be understood that if one of the second pair of distal crowns is located Y distance from the proximal hub, the other of the first pair of distal crowns is located Y distance plus or minus (+/-) 0.5 mm from the proximal hub. Optionally, instead of a distal hub, the basket includes an open distal end.

Optionally, the proximal hub is located approximately in the center of the first height and first width in the relaxed state. For example, preferably the proximal hub is located within 0.5 mm of the center of first width and the first height. Optionally, the catheter is comprised of a polymeric material (i.e., one or more polymeric materials such as silicone, PVC, latex rubber or braided nylon). Optionally, the pull wire is comprised of a biocompatible metallic material (e.g., a biocompatible metal or a biocompatible metal alloy). Optionally, in the relaxed state, the basket interior is substantially hollow. Optionally, the proximal end of a first proximal strip is located at least about 65 degrees (e.g., between about 65 and about 180 degrees) relative to the distal end of the first proximal strip, wherein the proximal end of a second proximal strip is located at least about 65 degrees (e.g., between about 65 and about 180 degrees) relative to the distal end of the second proximal strip, and further wherein the first and second proximal strips intersect adjacent and distal to the proximal hub (e.g., within about 0 mm and about 4 mm of the proximal hub). Optionally, each distal crown in the first and second pair of distal crowns forms part of an enlarged cell and further wherein the surface area of each enlarged cell in the relaxed state is at least twice as large as the surface area of each other individual cell of the basket and further wherein the enlarged cells are configured to allow a thrombus to pass therethrough and into the basket interior. Optionally, the pull wire is attached to the proximal hub. Optionally, the basket, the proximal hub and the proximal strips are comprised of a memory metal, wherein the proximal hub comprises a proximal end and a distal end, and further wherein the proximal strips are integral with the distal end of the proximal hub. Optionally, the distal body further comprises a lead wire extending distally from the distal hub, the lead wire having a length of from about 3 mm to about 10 mm. Optionally, the distal hub, the proximal hub, and the basket are comprised of a nitinol having the same material composition and further wherein the proximal and the distal hubs are tubular and generally cylindrical in shape and each has an outer diameter and an inner diameter, the inner diameter forming apertures of the proximal and distal hubs and further wherein the outer diameters of the proximal and distal hubs are substantially the same size and further wherein the inner diameters of the proximal and distal hubs are substantially the same size. Optionally, the length of the distal body from the proximal hub to the distal hub (not including any lead wire) is from about 20 mm to about 65 mm.

Optionally, the system is used in a method of removing a blood clot from a blood vessel of an animal the method comprising the steps of:
a) providing the system;
b) positioning the system in the lumen;
c) deploying the distal body from the distal end of the catheter;
d) allowing the height and width of the distal body to increase;
e) irradiating the distal body with x-rays;
f) moving the clot into the distal basket interior; and
g) moving the distal body proximally out of the blood vessel.

Optionally, the method further comprises irradiating the distal body with x-rays at at least two different angles.

The present invention provides a system for removing a blood clot from a human blood vessel. The system includes a proximal outer tube comprising a proximal outer tube exterior comprising a slot, a proximal outer tube interior that may be hollow, a proximal outer tube proximal end that may be open, a proximal outer tube distal end that may be open, and a proximal outer tube length extending from the proximal outer tube proximal end to the proximal outer tube distal end. Optionally, the slot comprises a slot proximal end, a slot distal end, and a slot length extending from the slot proximal end to the slot distal end and parallel to the proximal outer tube length. The system further includes a distal outer tube comprising a distal outer tube exterior, a distal outer tube interior that may be hollow, a distal outer tube proximal end that may be open and located distal to the proximal outer tube distal end, a distal outer tube distal end that may be open, a distal outer tube length extending from the distal outer tube proximal end to the distal outer tube distal end. The distal outer tube is not attached to the proximal outer tube. The system further includes an inner tube extending from the proximal outer tube interior to the distal outer tube interior. The inner tube comprises an inner tube interior that may be hollow, an inner tube exterior comprising a fin (which is positioned in the proximal outer tube slot), an inner tube proximal end that may be open, an inner tube distal end that may be open and an inner tube length extending from the inner tube proximal end to the inner tube distal end. The system further includes a compressible spring having a spring proximal end, a spring distal end, and a spring length extending from the spring proximal end to the spring distal end. The system further includes a pull wire extending through at least the distal outer tube tube distal end (and optionally, the distal outer tube proximal end, the inner tube distal end, the inner tube interior, the inner tube distal end, the proximal outer tube proximal end, the proximal outer tube interior and/or the proximal outer tube distal end), the pull wire having a pull wire proximal end (optionally located proximal to the proximal outer tube proximal end) and a pull wire distal end located distal to the distal outer tube distal end. The system further includes a distal body located distal to the distal outer tube and comprising a distal body interior, a distal body perimeter, a distal body proximal end connected to the pull wire, a distal body distal end, a distal body length extending from the distal body proximal end to the distal body distal end, and a distal body height and width perpendicular to the distal body length. The distal body comprises a framework formed by a plurality of memory metal strips. The distal body has a relaxed state wherein the distal body has a first height and a first width, and a collapsed state wherein the distal body has a second height and a second width, the second height of the distal body less than the first height of the distal body, the second width of the distal body less than the first width of the distal body. At least one of the distal outer tube and the inner tube (preferably, the inner tube distal end) comprises a clamp having a closed position in which the clamp closes around the pull wire and an open position in which the clamp is open with respect to the pull wire. When the clamp is in the open and closed position, the inner tube is attached to the distal outer tube and moves along with the distal outer tube in the lengthwise direction. At least when the clamp is in the closed position, the proximal outer tube is configured to move proximally for a pre-determined distance relative to the inner tube, distal outer tube, the pull wire and the distal body in the lengthwise direction. At least when the clamp is in the closed position, moving the proximal outer tube proximally relative to the distal outer tube, the inner tube, the pull wire and the distal body causes the proximal outer tube slot to move proximally relative to the fin and causes the spring to move from a relaxed length to a compressed length. Optionally, when the clamp is in the closed position, rotating the proximal outer tube about the proximal outer tube length in a clockwise and counterclockwise direction is configured to rotate the distal outer tube, the pull wire and the distal body. Optionally, when the clamp is in the open position, rotating the proximal outer tube about the proximal outer tube length in a clockwise and counterclockwise direction is configured to rotate the distal outer tube but neither the pull wire nor the distal body. Optionally, when the clamp is in the open position, the proximal outer tube is configured to move proximally for a pre-determined distance relative to at least the inner tube and the distal outer tube in the lengthwise direction. Optionally, the system comprises a relaxed position in which the proximal outer tube distal end abuts against the distal outer tube proximal end and in which the spring is the relaxed length and a second position in which the distal end of the proximal outer tube does not abut the proximal end of the distal outer tube and in which the spring is the compressed length. Optionally, the proximal outer tube exterior comprises indicia adjacent to the slot. Optionally, the proximal outer tube exterior comprises two handle flanges comprising grooves located on opposite sides of the proximal outer tube exterior. Optionally, the handle flanges have a proximal end and a distal end and further wherein the handle flanges extend laterally from the proximal outer tube to a greater extent at the proximal end of the handle flanges as compared to the distal end of the handle flanges. Optionally, the inner tube comprises an inner tube height and an inner tube width, and further wherein the inner tube distal end comprises a tapered portion in which the inner tube height and width decrease at the inner tube distal end. Optionally, the distal outer tube interior and the inner tube comprise mating threads, wherein moving the inner tube threads along the distal outer tube interior threads in a first direction moves the clamp to the closed position and further wherein moving the inner tube threads along the distal outer tube interior threads in a second direction moves the clamp to the open position. Optionally, the slot proximal end is at the proximal end of the proximal outer tube. Optionally, the inner tube is comprised of a rod and a collar enveloping the rod and the collar comprises the fin. Optionally, the slot length is less than the proximal outer tube length.

The present disclosure describes a method of removing a blood clot from a blood vessel of a human the method comprising the steps of a) providing the system; b) positioning a catheter comprising the distal body in the blood vessel; c) deploying the distal body from the catheter and allowing the height and width of the distal body to increase; d) capturing the blood clot with the distal body; and e) moving the distal body proximally out of the blood vessel by grasping and pulling proximally on the proximal outer tube. Optionally, the system further comprises the steps of moving the clamp from the open position to the closed position rotating the distal body in the blood vessel by rotating the proximal outer tube at least before step d).

Furthermore, the present disclosure describes a method of removing a blood clot from a blood vessel of a human the method comprising the steps of a) providing a gauge system that may include i) a pull wire having a proximal end and a distal end; ii) a distal
body that may comprises a distal body interior, a distal body perimeter, a distal body proximal end connected to the pull wire, a distal body distal end, a distal body length extending from the distal body proximal end to the distal body distal end, and a distal body height and width perpendicular to the distal body length, the distal body may comprise a framework formed by a plurality of memory metal strips, the distal body may have a relaxed state wherein the distal body has a first height and a first width, and a collapsed state wherein the distal body has a second height and a second width, the second height of the distal body less than the first height of the distal body, the second width of the distal body less than the first width of the distal body; and iii) a strain gauge that may be configured to measure tension placed on the pull wire, the strain gauge may be comprised of a tube, indicia and a clamp configured to releasably engage the pull wire; b) positioning a catheter comprising the distal body in the blood vessel; c) deploying the distal body from the catheter and allowing the height and width of the distal body to increase; d) capturing the blood clot with the distal body; and e) moving the distal body proximally out of the blood vessel by pulling proximally on the strain gauge.

The two above aforementioned methods may utilize any feature mentioned above with respect to the systems that include a distal outer tube, a proximal outer tube and an inner tube, including movement and rotation of the respective parts.

### BRIEF DESCRIPTION OF THE DRAWINGS

FIG. 1A illustrates a side, elevation view of a memory metal tube prior to being cut by a laser.
FIG. IB illustrates a side, elevation view of the memory metal tube of FIG. 1A being cut by a laser.
FIG. 2A illustrates a side, elevation view of the memory metal tube of FIG. IB after being cut by a laser; in FIG. 2A, the tube is shown as though it were flat for purposes of illustrating the cut pattern only.
FIG. 2B illustrates a side, perspective view of the memory metal tube of FIG. IB after being cut by a laser.
FIG. 2C illustrates another side, perspective view of the memory metal tube of FIG. IB after being cut by a laser; in FIG. 2C, the tube is rotated as compared to FIG. 2B.
FIGs. 3A-3H illustrate a method of manufacturing a distal body using the laser cut memory metal tube of FIGs. 1 and 2; in FIGs. 3A-3H, the basket portion of the distal body is not shown for simplicity of illustration.
FIGs. 4A-4D illustrate the welding steps of the method of manufacturing shown in FIG. 3; in FIGs. 4A-4D, the basket portion of the distal body is not shown for simplicity of illustration.
FIGs. 5 and 6 illustrate different locations that connector strips may be welded to the proximal memory metal strips.
FIG. 7 illustrates a side, elevation view of a catheter and the distal body of FIG. 6.
FIG. 8 illustrates a side, elevation view of a deployable system being used to capture a blood clot; in FIG. 8, the basket portion of the distal body is not shown for simplicity of illustration.
FIG. 9 illustrates a side, elevation view of a claw being closed by a claw actuator tube; in FIG. 9, the basket portion of the distal body is not shown for simplicity of illustration.
FIG. 10 illustrates a side, elevation view of a deployable system being used to capture a blood clot; in FIG. 10, the basket portion of the distal body is not shown for simplicity of illustration.
FIG. 11 illustrates a first, perspective view of a distal body ; the distal body is in what is referred to herein as "Orientation 1".
FIG. 12A illustrates a second, perspective view of the distal body of FIG. 11; the distal body is in what is referred to herein as "Orientation 2".
FIG. 12B illustrates a proximal, elevation view of the proximal strips of the distal body of FIG. 11.
FIG. 13 illustrates a close-up, perspective view of two unattached distal-pointing crowns of the distal body of FIG. 11.
FIG. 14A illustrates a native memory metal tube used to manufacture the distal body of FIG. 11; the native tube has been rolled out flat and the lines in the tube indicate where the tube has been cut by a laser.
FIG. 14B illustrates a first, perspective view of the distal body manufactured from the native tube of FIG. 14A; the distal body is in Orientation 1.
FIG. 14C illustrates a second, perspective view of the distal body manufactured from the native tube of FIG. 14A; the distal body is in Orientation 2.
FIGs. 15A-G illustrate stepwise use of the distal body of FIG. 11 in retrieving a soft clot; the distal body is in Orientation 1.
FIGs. 16A-H illustrate stepwise use of the distal body of FIG. 11 in retrieving a hard clot; the distal body is in Orientation 1.
FIGs. 17A-G illustrate stepwise use of the distal body of FIG. 11 in retrieving a soft clot; the distal body is in Orientation 2.
FIGs. 18A-G illustrate stepwise use of the distal body of FIG. 11 in retrieving a hard clot; the distal body is in Orientation 2.
FIGs. 19A-N illustrate stepwise use of the distal body of FIG. 11 in retrieving a deformable, cohesive adherent clot; the distal body is in Orientation 2.
FIG. 20A illustrates a view of a native memory metal tube used to manufacture a distal body ; the native tube has been rolled out flat, the lines in the tube indicate where the tube has been cut by a laser, and the distal body of FIGs. 20A-20C is slightly shorter than the distal body of FIGs. 11-19 and is meant for use in tortuous blood vessels.
FIG. 20B illustrates a first, perspective view of the distal body manufactured from the native tube of FIG. 20A; the distal body is in Orientation 1.
FIG. 20C illustrates a second, perspective view of the distal body manufactured from the native tube of FIG. 20A; the distal body is in Orientation 2.
FIG. 21 shows a perspective view of a clot retrieval system that includes the distal body of FIGs. 20B-C being delivered in a blood vessel using a delivery catheter.
FIG. 22 shows a perspective view of the distal body of FIG. 21, after deployment of the distal body and retraction of the delivery catheter, in a blood vessel.
FIG. 23 shows a perspective view of the distal body of FIG. 21; as compared to FIG. 22, the distal body has been moved proximally and tension has been exerted on the pull wire.
FIG. 24 shows a perspective view of a suction catheter that is being delivered over the pull wire of the system of FIG. 21.
FIG. 25 shows a perspective view of the distal end of the suction catheter of FIG. 24 being pushed into a clot; a syringe is sucking the clot to the suction catheter because the user has pulled back on the lever of the syringe.
FIG. 26 shows a perspective view of the distal end of the suction catheter of FIG. 24 being pushed into a clot; in FIG. 26, the user has locked the syringe lever at the desired volume.
FIG. 27 shows a perspective view of the system of FIG. 24; in FIG. 27, the suction catheter has partially sucked the distal body and clot into the suction catheter.
FIG. 28 shows a perspective view of the system of FIG. 24; in FIG. 28, the suction catheter has completely sucked the distal body and clot into the suction catheter.
FIG. 29 shows a perspective view of the system of FIG. 24; the system, and captured clot, is being removed proximally from the vessel.
FIG. 30 illustrates a right side perspective view of a mandrel used to prepare unattached distal-pointing crowns that curve radially toward the basket interior.
FIG. 31 illustrates a right side elevation view of the mandrel of FIG. 30.
FIG. 32A illustrates a side perspective view of a strain gauge and pull wire of a clot retrieval system of another embodiment of the present invention; in FIG. 32A, the proximal outer tube distal end abuts the distal outer tube proximal end.
FIG. 32B illustrates a side perspective view of the strain gauge and pull wire of FIG. 32A with portions of the distal outer tube and proximal outer tube removed to better show the internal components.
FIG. 33A illustrates a side perspective view of the strain gauge and pull wire of FIG. 32A; in FIG. 33A, the proximal outer tube distal end does not abut the distal outer tube proximal end.
FIG. 33B illustrates a side perspective view of the strain gauge and pull wire of FIG. 33A with portions of the distal outer tube and proximal outer tube removed to better show the internal components.
FIG. 34A illustrates a side perspective view of the strain gauge and pull wire of FIG. 32A; in FIG. 34A, the proximal outer tube distal end does not abut the distal outer tube proximal end, and as compared to FIG. 33A, the fin in FIG. 34A is located more distally relative to the proximal outer tube proximal end, indicating more tension is on the pull wire.
FIG. 34B illustrates a side perspective view of the strain gauge and pull wire of FIG. 34A with portions of the distal outer tube and proximal outer tube removed to better show the internal components.
FIG. 35A illustrates a side perspective view of the strain gauge and pull wire of FIG. 32A; in FIG. 35 A, the proximal outer tube distal end does not abut the distal outer tube proximal end, and as compared to FIGs. 33A and 34A, the fin in FIG. 35A is located more distally relative to the proximal outer tube proximal end, indicating more tension is on the pull wire.
FIG. 35B illustrates a side perspective view of the strain gauge and pull wire of FIG. 35 A with portions of the distal outer tube and proximal outer tube removed to better show the internal components.
FIG. 36 illustrates a partially exploded side perspective view of the strain gauge and pull wire of FIG. 32A; in FIG. 36, the inner tube is exploded from the distal outer tube.
FIG. 37 illustrates a partially exploded side perspective view of the strain gauge and pull wire of FIG. 36; like FIG. 36, in FIG. 37, the inner tube is exploded from the distal outer tube.
FIG. 38 illustrates a top plan view of the strain gauge and pull wire of FIG. 32A; in FIG. 38, the proximal outer tube distal end abuts the distal outer tube proximal end.
FIG. 39 illustrates a top sectional view of the strain gauge and pull wire of FIG. 38 taken along line 39-39 of FIG. 38.
FIG. 40 illustrates a top sectional view of the boxed area labelled 40 in FIG. 39.
FIG. 41 illustrates a top sectional view of the boxed area labelled 41 in FIG. 39.
FIG. 42 illustrates a top plan view of the strain gauge and pull wire of FIG. 32A attached to a distal body; in FIG. 42, the proximal outer tube distal end does not abut the distal outer tube proximal end.
FIG. 43 illustrates a top sectional view of the strain gauge and pull wire of FIG. 32A; in FIG. 43, the proximal outer tube distal end does not abut the distal outer tube proximal end.
FIG. 44 illustrates a fully exploded side perspective view of the strain gauge and pull wire of FIG. 3 2 A.
FIG. 45 illustrates a side perspective view of the proximal outer tube of the strain gauge of FIG. 32A.

### DETAILED DESCRIPTION

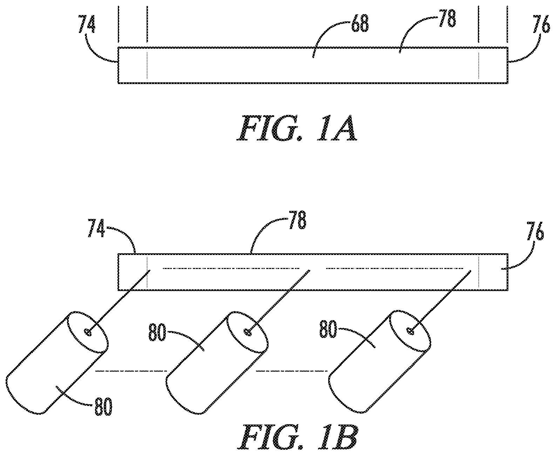

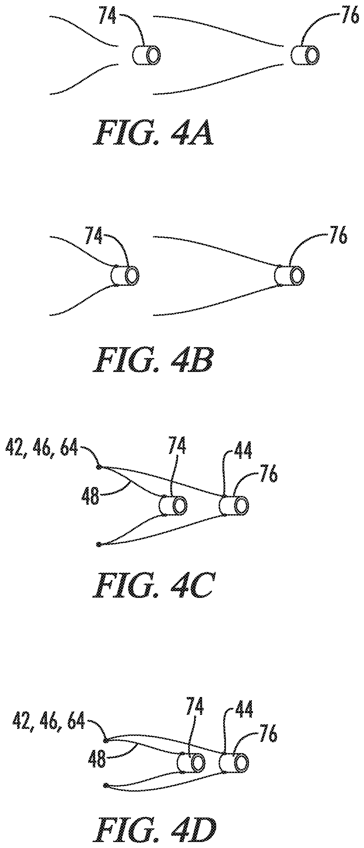

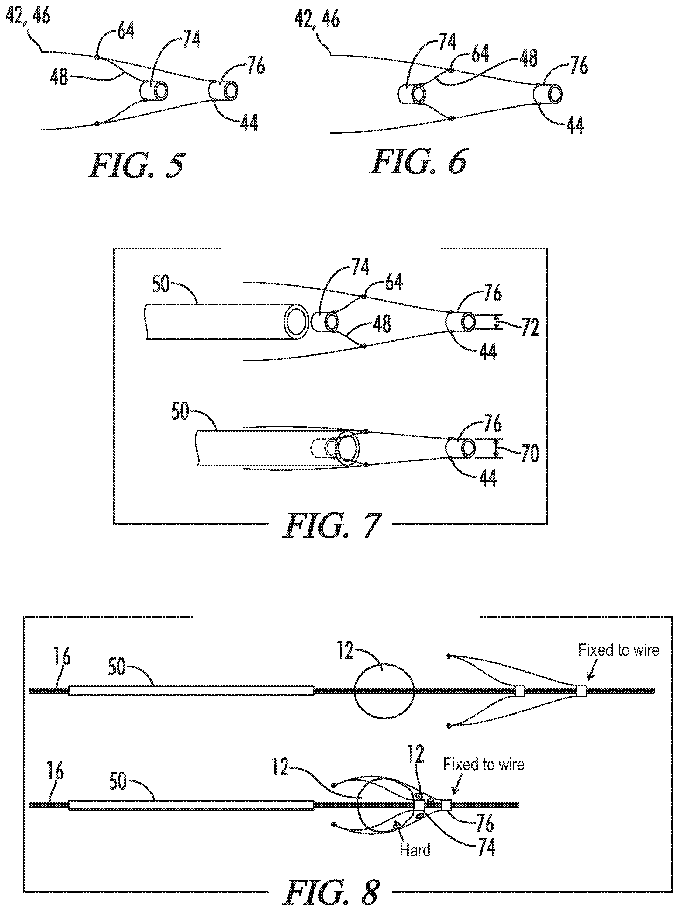

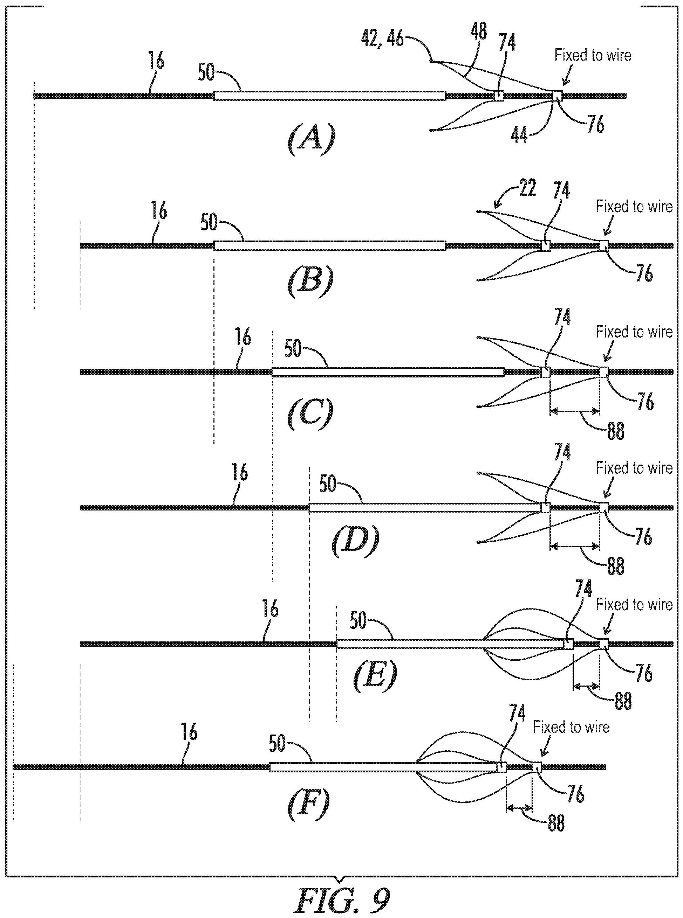

With reference to FIGs. 1-10, the present disclosure provides a deployable system, generally designated by the numeral 10, for removing an obstruction such as a blood clot 12 or other object from a blood vessel 14 or other interior lumen of an animal. In addition to a blood clot 12, the obstruction may be, for example, extruded coils during aneurysm treatment, intravascular embolic material such as onyx or other obstructions requiring mechanical intravascular removal from small distal vessels. In the drawings, not all reference numbers are included in each drawing for the sake of clarity.

Referring further to FIGs. 1-10, the deployable system 10 includes a pull wire 16 that has a proximal end (not shown) and a distal end 20. Optionally, the diameter of the pull wire is between about 0.008 inches and about 0.051 inches. Preferably, the pull wire 16 is comprised of a biocompatible metallic material.

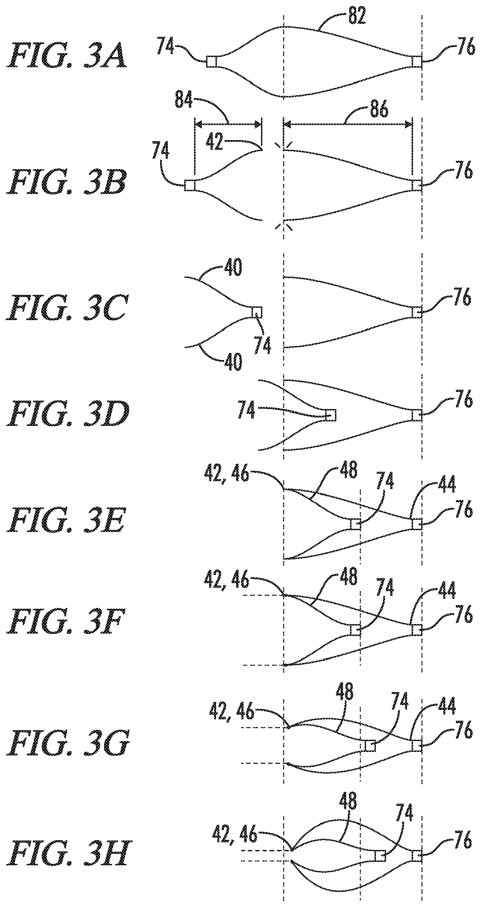

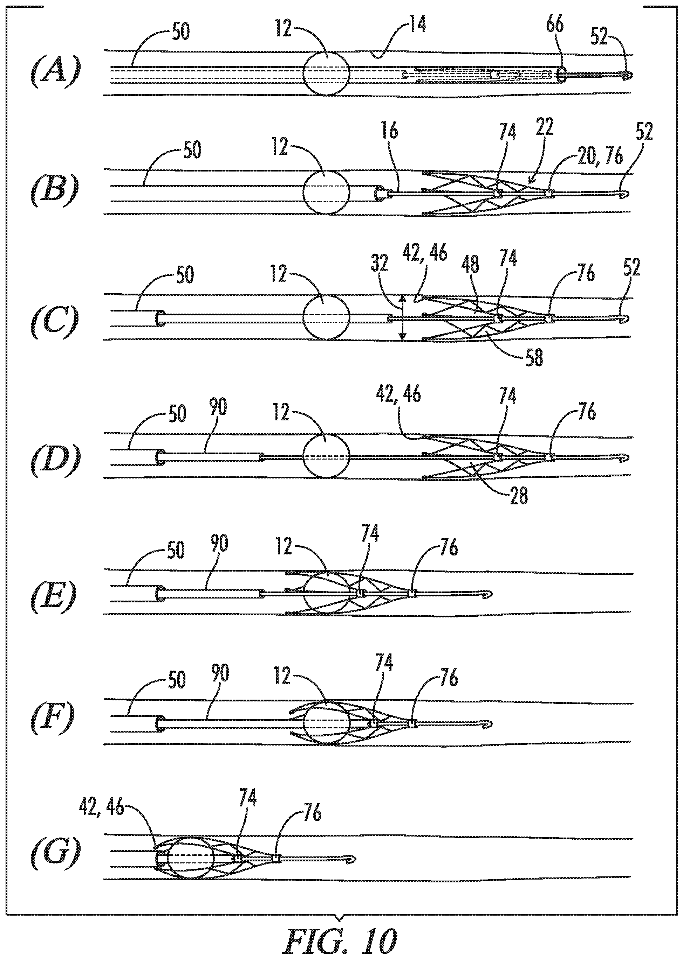

The system 10 further includes a distal body 22, which is attached to the pull wire 16. The distal body 22 has a proximal end 24, a distal end 26, an interior 28, and an exterior 30. The distal body 22 has a collapsed state, wherein the distal body 22 has a first height and width and is configured to fit into a catheter 50 (see FIG. 10A), and a relaxed state wherein the distal body 22 has a different height 32 and width and is configured to expand to about the height and width of a human blood vessel 14 when the distal body 22 is deployed from the catheter 50 (see FIGS. 10B-G). The distal body 22 further includes a proximal hub 74 and a distal hub 76 that is located distal relative to the proximal hub 74. In some examples, the distal body 22 includes a plurality of strips 40 comprised of a memory metal (e.g., a memory metal alloy such as nitinol) that form the proximal end 24 of the distal body 22. Optionally, the proximal memory metal strips 40 each have a distal end 44 and a proximal end 42 that forms an openable and closeable claw 46. Optionally, the proximal memory metal strips 40 are attached to the proximal hub 74 through connector memory metal strips 48. In such examples, the proximal hub 74 may be slideable along at least a segment of the pull wire 16, in contrast to the distal hub 76, which is optionally fixed to the pull wire 16 and not slideable along the pull wire 16. Moving the proximal hub 74 distally and closer to the distal hub 76 (i.e., shortening the distance 88 between the proximal hub 74 and distal hub 76 by moving the proximal hub 74 distally while keeping the distal hub 76 stationary) exerts tension on the connector memory metal strips 48 and, in turn, the proximal memory metal strips 40. This tension, in turn, causes the proximal ends 42 of the proximal memory metal strips 40 to move radially toward each other and the pull wire 16. As the proximal ends 42 of the proximal memory metal strips 40 move radially toward each other and the pull wire 16, the claw 46 (formed by the proximal memory metal strips 40) is brought from the open position to at least a partially closed position, which in turn, separates the obstruction 12 from the wall of the human lumen 14 and captures the obstruction 12. See FIG. 3H, FIG. 8, FIG. 9F, and FIG. 10F and 10G. Conversely, preferably, movement of the proximal hub 74 proximally and away from the distal hub 76 (i.e., increasing the distance 88 between the hubs 74 and 76) releases the tension in the proximal memory metal strips 40, which in turn, causes the proximal ends 42 of the proximal memory metal strips 40 to move away from each other and the pull wire 16, opening the claw 46. The claw 46 and proximal hub 74 form several functions. First, as described, closing of the claw 46 captures the obstruction 12. Second, closing the claw 46 retracts the claw 46 from the wall of the lumen 14 so that the claw 46 does not scrape against (and damage) the lumen wall while capturing the obstruction 12. Third, closing the claw 46 reduces the height and width of the distal body 22, which allows the distal body 22 to be re-sheathed in the catheter 50, which may be desired, for example, if the operator seeks to re-deploy the distal body 22 in another location in the body (which may be the case if the operator originally deploys the distal body **22** in the wrong location in the lumen **14**). For purposes of the present invention, "closing the claw" embraces both partially closing the claw **46** (where the proximal ends **42** of the proximal memory metal strips **40** do not contact the pull wire **16**) and fully closing the claw **46** (where the proximal ends **42** contact the pull wire **16**).

The claw **46** may be comprised of any number of proximal memory metal strips 40. Preferably, however, between 2 and 4 proximal memory metal strips **40** comprise the claw **46** (it being understood that the connector strips **48,** if present, merely serve to tether the claw **46** to the proximal hub **74**). Preferably, the proximal memory metal strips **40** have a length of between about 10 and about 60 millimeters. The proximal memory metal strips **40** can be thought of as arms of the claw **46.**

In some examples, the connector strips **48** are integral with the proximal hub **74** (i.e., formed from the same piece of memory metal). In other examples, the proximal hub **74** may be welded to the connector strips **48.** Optionally, in the relaxed state, the proximal memory metal strips **42** are distributed substantially evenly about a perimeter of the distal body **22.**

Optionally, the distal body **22** includes a lead wire **52** extending distally from the distal body **22.** Optionally, the lead wire **52** extends distally from the distal hub **76.** If present, the lead wire **52** may be used to facilitate movement of the system **10** in the lumen **14.**

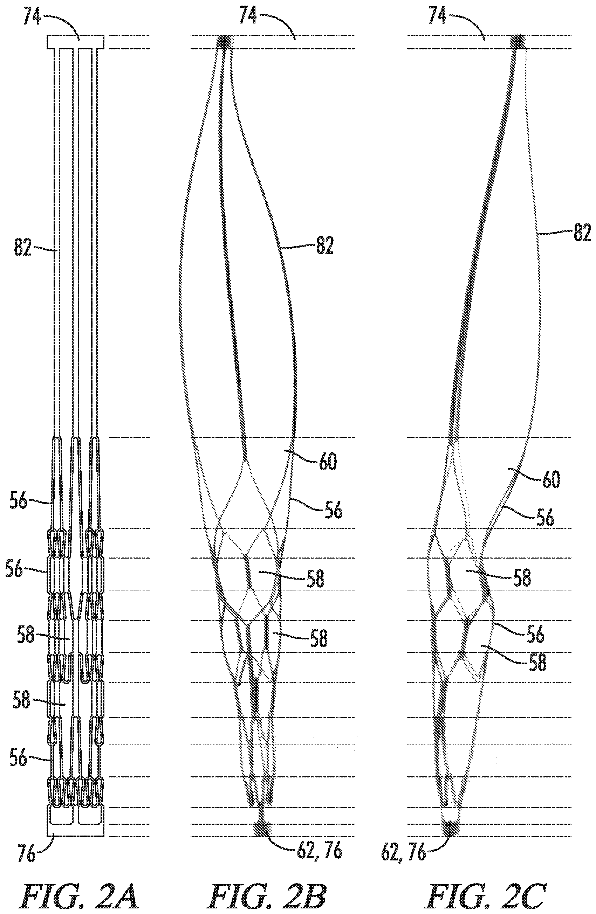

Optionally, the distal body **22** includes a basket **54** distal to the proximal memory metal strips **40,** the basket **54** comprised of a plurality of memory metal strips **56** distal relative to the proximal memory metal strips **40.** The distal memory metal strips **56** may, for example, form a basket **54** with a plurality of mesh openings **58.** Optionally, the size of the mesh openings **58** in the basket **54** when the distal body **22** is in its relaxed state is less (preferably significantly less) than the diameter of an average-sized ischemic blood clot **12** so that the blood clot **12** does not escape from the distal basket **54** after being captured by the distal body **22.** Optionally, the basket **54** has an open proximal end **60** and a substantially closed distal end **62,** which is formed by distal tube **76.** Optionally, the distal and proximal hubs **74** and **76** and the distal basket **54** are comprised of a nitinol having the same material composition. Optionally, the size of the mesh openings **58** decreases from the proximal end **60** of the basket **54** to the distal end **62.** The distal basket **54** is best seen in FIG. 2 and can be comprised of a different number of cell patterns. The distal basket **54** is not shown in FIGs. 3-10 for ease of illustrating the other components in the system **10.**

Optionally, the proximal hub **74** and the distal hub **76** are cylindrical tubes comprising substantially circular apertures that span the length of the hubs **74** and **76** and the hubs **74** and **76** have approximately the same inner diameter 72 and the same outer diameter 70. Preferably, the inner diameter 72 is at least slightly larger than the diameter of the pull wire 16 so that the pull wire 16 can slide through the proximal hub 74. In some examples, the outer diameters 70 of the proximal and distal hubs 74 and 76 may be from about 0.011 inches to about 0.054 inches and the inner diameters 72 of the proximal and distal hubs 74 and 76 may be from about 0.008 inches to about 0.051 inches.

Optionally, the distal body **22** further comprises an x-ray marker **64** that is more visible under x-ray as compared to the proximal memory metal strips **40** when the distal body **22** is located in a cranial blood vessel inside the body of a human and the x-ray is taken from outside the human's body. If the connector strips **48** are welded to the proximal memory metal strips **40,** the x-ray markers **64** may be, for example, located at the welding site. In some cases, the increased thickness at the welding site may in of itself comprise the x-ray marker **64.** Preferably, the x-ray marker **64** is comprised of a radiopaque material. Some examples of radiopaque materials can include, but are not limited to, gold, platinum, palladium, tantalum, tungsten alloy, polymer material loaded with radiopaque filler, and the like. Preferably, the proximal memory metal strips **40** are comprised of nitinol and the x-ray marker **64** is comprised of a material having a density greater than the nitinol.

A catheter **50** with an open proximal end (not shown) and an open distal end **66** initially envelopes the system **10.** As used herein, the term "catheter" generally refers to any suitable tube through which the system **10** can be deployed. Preferably, the catheter **50** is sterile and comprised of a biocompatible material (i.e., a material that does not irritate the human body during the course of a 45 minute operation that involves using the system **10** to remove a clot **12** from an intracranial blood vessel **14**). The catheter **50** can be any suitable shape, including but not limited to generally cylindrical. Preferably, the catheter **50** is a microcatheter. For purposes of the present invention, when it is said that the catheter **50** envelopes the system **10,** it will be understood that the catheter **50** envelopes at least one component of the system **10** (preferably, the distal body **22,** the lead wire **52,** and the pull wire **16**). In some examples, the catheter **50** is about 2.5 French in diameter. Optionally, the catheter **50** is delivered to the region of the lumen **14** that has the obstruction **12** as follows: a guide wire is delivered to the obstruction region past the obstruction **12;** the catheter **50** is delivered over the guide wire; the guide wire is removed; and the system **10** is delivered with its pull wire **16** and lead wire **52** through the catheter **50.** Optionally, the pull wire **16** is used to push the system **10** through the catheter **50** as well as to retrieve the distal body **22** after capturing the obstruction **14** as described below.

The system **10** may utilize a plurality of catheters **50,** such as, for example, a wider catheter that travels to the brain and a very flexible, smaller diameter microcatheter that is delivered from the first catheter and travels through the small arteries of the brain. Preferably, the catheter **50** is comprised of a biocompatible, polymeric material (i.e., one or more polymeric materials such as silicone, PVC, latex rubber or braided nylon).

Optionally, in the relaxed, opened-claw state, the distal body **22** or optionally just the distal basket **54** has a tapered shape (e.g., substantially conical or bullet in shape) so that the distal body **22** or just the distal basket **54** tapers from the distal body **22** or the distal basket's **54** proximal end to the distal end.

The proximal end of the system **10** is shown at the left end of FIGs. 1 and 3-10 and the distal end of the system **10** is shown at the right end of FIGs. 1 and 3-10 because a principal use of the system **10** is to remove a blood clot **12** from a human intracranial artery **14,** in which case the system **10** generally will enter the artery **14** at its proximal end by the surgeon entering the patient's body near the groin and pushing the catheter **50** towards the brain. The diameter of human arteries **14** generally decrease from their proximal end to their distal end. However, when used in other types of lumens, the distal body **22** may be located proximally relative to the catheter **50** as the term proximally and distally are used in that lumen.

The surgeon may deploy the distal body **22** by, for example, moving the catheter **50** proximally so as to unsheathe the distal body **22** or by pushing the distal body **22** out of the catheter **50.**

Use of the system **10** will now be described to remove a blood clot **12** from an intracranial artery **14** of a human ischemic stroke patient, however, it will be appreciated that the system **10** may be used to remove other objects from other interior lumens.

A catheter **50,** which contains the collapsed distal body **22** is positioned in the lumen **14** distal to the clot **12.** See FIG. 10A.

The distal body **22** is deployed from the catheter **50** and the height and width of the distal body **22** expand to about the height and width of the blood vessel **14.** See FIG. 10B.

The catheter **50** is pulled proximally and a claw-actuator tube **90** is deployed into the blood vessel **14.** See FIG. 10C.

The distal body **22** is moved proximally so that the clot **12** is located in the interior **28** of the distal body **22.** See FIGs. 10D and 10E.

The claw-actuator tube **90** is moved distally, which pushes the proximal hub **74** distally so that the distance **88** between the proximal hub **74** and the distal hub **76** (which is fixed to the pull wire 16 and kept stationary) decreases. Distal movement of the proximal hub 74 exerts tension on the connector and proximal memory metal strips 40 and 48, which in turn, closes the claw 46. See FIG. 10F. (The claw actuator tube 90 should float on the pull wire 16 - i.e., have an aperture extending the tube's length that has a diameter larger than the diameter of the pull wire 16 - and the aperture of the claw actuator tube 90 should be smaller than the diameter of the proximal hub 74 so that the claw actuator tube 90 pushes the proximal hub 74).

The system 10 is withdrawn proximally and removed from the body. See FIG. 10G.

To test the efficacy of the system 10, a distal body 22 with a distal basket 54, proximal and distal hubs 74 and 76, and a claw 46 comprised of three proximal memory metal strips 42 was tested in a flow model that included a tube and a moist cotton ball located in the tube. The cotton ball was used to simulate a blood clot. The system 10 was deployed distal to the cotton ball. The claw 46 was closed by moving the proximal hub 74 distally to capture the cotton ball. The system 10 and cotton ball were withdrawn proximally in the tube.

In some examples, the distal body 22 is prepared by a process that includes one or more of the following steps, as illustrated in FIGs. 1-4
a) providing a single tube 68 comprised of a memory metal such as nitinol, the single tube 68 having an exterior, a substantially hollow interior, a wall separating the exterior from the substantially hollow interior, an open proximal end 74, an open distal end 76, a middle portion 78 between the open proximal end 74 and the open distal end 76 (see FIG. 1 A);
b) cutting the wall of the middle portion 78 with a laser 80 (see FIG. IB);
c) removing the pieces of the middle portion 78 cut by the laser 80 to form a proximal tube 74, a distal tube 76 and a middle portion 78 comprising a plurality of memory metal strips 82 attached to the proximal tube 74;
d) altering the shape of the middle portion 78 using a mandrel and allowing the middle portion 78 to expand relative to the distal tube 76 and proximal tube 74 to form the distal basket 54;
e) quenching the middle portion 78 at room temperature;
f) removing the mandrel from the middle portion 78 (see FIGs. 2 and 3 A);
g) mechanically or chemically electropolishing the middle portion 78 to remove oxides;
h) cutting the memory metal strips 82 to form a first segment 84 comprising the proximal tube 74 and a proximal segment of the memory metal strips 82 and a second segment 86 comprising the distal tube 76 and a distal segment of the memory metal strips 82 (see FIG. 3B); and
i) joining the proximal segments to the distal segments such that the distal segments form the proximal end 24 of the distal body 22, such that the proximal tube 74 is located inside the interior 28 of the distal body 22, and such the proximal tube 74 is located distal relative to the distal body proximal end 24 (see FIGs. 3C-3E).

In some examples, the method further includes placing the pull wire 16 through the proximal tube 74 so that the proximal tube 74 is slideable along at least a segment of the pull wire 16.

In some examples, the method further includes attaching the pull wire 16 to the distal tube 76 so that the distal tube 76 is not slideable along the pull wire 16 but instead the distal tube 76 moves with the pull wire 16.

In some examples, after step i, the proximal end 24 of the distal body 22 forms a claw 46 comprised of between 2 to 4 proximal memory metal strips 40, the claw proximal memory metal strips 40 configured to move towards each other and the pull wire 16 by moving the proximal tube 74 distally and toward the distal tube 76 (i.e., decreasing the distance 88 between the tubes 74 and 76) and the claw memory metal strips 40 configured to move away from each other and away from the pull wire (i.e., increasing the distance 88 between the tubes 74 and 76) by moving the proximal tube 76 proximally and away from the distal tube 76 (as described previously).

In some examples, the middle portion 78 is expanded by heating the mandrel and the middle portion 78 by, for example, placing the mandrel and the middle portion 78 in a fluidized sand bath at about 500°C for about 3 to about 7 minutes. As the middle portion 78 is heated, the heating causes the crystalline structure of the memory metal tube 68 to realign. Preferably, the mandrel is tapered (e.g., substantially conical or bullet in shape) so that the distal basket 54 formed from the middle portion 78 tapers from the proximal end 60 to the distal end 62. Preferably, the proximal and distal ends of the tube 74 and 76 are not shape set by the mandrel and are not cut by the laser 80 so that the proximal and distal ends 74 and 76 do not change in shape and only slightly expand in size under heating and return to the size of the native tube 68 after the heat is removed. Preferably, the laser cuts are programmed via a computer. To ensure that the laser cuts only one surface of the tube wall at the time (and not the surface directly opposite the desired cutting surface), the laser 80 is preferably focused between the inner and outer diameter of the desired cutting surface and a coolant is passed through the memory metal tube **68** so that the laser **80** cools before reaching the surface directly opposite the desired cutting surface.

The portions of the wall not cut by the laser **80** create the distal basket **53,** proximal and distal tubes **74** and **76,** and memory metal strips **40, 48** and **56,** as described.

Preferably, the memory metal selected for the native tube **68** has a heat of transformation below average human body temperature (37°C) so that the distal body **22** has sufficient spring and flexibility after deployment from the catheter **50** in the human blood vessel **14**

In some examples, the native tube **68** (and hence the distal and proximal tubes **74** and **76**) have an outer diameter of less than about 4 French, e.g., a diameter of about 1 to about 4 French. In some examples, the diameter of the pull wire **16** is between about 0.008 inches and about 0.051, as noted above, and in such examples, the diameter of the pull wire **16** may be approximately equal to the inner diameter **72** of the native nitinol tube **68.**

Without being bound by any particular theory, it is believed that manufacturing the distal body **22** from a single memory metal tube **68** provides ease of manufacturing and safety from mechanical failure and provides tensile strength necessary for the system **10** to remove hard thrombus **12** and other obstructions.

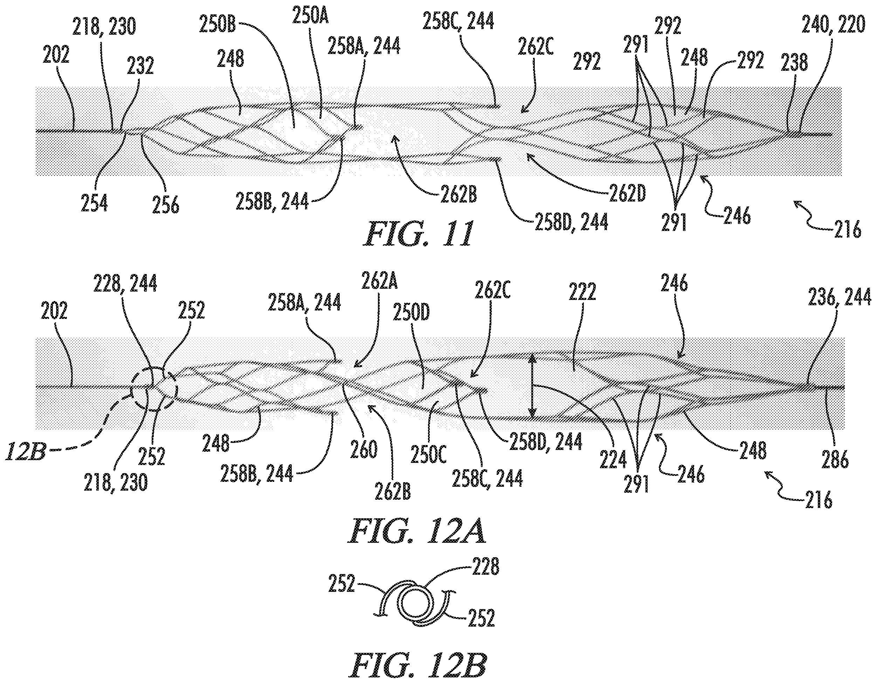

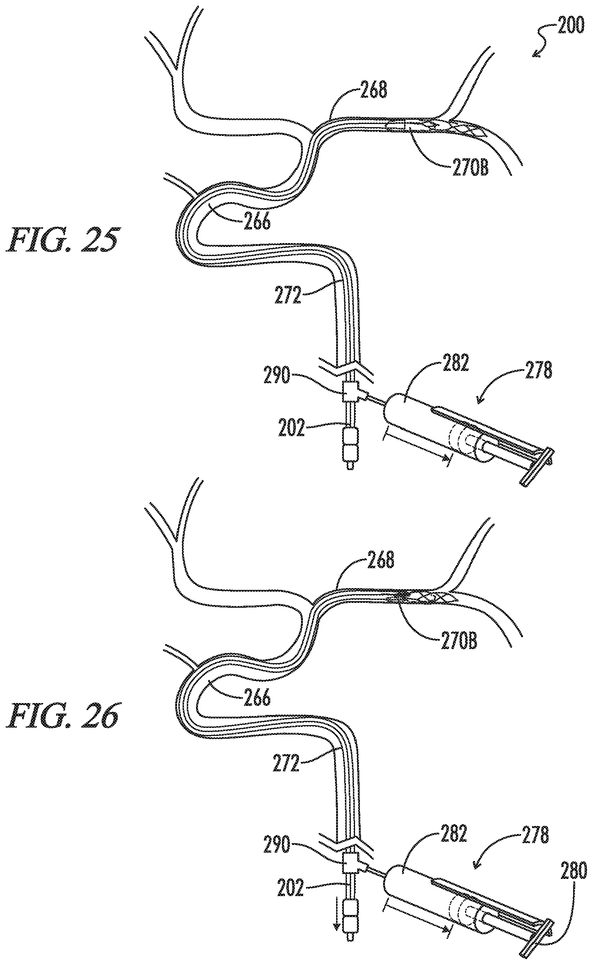

### The Examples of Figures 11-29

Figures 11-29 illustrate an alternate example **200,** shown for reference purposes only, that includes one or more of the following additional features, as described below: twisting proximal strips/tethers **252,** unattached/free distal-pointing crowns **258** that optionally curve inward and have x-ray markers **244,** and enlarged openings/drop zones **262** in the basket **246** immediately distal to the unattached, distal-pointing crowns **258** that allow the obstruction or other object **270** to enter the distal basket interior **222.**

More specifically, as shown in FIGs. 11-29, the system **200** may include a pull wire **202** having a proximal end **204** and a distal end **206,** as described above, a distal body **216** attached to the pull wire **202,** the distal body **216** comprising an interior **222,** a proximal end **218,** a distal end **220,** a distal body length **226** extending from the proximal end **218** to the distal end **220,** a distal body height **224,** a proximal hub **228** (preferably in the form of a tube and which has a proximal end **230** and a distal end **232**) forming the proximal end **218** of the distal body **216,** a basket **246** comprised of a plurality of cells/openings **248** formed by a plurality of basket strips **291** that preferably are comprised of a memory metal, optionally a distal hub **236** that forms the distal end **220** of the basket **246** (preferably in the form of a tube that has a proximal end **238** and a distal end **240**), and a plurality of proximal strips **252** (preferably the proximal strips **252** are comprised of a memory metal), each proximal strip **252** having a proximal end **254** attached to the proximal hub/tube **228,** and a distal end **256** attached to a cell **248** (more specifically a proximal-pointing crown of a cell **248** located at the proximal end of the basket **246**), the basket comprising a basket interior **292,** the distal body **216** having a relaxed state wherein the distal body **216** has a first height and width, a collapsed state wherein the distal body **216** has a second height and width, the second height less than the first height, the second width less than the first width; and a delivery catheter **208** for delivering the distal body **216,** as described above, having an interior **210,** a proximal end **212** leading to the interior **210** and a distal end **214** leading to the interior **210,** the delivery catheter **208** comprised of a biocompatible (preferably polymeric) material and configured to envelope the distal body **216** when the distal body **216** is in the collapsed state. Optionally, the basket interior **292** is substantially hollow - i.e., unlike U.S. Patent Publication No. 2013/0345739, the basket interior **292** does not contain an inner elongate body. Optionally, instead of a distal hub **236,** the basket **246** includes an open distal end. Optionally, at least two cells **250** of the basket **246** comprise a proximal crown **260** pointing generally in the proximal direction and a distal crown **258** pointing generally in the distal direction, and the distal crowns **258** of the at least two cells **250** are not attached to another cell **248** of the basket **246.** In other words, the distal crowns **258** of at least two cells **250** are free floating and are not attached to any strip except for the strips forming part of the at least two cells **250;** such distal crowns **258** are referred to below as unattached, distal-pointing crowns **258.** Preferably, the distal tips of the unattached, distal-pointing crowns **258** terminate at an x- ray marker **244.** (Cells labeled with the numerals **250, 250A, 250B, 250C,** and **250D** refer to the at least two cells that include a proximal crown **260** pointing generally in the proximal direction and an unattached, distal-pointing crown **258,** cells labeled with the numerals **262, 262A, 262B, 262C,** and **262D** refer to the enlarged cells/drop zones adjacent to (preferably immediately distal to) an unattached, distal-pointing crown **258,** and cells designated with numeral **248** refer to generally the cells of the basket **246**). (When it is said that the enlarged cells/drop zones **262** are preferably immediately distal to an unattached, distal-pointing crown **258,** it will be understood that at least a portion of an enlarged cell/drop zone **262** is immediately distal to an unattached, distal-pointing crown **258,** and that a portion of the enlarged cell/drop zone **262** may be proximal to an unattached, distal-pointing crown **258,** as shown in FIGs. 11-12 due to the shape of the enlarged cells/drop zones **262**). It will be understood that part number **250** refers generally to one or more of the at least two cells, whereas part numbers **250A, 250B, 250C,** and **250D** refer to a specific one of the at least two cells. Similarly, it will be understood that part number **262** refers generally to one or more of the enlarged cells/drop zones, whereas part numbers **262A, 262B, 262C,** and **262D** refer to a specific one of the enlarged cells/drop zones. Similarly, it will be understood that part number **258** refers generally to one or more of the unattached, distal- pointing crowns, whereas part numbers **258A, 258B, 258C,** and **258D** refer to a specific one of the unattached, distal-pointing crowns.

Optionally, at least two of the unattached, distal-pointing crowns **258** are located approximately 180 degrees (e.g., about 150 to about 180 degrees) relative to each other and approximately the same distance from the proximal hub/tube **228,** as best seen in FIG. 12A. Optionally, the basket **246** comprises a first pair of unattached, distal-pointing crowns **258A** and **258B,** each of the first pair of unattached, distal-pointing crowns **258A** and **258B** is located approximately the same distance from the proximal hub/tube **228** and approximately 180 degrees relative to each other, and the basket **246** further comprises a second pair of unattached, distal- pointing crowns **258C** and **258D** located distally relative to, and approximately 90 degrees (e.g., between about 60 and about 90 degrees) relative to, the first pair of unattached, distal-pointing crowns **258A** and **258B.** Optionally, the second pair of unattached, distal-pointing crowns **258C** and **258D** form cells **250C** and **250D** that are adjacent to, but offset from, the cells **250A** and **250B** formed by the first pair of unattached, distal-pointing crowns **258A** and **258B.** (In other words, optionally, the center of cell **250A** is about 90 degrees relative to the centers of cells **250C** and **250D** and optionally the center of cell **250B** is also about 90 degrees relative to the centers of cells **250C** and **250D**). Optionally, at least one of (and preferably all) the unattached, distal-pointing crowns **258A, 258B, 258C or 258D** comprise an x-ray marker **244** that is more visible under x-ray as compared to the basket strips **291** when the distal body **216** is located in a cranial blood vessel **266** inside the body of a human and the x-ray is taken from outside the human's body. Preferably, the x-ray marker **244** is a radiopaque material. Some examples of radiopaque materials can include, but are not limited to, gold, platinum, palladium, tantalum, tungsten alloy, polymer material loaded with radiopaque filler, and the like. Preferably, the basket strips **291** are comprised of nitinol and the x-ray marker **244** is comprised of a material having a density greater than the nitinol. In some examples, the x-ray markers **244** comprise a heavy metal welded to the unattached, distal-pointing crowns **258.** Optionally, the unattached, distal-pointing crowns **258** curve subtly towards the interior **222** of the distal basket **246,** which decreases the likelihood that the unattached, distal-pointing crowns **258** will rub against and damage the vessel wall **268.** Optionally, the basket **246** comprises at least two cells proximal to the at least two cells **250** that include the unattached, distal-pointing crowns **258.** Optionally, the unattached, distal-pointing distal crowns **258** are located about at least 5 mm (e.g., about 5 to about 30 mm) from the proximal hub/tube **228.** Optionally, the unattached, distal-pointing crowns **258** are located at least about 5 mm from the distal hub/tube **236.** Optionally, the unattached, distal-pointing crowns **258** of the at least two cells **250** also each form part (namely a portion of the proximal boundary) of an enlarged cell **262** (which is the entry point of hard thrombus **270B** into the basket interior **222**) and further wherein the surface area of the enlarged cells **262** in the relaxed state is greater than the surface area of the other cells of the basket **246** in the relaxed state. Optionally, the unattached, distal-pointing crowns **258** serve several functions: 1) they form flex points of the basket **246,** which makes it easier for the system **200** to navigate the curves of the blood vessels **266** of the brains; 2) through the use of x-ray markers **244** on the unattached, distal-pointing crowns **258,** they allow the operator to locate the enlarged cells **262** of the basket **246** that form the point at which hard thrombuses **270B** enter the basket **246;** and 3) they allow the operator to ratchet or force the object **270** into the basket **246** by moving the unattached, distal-pointing crowns **258** proximally and distally relative to the object **270.** (As explained below, the numeral **270** refers to clots/thrombuses and other objects generally, and **270A** refers to a soft clot, **270B** refers to a hard clot and **270C** refers to a deformable, cohesive, adherent clot). Optionally, the proximal end **254** of a proximal strip **252** is located about 65-180 degrees (preferably approximately 180 degrees) relative to the distal end **256** of the same proximal strip **252,** as best seen in FIG. 12B. In other words, preferably the proximal end **254** of a first proximal strip **252** is attached to the 12 o'clock position on the proximal tube **228** and the distal end **256** of the first proximal strip **252** (which terminates at a proximal cell **248** of the basket **246**) is located at the 6 o'clock position (i.e., 180 degrees from the start position), and the proximal end **254** of a second proximal strip **252** is attached to the 6 o'clock position on the proximal tube **228** and the distal end **254** (which terminates at a cell **248** of the basket **246**) of the second proximal strip **252** is located at the 12 o'clock position (i.e., 180 degrees from the start position). This twisting feature serves two functions: 1) it allows the proximal strips **252** to surround the object **270;** and 2) it allows the manufacturer to insert a mandrel into the basket **246** during the shape-setting procedure. Optionally, the pull wire **202** is attached to the proximal tube **228** (e.g., by gluing, welding or the like). Preferably, the pull wire **202** does not extend through the distal basket interior **222.** Optionally, the proximal strips **252** are integral with the distal end **232** of the proximal tube **228** and the entire distal body **216** is created from a single tube **264** of a memory metal. Optionally, the proximal crowns **260** of the at least two cells **250** that include the unattached, distal pointing-crowns **258** are each attached to another cell **248** of the basket **246.** In other words, preferably the basket **246** does not have any free-floating proximal-pointing crowns, as free-floating proximal-pointing crowns could damage the vessel **266** when the distal body **216** is pulled proximally. Optionally, the system **200** further comprises a lead wire **286** extending distally from the distal tube **236,** the lead wire **286** having a length of from about 3 mm to about 10 mm. Optionally, the distal hub/tube **236,** the proximal hub/tube **228,** and the basket **246** are comprised of a nitinol having the same material composition. In other words, as with the prior example of FIGs. 1-10, optionally the entire distal body **216** is manufactured from a single tube of nitinol **264.** Optionally, the proximal and distal hubs/tubes **228** and **236** comprise an x-ray marker **244** that is more visible under x-ray as compared to the basket strips **291** when the distal body **216** is located in a cranial blood vessel **266** inside the body of a human and the x-ray is taken from outside the human's body. Preferably, the x-ray marker **244** is a radiopaque material. Some examples of radiopaque materials can include, but are not limited to, gold, platinum, palladium, tantalum, tungsten alloy, polymer material loaded with radiopaque filler, and the like. Preferably, the basket strips **291** are comprised of nitinol and the x-ray marker **244** is comprised of a material having a density greater than the nitinol. In some examples, the proximal and distal hubs/tube interiors **234** and **242** may comprise tantalum welded or otherwise attached to the interior **234** and **242** of the proximal and distal hubs/tubes **228** and **236.** Optionally, the proximal and the distal tubes **228** and **236** are generally cylindrical in shape and each has an outer diameter and an inner diameter, the inner diameter forming apertures of the proximal and distal tubes **228** and **236** and further wherein the outer diameters of the proximal and distal tubes **228** and **236** are substantially the same size and further wherein the inner diameters of the proximal and distal tubes **228** and **236** are substantially the same size. Optionally, the outer diameters of the proximal and distal tubes **228** and **236** are from about 0.011 inches to about 0.054 inches, and further wherein the inner diameters of the proximal and distal tubes **228** and **236** are from about 0.008 inches to about 0.051 inches. Optionally, the pull wire **202** is generally cylindrical and further wherein the diameter of the pull wire **202** is between about 0.008 inches and about 0.051 inches. Optionally, the distal body **216** has a length of between about 10 and about 60 millimeters. Optionally, the first height **224** and first width **226** of the distal body **216** are between about 2 millimeters and about 6 millimeters.

The present disclosure also provides a method of removing a clot or other object **270** from an interior lumen **266** of an animal, the method comprising the steps of:
a) providing the system **200** of Figures 11-29, wherein at least two cells **250** of the basket **246** comprise a proximal crown **260** pointing generally in the proximal direction and a distal crown **258** pointing generally in the distal direction, and the distal crowns **258** of the at least two cells **250** are not attached to another cell **248** of the basket **246** (i.e., free-floating), and further wherein at least one of the unattached, distal-pointing crowns **258** comprises an x-ray marker **244;**
b) positioning the system **200** in the lumen **266;**
c) deploying the distal body **216** from the distal end **214** of the delivery catheter **208;**
d) allowing the height and width **224** and **226** of the distal body **216** to increase;
e) irradiating the x-ray marker **244** with x-ray radiation and
f) moving the object **270** into the distal basket interior **222.**

Optionally, the object **270** enters the distal basket interior **222** adjacent to (preferably adjacent and immediately distal to) at least one of the unattached, distal-pointing crowns **258** - i.e., in the enlarged cells/drop zones **262.** In some examples, the distal body **216** is deployed so that at least one (e.g., preferably the two proximal **258A** and **258B**) of the unattached, distal-pointing crowns **258** is distal to the object **270.** As explained below, the x-ray markers **244** of the unattached, distal-pointing crowns **258** are used to locate the distal body **216** relative to the clot or other object **270.** It will be appreciated that clots **270** can generally be located in blood vessels **266** by injecting a contrast dye, for example, into the blood vessel **266** proximal and distal to the believed area of obstruction and viewing on an x-ray where the fluid stops moving in the blood vessel **266.** It will also be appreciated that if the object **270** is not a blood clot but is a radio-opaque object, the object **270** may be viewed on an x-ray.

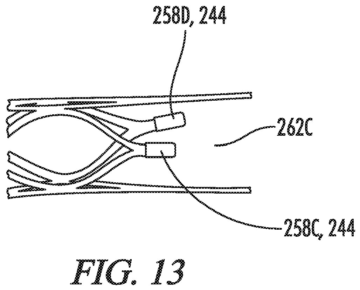

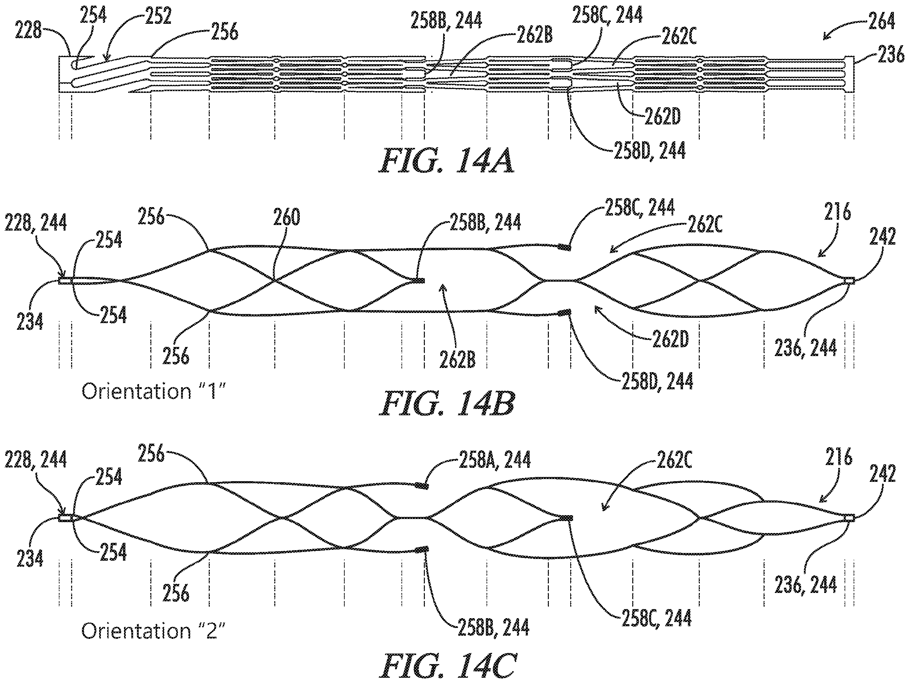

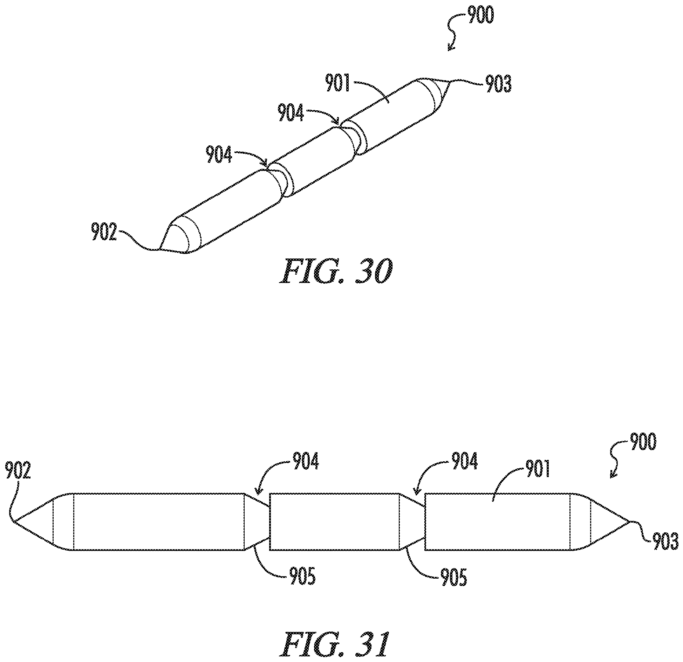

FIGs. 11 and 14B illustrate a first, perspective view of one embodiment of a distal body **216** with twisting proximal strips **252,** unattached distal-pointing crowns **258** that subtly curve inward and have x-ray markers **244,** and enlarged openings/drop zones **262** in the basket **246** that allow the obstruction or other object **270** to enter. In FIGs. 11 and 14B, the distal body **216** is in Orientation 1. (To prepare a basket **246** with unattached distal-pointing crowns **258** that curve inward toward the basket interior **292,** a mandrel **900** such as that illustrated in FIGs. 30 and 31 may be used. The mandrel **900** includes a generally cylindrical body **901** with tapered proximal and distal ends **902** and **903** that slope like the ends of a pencil. The cylindrical body **901** includes two grooves **904** that extend around the circumference of the cylindrical body **901.** The grooves **904** include tapered portions **905** that slope towards the distal end **903,** which are designed to shape the unattached distal-pointing crowns **258.** The grooves **904** are generally in the shape of a truncated cone, as shown in FIGs. 30-31). The two proximal, unattached distal- pointing crowns **258A** and **258B** are located approximately the same distance from the proximal hub/tube **228** and are oriented approximately 180 degrees relative to each other. The two distal, unattached distal-pointing crowns **258C** and **258D** are located approximately the same distance from the proximal hub/tube **228** as each other (and distal to the two proximal, unattached distal- pointing crowns **258A** and **258B**) and are oriented approximately 180 degrees relative to each other and approximately 90 degrees to the proximal, unattached distal-pointing crowns **258A** and **258B.** The two proximal enlarged openings/drop zones **262A** and **262B** distal to the proximal, unattached distal pointing crowns **258A** and **258B** are located approximately the same distance from the proximal hub/tube **228** and the centers of the two proximal enlarged openings/drop zones **262A** and **262B** are oriented approximately 180 degrees relative to each other. (As noted above, preferably, the proximal, unattached distal-pointing crowns **258A** and **258B** form part of the proximal boundary of the proximal, enlarged cells/drop zones **262A** and **262B,** and the distal, unattached distal-pointing crowns **258C** and **258C** form part of the proximal boundary of the distal, enlarged cells/drop zones **262C** and **262D**). The two distal, enlarged openings/drop zones **262C** and **262D** distal to the distal, unattached distal pointing crowns **258C** and **258D** are located approximately the same distance from the proximal hub/tube **228** and the centers of the distal, enlarged openings/drop zones **262C** and **262D** are oriented approximately 180 degrees relative to each other and approximately 90 degrees relative to the proximal enlarged openings/drop zones **262A** and **262B.** FIGs. 12A and 14C illustrate a second view of the distal body **216** of FIG. 11 (Orientation 2). FIG. 13 is a close-up view of two unattached, distal-pointing crowns **262.** The lines in FIG. 14 show how a nitinol tube **264** is cut with a laser to create the distal body **216** shown in FIG. 14B and FIG. 14C. It will be appreciated that FIG. 14B is a simplified view of the distal body **216** and orientation shown in FIG. 11 and FIG. 14C is a simplified view of the distal body **216** and orientation shown in FIG. 12A.

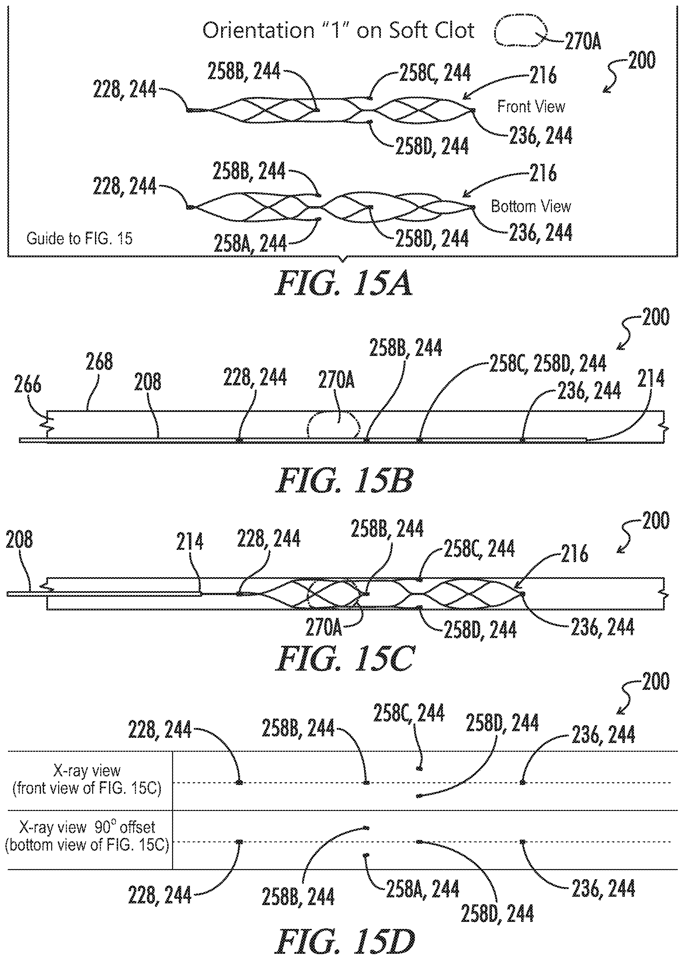

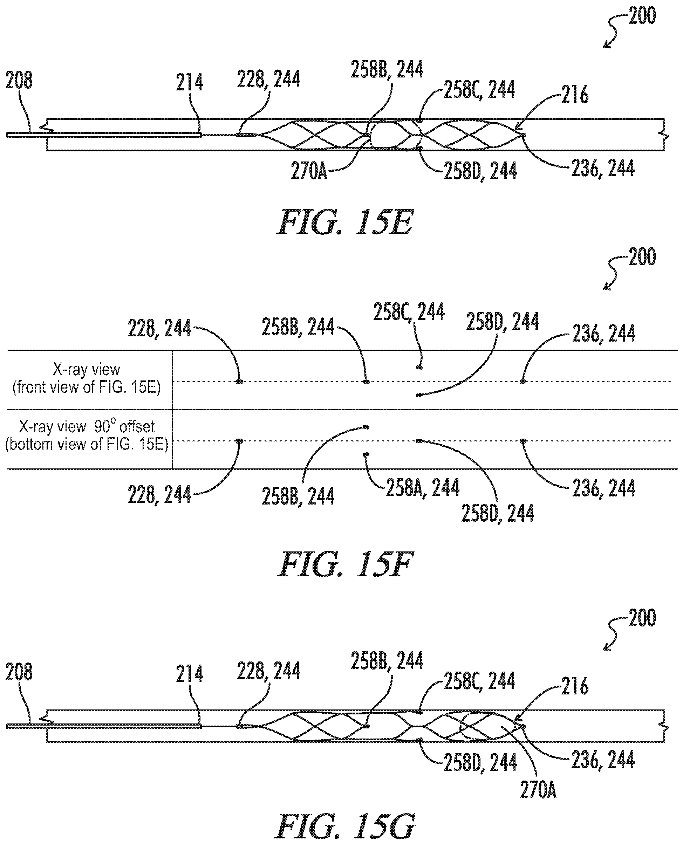

As described below, FIGs. 15-19 describe how the distal body **216** is used to retrieve, soft clots **270A,** hard clots **270B,** and deformable, cohesive adhesive clots **270C** in a human intracranial artery **266.** (In FIGs. 15-19, the center of the artery **266** is denominated by the dashed line). As explained below, the distal body **216** has four rows of x-ray markers namely, 1) a first row of one x-ray marker, which is located inside the proximal tube denominated by the numeral **228, 244;** 2) a second row of two x-ray markers, which are located at the two proximal, unattached distal-pointing crowns (the two markers are oriented 180 degrees relative to each other) denominated by the numerals **258A, 244** and **258B, 244;** 3) a third row of two x-ray markers, which are located at the two distal, unattached distal-pointing crowns (these two markers are oriented 180 degrees relative to each other and 90 degrees relative to the two proximal, unattached distal-pointing crowns) denominated by the numerals **258C, 244** and **258D, 244;** and 4) a fourth row of one x-ray marker, which is located inside the distal tube denominated by the numeral **236, 244.** (It will be appreciated that the first number in the sequence describes the position of the x-ray marker and the second number, **244,** represents the fact that the item is an x-ray marker). As explained below, upon deploying the distal body **216** so that the two proximal, unattached distal-pointing crowns **258A, 244** and **258B, 244** are immediately distal to the clot **270,** the surgeon interventionalist (i.e., operator of the distal body **216**) detects the four rows of x-ray markers using x-ray radiation from a first vantage point and from a second vantage point that is offset from the first vantage point (e.g. 90 degrees). Next, the surgeon moves the distal body **216** proximally relative to the clot **270** and takes additional x-rays from the first and second vantage points. As explained in greater detail below, the surgeon uses the x-ray markers of the proximal and distal, unattached distal-pointing crowns, namely **258A, 244; 258B, 244; 258C, 244;** and **258D, 244** (more specifically, the convergence or lack thereof of the proximal and distal, unattached distal-pointing crowns **258A, 244; 258B, 244; 258C, 244;** and **258D, 244** as shown on the x-ray) to determine whether the clot **270** is located inside the distal body interior **222** or whether the clot **270** is collapsing the distal body **216.**

More specifically, FIGs. 15A-G illustrate stepwise use of the distal body **216** in retrieving a soft clot **270A** in a human intracranial artery **266.** (The distal body **216** in FIGS. 15A-15G is in Orientation 1). First, as always, the surgeon determines the location of the clot **270A** in the vessel **266** using, for example, a contrast dye injected proximal and distal to the clot **270A.** Next, the delivery catheter **208,** which is enveloping the distal body **216,** is positioned in the blood vessel **266** so that the two proximal, unattached distal-pointing crowns **258A** and **258B** are immediately distal to the clot **270A.** See FIG. 15B. The distal body **216** is then deployed from the delivery catheter **208** by moving the catheter **208** proximally. The soft clot **270A,** which is unable to collapse the distal body **216,** then enters the distal body interior **222.** See FIG. **15C****.** However, at this time, the surgeon is unaware that the clot **270A** has entered into the distal body interior **222.** Thus, without moving the distal body **216,** the surgeon irradiates the four rows of x-ray markers at a first vantage point (i.e., from the front of the distal body **216** in the orientation shown in FIGs. 15A-G; i.e., into the page). As shown in FIG. 15D, the first vantage point shows four rows of x-ray markers. The first row is a single point, which represents the x- ray marker located in the proximal tube **228, 244;** the proximal tube x-ray marker **228, 244** always appears as a single point. The second row is a single point, which represents the x-ray marker located at the front, proximal, unattached distal-pointing crown **258B, 244;** the reason that this second row of markers is a single point is that the rear x-ray marker of the second row **258A, 244** is hidden from view because it is directly behind the front x-ray marker of the second row **258B, 244.** The third row has two points, which represents the two x-ray markers located at the distal, unattached distal-pointing crowns **258C, 244** and **258D, 244;** the reason that this third row of markers has two points is that neither marker in the third row **258C, 244** and **258D, 244** is hidden from view on the x-ray at this angle - rather, one marker **258C, 244** is located above the other marker **258D, 244** - and as shown in FIG. **15C****,** the distal body **216** is not collapsed at the distal, unattached distal-pointing crowns **258C, 244** and **258D, 244.** The fourth row is a single point, which represents the x-ray marker located in the distal tube **236, 244;** the distal tube x-ray marker **236, 244** always appears as a single point. Without moving the distal body **216,** the surgeon then irradiates the four rows of x-ray markers from a second vantage point 90 degrees offset from the first vantage point (i.e., from the bottom of the distal body **216** in the orientation shown in FIG. 15A). As shown, the first row is, as always, a single point, which represents the x-ray marker located in the proximal tube **228, 244.** The second row has two points, which represents the two x-ray markers located at the proximal, unattached distal-pointing crown **258A, 244** and **258B, 244;** the reason that this second row of markers shows up as two points is that neither marker **258A, 244** and **258B, 244** in the second row is hidden from view on the x-ray at this offset angle - rather, one marker **258B, 244** is located above the other marker **258A, 244** - and the distal body **216** is not collapsed at the proximal, unattached distal-pointing crowns **258A, 244** and **258B, 244.** The third row is a single point, which represents the x-ray marker located at the bottom, distal, unattached distal-pointing crown **258D, 244;** the reason that this third row of markers is a single point is that the top x-ray marker of the third row **258C, 244** is directly behind the bottom x-ray marker of the third row **258D, 244,** and thus, hidden from view. The fourth row is, as always, a single point, which represents the x-ray marker located in the distal tube **236, 244.** The surgeon, thus, concludes that neither the x-ray markers at the second row **258A, 244** and **258B, 244** nor the x-ray markers at the third row **258C, 244** and **258D, 244** (i.e., the x-ray markers at both the proximal and distal unattached distal pointing-crowns) have converged. As shown in FIG. 15E, the surgeon then moves the distal body **216** proximally relative to the soft clot **270A** so that the distal, unattached distal-pointing crowns **258C, 244** and **258D, 244** are immediately distal to the clot **270A** and then the surgeon irradiates the four rows of x-ray markers again from the first vantage point and the second vantage point. As shown in FIG. 15F, the results are the same as FIG. 15D. With the results from FIGs. 15D and 15F, the surgeon concludes that neither x-ray markers at the second row **258A, 244** and **258B, 244** nor the x-ray markers at the third row **258C, 244** and **258D, 244** (i.e., the x-ray markers at both the proximal and distal unattached distal pointing-crowns) converged at either the original position of the distal body **216** (FIGs. 15C and 15D) or the position after moving the distal body **216** proximally (FIGs. 15E and 15F), and, thus, the distal body **216** was expanded in the vessel **266** in both positions. Thus, the surgeon concludes that the clot is a soft clot **270A** that has entered into the distal body interior **222** and the surgeon removes the distal body **216** and the soft clot **270A,** captured by the distal body **216,** by moving the distal body **216** proximally out of the vessel **266,** as shown in FIG. 15G.

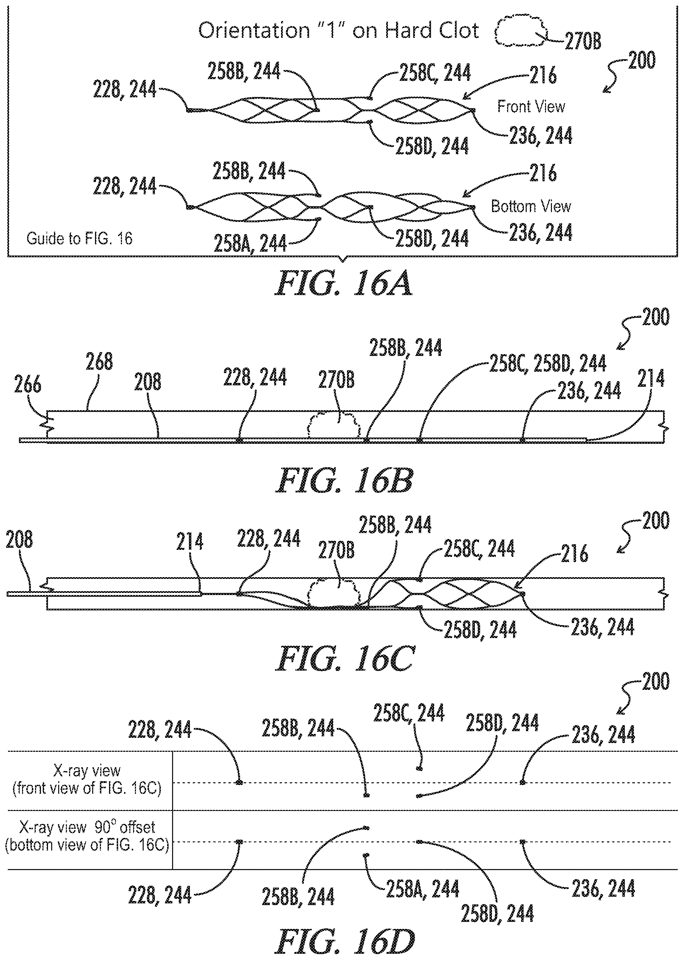

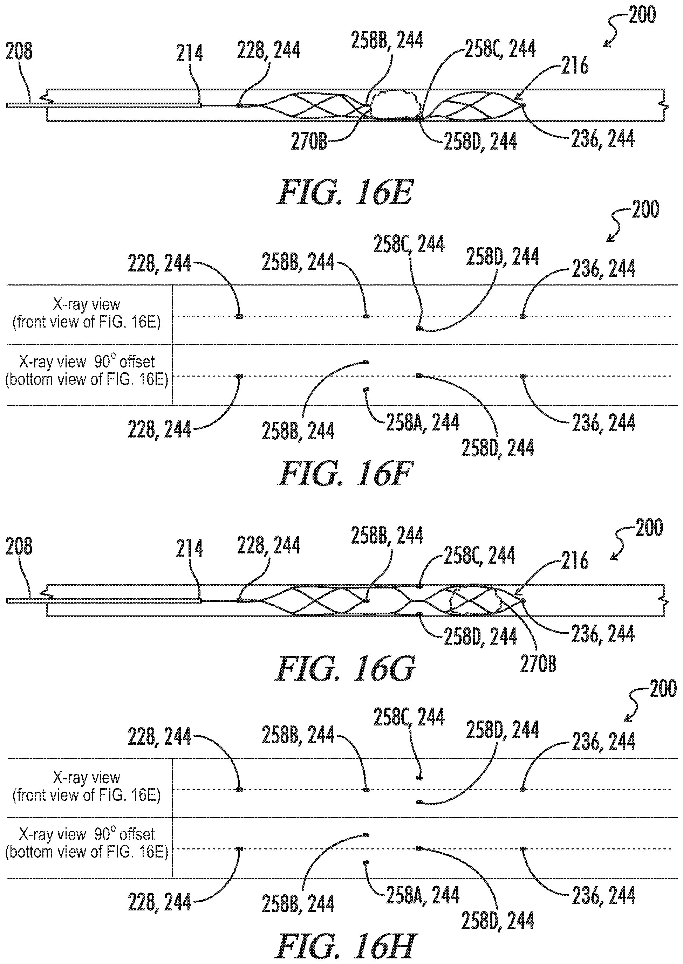

FIGs. 16A-H illustrate stepwise use of the distal body **216** in retrieving a hard clot **270B** in a human intracranial artery **266.** (In FIGs. 16A-H, the distal body **216** is in Orientation 1). First, as always, the surgeon determines the location of the clot **270B** in the vessel **266** using, for example, a contrast dye injected proximal and distal to the clot **270B.** Next, the delivery catheter **208,** which is enveloping the distal body **216,** is positioned in the blood vessel **266** so that the two proximal, unattached distal-pointing crowns **258A** and **258B** are immediately distal to the clot **270B.** See FIG. 16B. The distal body **216** is then deployed from the delivery catheter **208** by moving the catheter **208** proximally. The hard clot **270B,** which is located above the distal body **216,** collapses the distal body **216,** as shown in FIG. **16C****.** However, at this time, the surgeon is unaware that the clot **270B** has collapsed the distal body **216.** Thus, without moving the distal body **216,** the surgeon irradiates the x-ray markers at a first vantage point (i.e., from the front of the distal body **216;** i.e., into the page). As shown in FIG. 16D, the first vantage point shows four rows of x-ray markers. The first row is, as always, a single point, representing the x- ray marker located in the proximal tube - i.e., **228, 244.** The second row is a single point, which represents the x-ray marker located at the front, proximal, unattached distal-pointing crown **258B, 244;** the reason that this second row of markers is a single point is that the rear x-ray marker of the second row **258A, 244** is hidden from view because it is directly behind the front x-ray marker of the second row **258B, 244.** The third row has two points, which represents the two x-ray markers located at the distal, unattached distal-pointing crowns **258C, 244** and **258D, 244;** the reason that this third row of markers has two points is that neither marker in the third row is hidden from view on the x-ray at this angle - rather, one marker **258C, 244** is located above the other marker **258D, 244 -** and as shown in FIG. 16C, the distal body **216** is not collapsed at the distal, unattached distal-pointing crowns **258C, 244** and **258D, 244.** The fourth row is, as always, a single point, representing the x-ray marker located in the distal tube **236, 244.** Without moving the distal body **216,** the surgeon then irradiates the markers from a second vantage point 90 degrees offset from the first vantage point (i.e., from the bottom of the distal body **216**). As shown, the first row is, as always, a single point, which represents the x-ray marker located in the proximal tube **228, 244.** The second row has two points, which represents the two x-ray markers located at the proximal, unattached distal-pointing crowns **258A, 244** and **258B, 244;** the reason that this second row of markers shows up as two points is that neither marker in the second row is hidden from view on the x-ray at this offset angle - rather, one marker **258B, 244** is located above the other marker **258A, 244 -** and although the distal body **216** is collapsed at the proximal, unattached distal-pointing crowns as shown in FIG. 16C, the second row of x-ray markers have not converged because the clot **270B** is on top of the second row of x-ray markers. The third row is a single point, which represents the x-ray marker located at the bottom, distal, unattached distal-pointing crown **258D, 244;** the reason that this third row of markers is a single point is that the top x-ray marker of the third row **258C, 244** is directly behind the bottom x-ray marker of the third row **258D, 244,** and thus, hidden from view. The fourth row is, as always, a single point, which represents the x-ray marker located in the distal tube **236, 244.** The surgeon, thus, concludes that neither the second row **258A, 244** and **258B, 244** nor the third row **258C, 244** and **258D, 244** of x-ray markers (i.e., the x-ray markers at both the proximal and distal unattached distal pointing-crowns) has converged. As shown in FIG. 16E, the surgeon then moves the distal body 216 proximally so that the distal, unattached distal-pointing crowns **258C, 244** and **258D, 244** are immediately distal to the clot **270B** and the surgeon then irradiates the x-markers again from the first vantage point. As shown in FIG. 16F, the first row is, as always, a single point, representing the x-ray marker located in the proximal tube **228, 244.** The second row is a single point, which represents the x-ray marker located at the front, proximal, unattached distal-pointing crown **258B, 244;** the reason that this second row of markers is a single point is that the rear x-ray marker of the second row **258A, 244** is hidden from view because it is directly behind the front x-ray marker of the second row **258B, 244.** The third row has only one point because the clot **270B,** which is on top of the third row of x-ray markers **258C, 244** and **258D, 244** (i.e., the markers at the distal, unattached distal-pointing crowns), has pushed the third row of x-ray markers **258C, 244** and **258D, 244** together. The fourth row is, as always, a single point, representing the x-ray marker located in the distal tube **236, 244.** Without moving the distal body **216,** the surgeon then irradiates the markers from a second vantage point 90 degrees offset from the first vantage point (i.e., from the bottom of the distal body). As shown, the first row is, as always, a single point, which represents the x-ray marker located in the proximal tube **228, 244.** The second row has two points, which represents the two x-ray markers located at the proximal, unattached distal-pointing crown **258A, 244** and **258B, 244;** the reason that this second row of markers shows up as two points is that neither marker in the second row is hidden from view on the x-ray at this offset angle and the distal body **216** is not collapsed at the proximal, unattached distal-pointing crowns **258A, 244** and **258B, 244.** The third row is a single point, which represents the x-ray marker located at the bottom, distal, unattached distal-pointing crown **258D, 244;** the reason that this third row of markers is a single point is that the bottom x-ray marker of the third row **258D, 244** is directly in front of the top x-ray marker of the third row **258C, 244,** and thus, the top x-ray marker of the third row **258C, 244** is hidden from view. The fourth row is, as always, a single point, which represents the x-ray marker located in the distal tube **236, 244.** Knowing that the distal, unattached distal- pointing crowns **258C, 244** and **258D, 244** have converged as shown in FIG. 16F, the surgeon moves the distal body **216** proximally and the hard clot **270B** falls into the distal body interior **222** in the enlarged cell/drop zone **262C** immediately distal to the top, distal, unattached distal- pointing crown **258C.** See FIG. 16G. To confirm that the hard clot **270B** has entered the distal body interior **222,** the surgeon takes x-rays from the first and second vantage points. The results are shown in FIG. 16H. As compared to 16F, the front x-ray view of FIG. 16H shows that the distal, unattached distal-pointing crowns **258C, 244** and **258D, 244** are not converged, and, thus, the surgeon concludes that the hard clot **270B** has entered the distal body interior **222.** The surgeon then removes the distal body **216** and the hard clot **270B,** captured by the distal body **216,** by moving the distal body **216** proximally out of the vessel **266.**

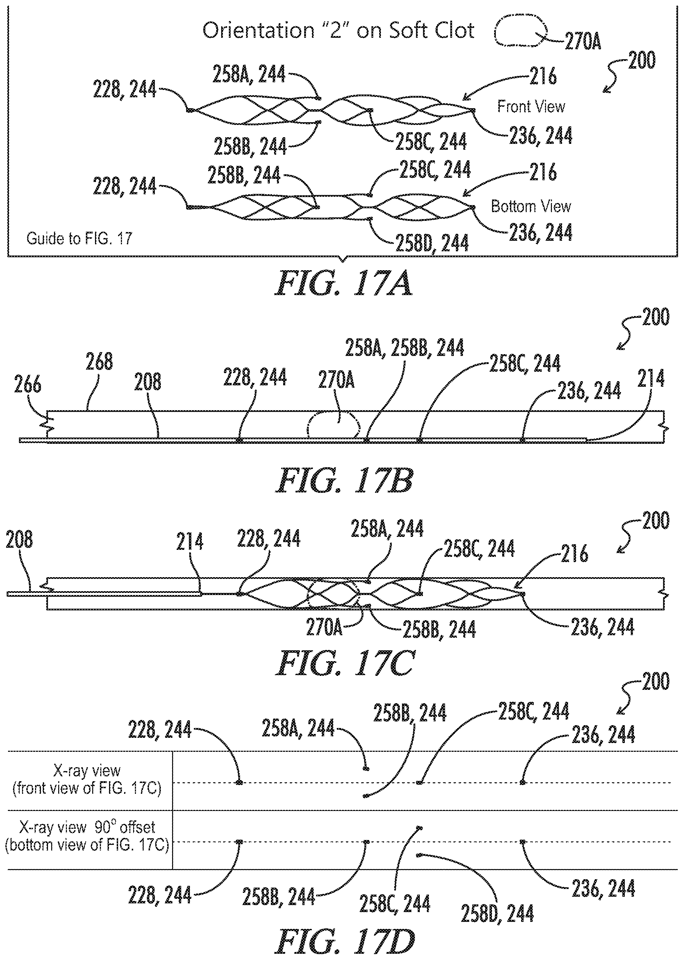

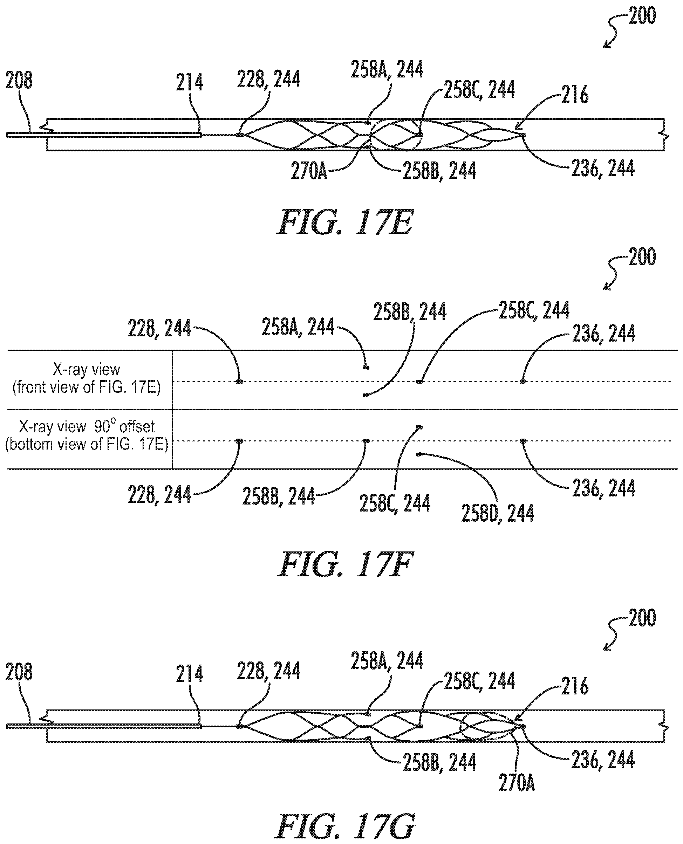

FIGs. 17A-G illustrate stepwise use of the distal body **216** in retrieving a soft clot **270A** in a human intracranial artery **266.** (In FIGs. 17A-G, the distal body **216** is in Orientation 2). First, as always, the surgeon determines the location of the clot **270A** in the vessel **266** using, for example, a contrast dye injected proximal and distal to the clot **270A.** Next, the delivery catheter **208,** which is enveloping the distal body **216,** is positioned in the blood vessel **266** so that the two proximal, unattached distal-pointing crowns **258A** and **258B** are immediately distal to the clot **270A.** See FIG. 17B. The distal body **216** is then deployed from the catheter **208** by moving the catheter **208** proximally. The soft clot **270A,** which is unable to collapse the distal body **216,** then enters the distal body interior **222.** See FIG. 17C. However, at this time, the surgeon is unaware that the clot **270A** has entered into the distal body interior **222.** Thus, without moving the distal body **216,** the surgeon irradiates the x-ray markers at a first vantage point (i.e., from the front of the distal body; into the page). As shown in FIG. 17D, the first vantage point shows four rows of x-ray markers. The first row is, as always, a single point, representing the x-ray marker located in the proximal tube **228, 244.** The second row has two points, which represents the two x-ray markers located at the proximal, unattached distal- pointing crowns **258A, 244** and **258B, 244;** the reason that this second row of markers has two points is that neither marker in the second row is hidden from view on the x-ray at this angle - rather, one marker **258A, 244** is located above the other marker **258B, 244** - and as shown in FIG. **17C****,** the distal body **216** is not collapsed at the proximal, unattached distal-pointing crowns **258A, 244** and **258B, 244.** The third row has a single point, which represents the x-ray marker located at the front (in Orientation 2), distal, unattached distal-pointing crown **258C, 244;** the reason that this third row of markers is a single point is that the rear (in Orientation 2) x-ray marker **258D, 244** of the third row is hidden from view because it is directly behind the front x- ray marker **258C, 244** of the third row. The fourth row is, as always, a single point, representing the x-ray marker located in the distal tube **236, 244.** Without moving the distal body, the surgeon then irradiates the markers from a second vantage point 90 degrees offset from the first vantage point (i.e., from the bottom of the distal body, as shown in this view). As shown, the first row is, as always, a single point, which represents the x-ray marker located in the proximal tube **228, 244.** The second row is a single point, which represents the x-ray marker located at the bottom (in Orientation 2), proximal, unattached distal-pointing crown **258B, 244;** the reason that this second row of markers is a single point is that the top (in Orientation 2) x-ray marker of the second row **258A, 244** is directly behind the bottom x-ray marker of the second row **258B, 244,** and thus, hidden from view. The third row has two points, which represents the two x-ray markers located at the distal, unattached distal-pointing crowns **258C, 244** and **258D, 244;** the reason that this third row of markers shows up as two points is that neither marker in the third row is hidden from view on the x-ray at this offset angle and the distal body **216** is not collapsed at the distal, unattached distal-pointing crowns **258C, 244** and **258D, 244.** The fourth row is, as always, a single point, which represents the x-ray marker located in the distal tube **236, 244.** The surgeon, thus, concludes that neither the second row **258A, 244** and **258B, 244** nor the third row of x-ray markers **258C, 244** and **258D, 244** (i.e., the x-ray markers at both the proximal and distal unattached distal pointing-crowns) has converged. As shown in FIG. 17E, the surgeon then moves the distal body 216 proximally relative to the clot **270A** so that the distal, unattached distal-pointing crowns **258C, 244** and **258D, 244** are immediately distal to the clot **270A** and then the surgeon irradiates the x-markers again from the first vantage point and the second vantage point. As shown in FIG. 17F, the results are the same as FIG. 17D. With the results from FIGs. 17D and 17F, the surgeon concludes that neither the second row **258A, 244** and **258B, 244** nor the third row of x-ray markers **258C, 244** and **258D, 244** (i.e., the x-ray markers at both the proximal and distal unattached distal pointing-crowns) were converged at either the original position of the distal body **216** (FIG. 17C and 17D) or the position after moving the distal body **216** proximally (FIG. 17E and 17F), and, thus, the distal body **216** was expanded in the vessel **266** in both positions. Thus, the surgeon concludes that the clot **270A** is a soft clot **270A** that has entered into the distal body interior **222** and the surgeon removes the distal body **216** and the soft clot **270A,** captured by the distal body **216,** by moving the distal body **216** proximally out of the vessel **266,** as shown in FIG. 17G.

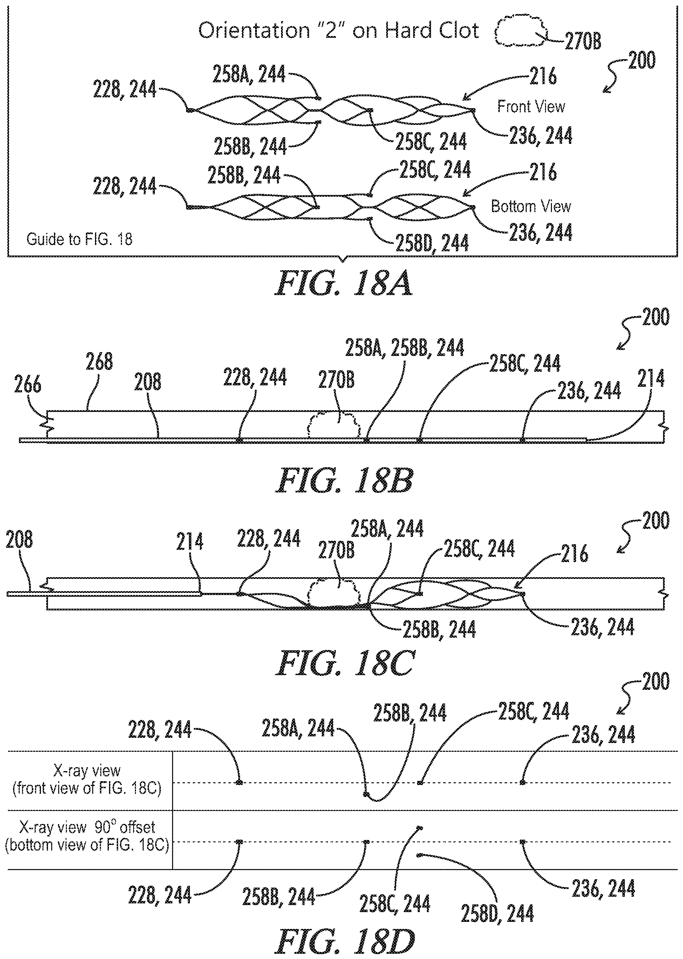

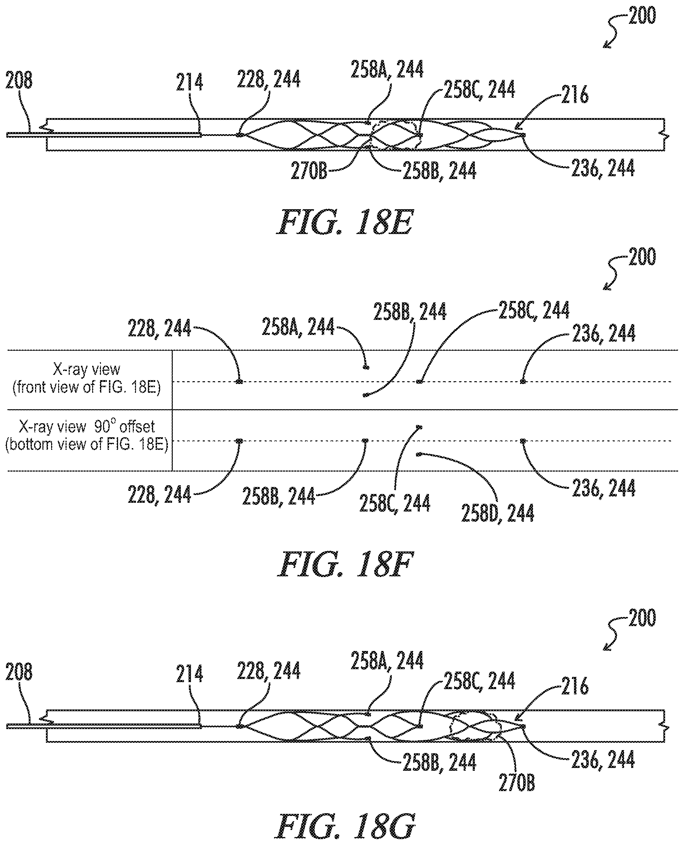

FIGs. 18A-G illustrate stepwise use of the distal body **216** in retrieving a hard clot **270B** in a human intracranial artery **266.** (In FIGS. 18A-G, the distal body **216** is in Orientation 2). (As described below, the primary differences between FIGs 18A-G and FIGs. 16A-G is that the clot **270B** enters the distal body interior **222** in an enlarged cell/drop zone **262A** immediately distal to one of the proximal, unattached distal-pointing crowns **258A** in FIGs. 18A-G, as compared to FIGs. 16A-G where the clot **270B** enters the distal body interior **222** in an enlarged cell/drop zone **262C** immediately distal to one of the distal, unattached distal-pointing crowns **258C**). First, as always, the surgeon determines the location of the clot **270B** in the vessel **266** using, for example, a contrast dye injected proximal and distal to the clot **270B.** Next, the delivery catheter **208,** which is enveloping the distal body **216,** is positioned in the blood vessel **266** so that the two proximal, unattached distal-pointing crowns **258A** and **258B** are immediately distal to the clot **270B.** See FIG. 18B. The distal body **216** is then deployed from the catheter **208** by moving the catheter **208** proximally. The hard clot **270B,** which is located above the distal body **216,** collapses the distal body **216,** as shown in FIG. **18C****.** However, at this time, the surgeon is unaware that the clot **270B** has collapsed the distal body **216.** Thus, without moving the distal body **216,** the surgeon irradiates the x-ray markers at a first vantage point (i.e., from the front of the distal body in Orientation 2; into the page). As shown in FIG. 18D, the first vantage point shows four rows of x-ray markers. The first row is, as always, a single point, representing the x-ray marker located in the proximal tube **228, 244.** The second row has only one point because the clot **270B,** which is on top of the second row of x-ray markers **258A, 244** and **258B, 244** (i.e., the markers at the proximal, unattached distal-pointing crowns), has pushed them together. The third row has only one point, which represents the x-ray marker located at the front (in Orientation 2), proximal, unattached distal-pointing crown **258C, 244;** the reason that this third row of markers is a single point is that the rear (in this view) x-ray marker of the third row **258D, 244** is hidden from view because it is directly behind the front x-ray marker of the third row **258C, 244.** The fourth row is, as always, a single point, representing the x-ray marker located in the distal tube **236, 244.** Without moving the distal body, the surgeon then irradiates the markers from a second vantage point 90 degrees offset from the first vantage point (i.e., from the bottom of the distal body **216**). As shown, the first row is, as always, a single point, which represents the x-ray marker located in the proximal tube **228, 244.** The second row has a single point because the top (in Orientation 2) x-ray marker of the second row **258A, 244** is located behind the bottom (in Orientation 2) x-ray marker **258B, 244** and thus, the top x-ray marker of the second row **258A, 244** is hidden from view. The third row has two points, which represents the x-ray markers located at the distal, unattached distal-pointing crowns **258C, 244** and **258D, 244;** in this x-ray view neither of the x-ray markers of the third row is hidden from view. The fourth row is, as always, a single point, which represents the x-ray marker located in the distal tube **236, 244.** The surgeon, thus, concludes that the second row of x-ray markers **258A, 244** and **258B, 244** (i.e., the x-ray markers at the proximal, unattached distal pointing-crowns) has converged. As shown in FIG. 18E, the surgeon then moves the distal body **216** proximally so that the distal, unattached distal-pointing crowns **258C, 244** and **258D, 244** are immediately distal to the clot **270B.** Unbeknownst to the surgeon, the clot **270B** enters the distal body interior 222 immediately distal to the top (in Orientation 2), proximal unattached distal-pointing crown **258A** and the distal body **216** is no longer collapsed. The surgeon then irradiates the x-markers again from the first vantage point. As shown in FIG. 18F, the first row is, as always, a single point, representing the x-ray marker located in the proximal tube **228, 244.** The second row has two x-ray markers because the distal body **216** is not collapsed and neither the top (in Orientation 2) **258A, 244** nor the bottom **258B, 244** (in Orientation 2) x-ray marker of the second row (i.e., the marker at the proximal, unattached distal-pointing crowns) is hidden from view. The third row has only one point because the rear (in Orientation 2), distal unattached distal- pointing crown **258D, 244** is hidden behind the front (in Orientation 2), distal, unattached distal pointing-crown **258C, 244.** The fourth row is, as always, a single point, representing the x-ray marker located in the distal tube **236, 244.** Without moving the distal body **216,** the surgeon then irradiates the markers from a second vantage point 90 degrees offset from the first vantage point (i.e., from the bottom of the distal body **216**). As shown, the first row is, as always, a single point, which represents the x-ray marker located in the proximal tube **228, 244.** The second row has a single point because the x-ray marker at the top (in Orientation 2), proximal, unattached distal-pointing crown **258A, 244** is hidden behind the bottom (in Orientation 2), proximal, unattached-distal pointing crown **258B, 244.** The third row has two points because neither the front nor the rear x-ray markers at the distal, unattached, distal-pointing crowns **258C, 244** and **258D, 244** is hidden from view. The fourth row is, as always, a single point, which represents the x-ray marker located in the distal tube **236, 244.** Based on the information from FIGs. **18D** and 18F, the surgeon concludes that the clot **270B** has entered into the distal body interior **222.** The surgeon then removes the distal body **216** and the hard clot **270B,** captured by the distal body **216,** by moving the distal body **216** proximally out of the vessel **266,** as shown in FIG. 18G. Upon comparing FIGs. 16A-G and FIGs. 18A-G it will be appreciated that the orientation of the enlarged cells/drop zone **262A-D** relative to the orientation of a hard clot **270B** determine which enlarged cell/drop zone **262A, 262B, 262C,** or **262D,** the hard clot **270** enters the distal body interior **222** through. For example, in FIG. 16C, the hard clot **270B** is located above the distal body **216,** and thus, the hard clot **270B** must enter through the enlarged cell/drop zone located at the top of the distal body, which in the orientation of the distal body shown in **FIGs. 16A-G**, is the enlarged cell/drop zone **262C** immediately distal to the top, distal, unattached, distal-pointing crown **258C.** In FIG. 18C, the hard clot **270B** is again located above the distal body and, thus, the hard clot **270B** must enter through the enlarged cell/drop zone located at the top of the distal body. However, in FIG. 18C, the enlarged cell/drop zone located at the top of the distal body **216,** in the orientation of the distal body **216** shown in FIGs. 18A-G, is the enlarged cell/drop zone **262A** immediately distal to the top, proximal, unattached, distal-pointing crown **258A.**

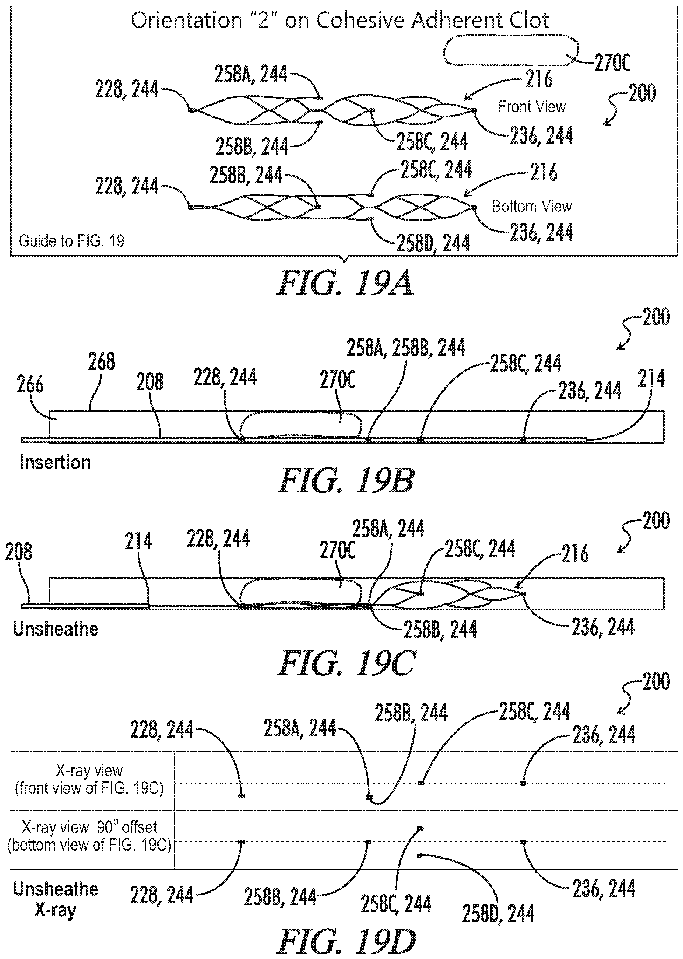

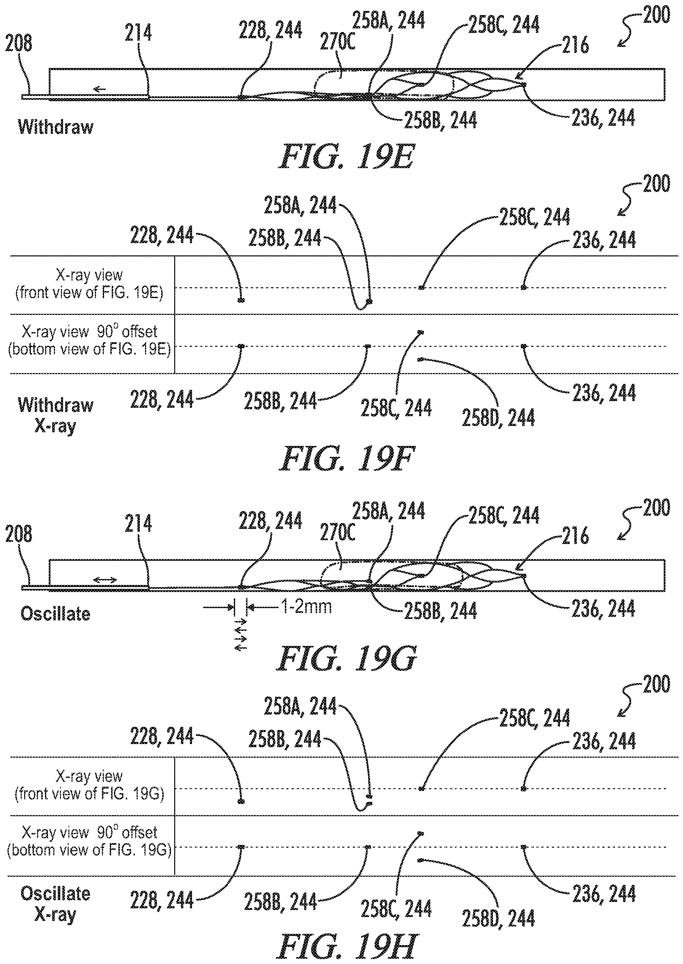

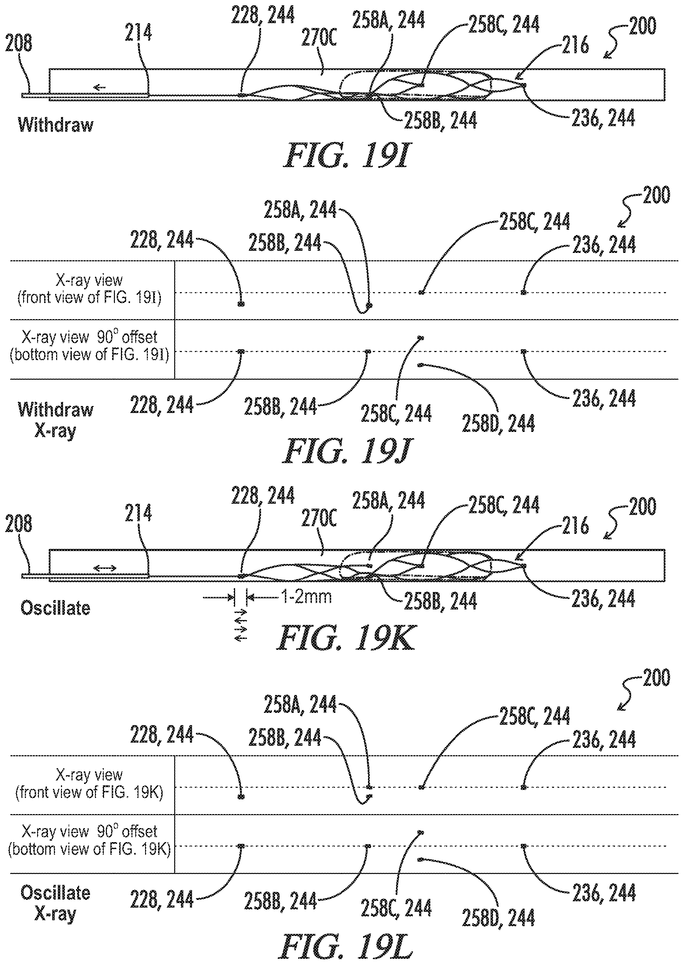

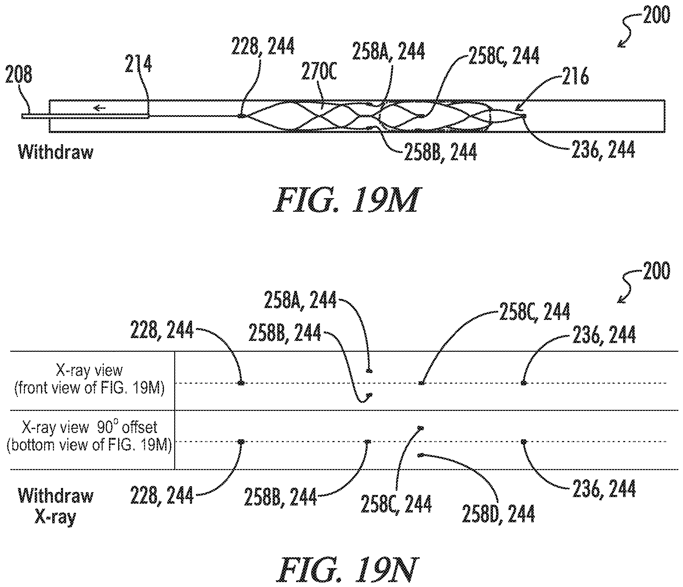

FIGs. 19A-N illustrate stepwise use of the distal body **216** in retrieving a deformable cohesive, adherent clot **270C-** i.e., a clot that is difficult to break up and is tightly adhered to the vessel wall **268** - in a human intracranial artery **266.** (In FIGS. 19A-N, the distal body **216** is in Orientation 2). First, as always, the surgeon determines the location of the clot **270C** in the vessel **266** using, for example, a contrast dye injected proximal and distal to the clot **270C.** Next, the delivery catheter **208,** which is enveloping the distal body **216,** is positioned in the blood vessel **266** so that the two proximal, unattached distal-pointing crowns **258A** and **258B** are immediately distal to the clot **270C.** See FIG. 19B. The distal body **216** is then deployed from the catheter **208** by moving the catheter **208** proximally. The deformable, cohesive adherent clot **270C,** which is located above the distal body **216,** collapses the distal body **216,** as shown in FIG. 19C. However, at this time, the surgeon is unaware that the clot **270C** has collapsed the distal body **216.** Thus, without moving the distal body **216,** the surgeon irradiates the x-ray markers at a first vantage point (i.e., from the front of the distal body; i.e., into the page). As shown in FIG. 19D, the first vantage point shows four rows of x-ray markers. The first row is, as always, a single point, representing the x-ray marker located in the proximal tube **228, 244.** The second row has a single point, corresponding to the top (in Orientation 2) and bottom (in Orientation 2), proximal, unattached distal-pointing crowns **258A, 244** and **258B, 244,** which have converged because the clot **270C** is collapsing the distal body **216.** The third row has a single point, which represents the x-ray marker located at the front (in Orientation 2), distal, unattached distal-pointing crown **258C, 244;** the x-ray marker located at the rear, distal, unattached distal-pointing crown **258D, 244** is hidden from view. The fourth row is, as always, a single point, representing the x-ray marker located in the distal tube **236, 244.** Without moving the distal body **216,** the surgeon then irradiates the markers from a second vantage point 90 degrees offset from the first vantage point (i.e., from the bottom of the distal body). As shown, the first row is, as always, a single point, which represents the x-ray marker located in the proximal tube **228, 244.** The second row has a single point, which corresponds to the bottom (in Orientation 2), proximal, unattached distal-pointing crown **258B, 244;** the top (in Orientation 2), proximal, unattached distal-pointing crown **258A, 244** is located behind the bottom, proximal, unattached distal-pointing crown **258B, 244** and hidden from view. The third row has two points, which correspond to the front (in Orientation 2) **258C, 244** and rear **258D, 244** (in Orientation 2), distal, unattached distal-pointing crowns, neither of which is blocked in this view. The fourth row is, as always, a single point, which represents the x-ray marker located in the distal tube **236, 244.** As shown in FIG. 19E, the surgeon then moves the distal body **216** proximally (i.e., slightly withdraws the distal body **216**). The surgeon then irradiates the x- markers again from the first and second vantage points. As shown in FIG. 19F, the results are exactly the same as in FIG. 19D. Based on the observation that the proximal, unattached distal- pointing crowns **258A, 244** and **258B, 244** have converged at both the original position (FIGs. 19C and 19D in which the proximal, unattached distal-pointing crowns **258A, 244** and **258B, 244** are immediately distal to the clot **270C**) and the second position (FIGs. 19E and 19F), the surgeon concludes that the clot **270C** is a deformable cohesive, adherent clot **270C.** The surgeon then oscillates the distal body **216** proximally and distally a small distance (e.g., about 1mm to about 2 mm) in the vessel **266,** and the clot **270C** begins to enter the distal body **216,** as shown in FIG. 19G. The surgeon then irradiates the x-markers again from the first and second vantage points. As shown in FIG. 19H, the results are exactly the same as in FIG. 19D and FIG. 19F except that the second row of markers **258A, 244** and **258B, 244** (at the proximal, unattached distal-pointing crowns) are beginning to move apart. The surgeon then moves the distal body **216** proximally again, as shown in FIG. 19I. The surgeon then irradiates the x-markers again from the first and second vantage points. As shown in FIG. 19J, the results are exactly the same as in FIGs. 19D and 19F, as the clot **270C** has caused the second row of markers **258A, 244** and **258B, 244** to re-converge. The surgeon then oscillates the distal body **216** proximally and distally a small distance (e.g., about 1mm to about 2 mm) in the vessel **266,** and the clot **270C** begins to further enter the distal body interior **222,** as shown in FIG. 19K. The surgeon then irradiates the x-markers again from the first and second vantage points. As shown in FIG. 19L, the results are the same as in FIG. 19H. The surgeon then moves the distal body **216** again proximally, and, instead of collapsing the second row of markers **258A, 244** and **258B, 244,** the clot **270C** fully enters the distal body interior **222,** as shown in FIG. 19M. The surgeon then irradiates the x-markers again from the first and second vantage points. As shown in FIG. 19N, the results show that the second row of markers **258A, 244** and **258B, 244** (at the proximal, unattached distal-pointing crowns) have moved apart. Satisfied that the x-ray markers in the second row **258A, 244** and **258B, 244** (at the proximal, unattached distal-pointing crowns) are sufficiently far apart and that the x-ray markers in the third row (at the distal, unattached distal- pointing crowns) **258C, 244** and **258D, 244** have stayed far apart, the surgeon concludes that the deformable cohesive, adherent clot **270C** has been sufficiently captured by the distal body **216** and the surgeon then removes the distal body **216** and the clot **270C,** captured by the distal body **216,** by moving the distal body **216** proximally out of the vessel **266.**

Several observations can be made from FIGs. 15-19, as indicated above. For example, the x-ray markers at the proximal and distal, unattached distal-pointing crowns **258A- D, 244** provide the surgeon feedback concerning the interaction between the distal body **216** and the clot **270** in the blood vessel **266.** In addition, the guiding principle of a soft clot **270A** is that the soft clot **270A** does not collapse the distal body **216,** and thus, x-ray markers at the proximal and distal, unattached distal-pointing crowns **258A-D, 244** always appear as two points except when a marker is hidden behind another marker (due to the view). When it comes to a hard clot **270B,** the hard clot **270B** is generally able to enter the distal body interior **222** without needing to oscillate the distal body **216** proximally and distally (unlike a deformable cohesive, adherent clot **270C**). However, to capture the hard clot **270B,** the hard clot **270B** must be oriented properly relative to the enlarged cell/drop zones **262A, 262B, 262C,** or **262D.** (This is the reason that the distal body **216** has four enlarged cells/drop zones: one enlarged cells/drop zone at 0 degrees **262B,** one enlarged cells/drop zone at 90 degrees **262C,** one enlarged cells/drop zone at 180 degrees **262A** and one enlarged cells/drop zone at 270 degrees **262D**). As a guiding principle, an enlarged cell/drop zone **262A, 262B, 262C,** or **262D** is properly oriented to the clot **270B** when the x-ray markers at the proximal, unattached distal-pointing crowns **258A, 244** and **258B, 244** or the distal, unattached distal pointing crowns **258C, 244** and **258D, 244** are together at both a first x-ray view and a second x-ray view 90 degrees relative to the first x-ray view, and the hard clot **270B** can enter the enlarged cell/drop zone **262A, 262B, 262C,** or **262D** by moving the distal body **216** proximally. See FIG. 16F and 18D. Finally, the guiding principal of retrieval of deformable cohesive, adherent clots **270C** is that oscillation of the distal body **216** causes the deformable cohesive, adherent clots **270C** to gradually enter the distal basket interior **222** over time.

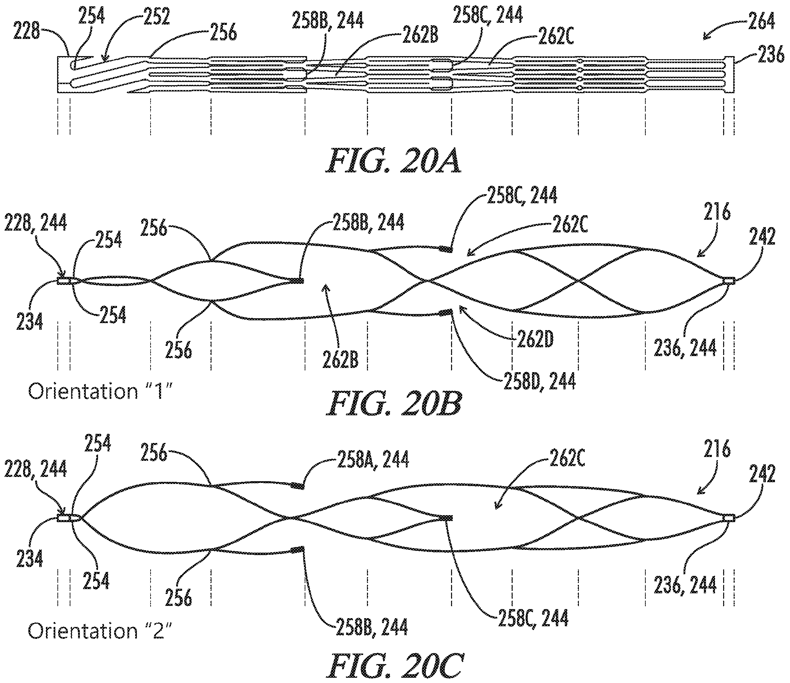

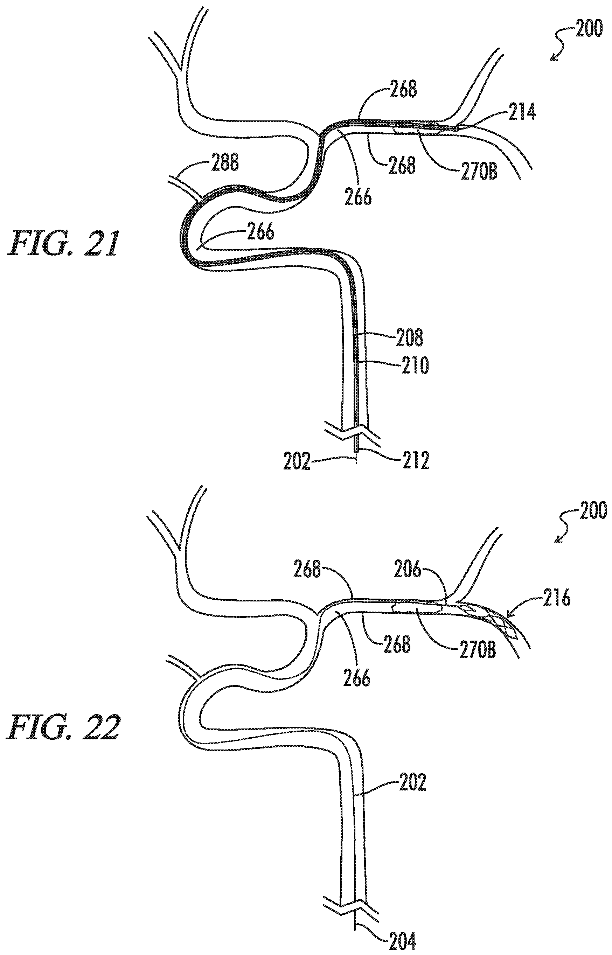

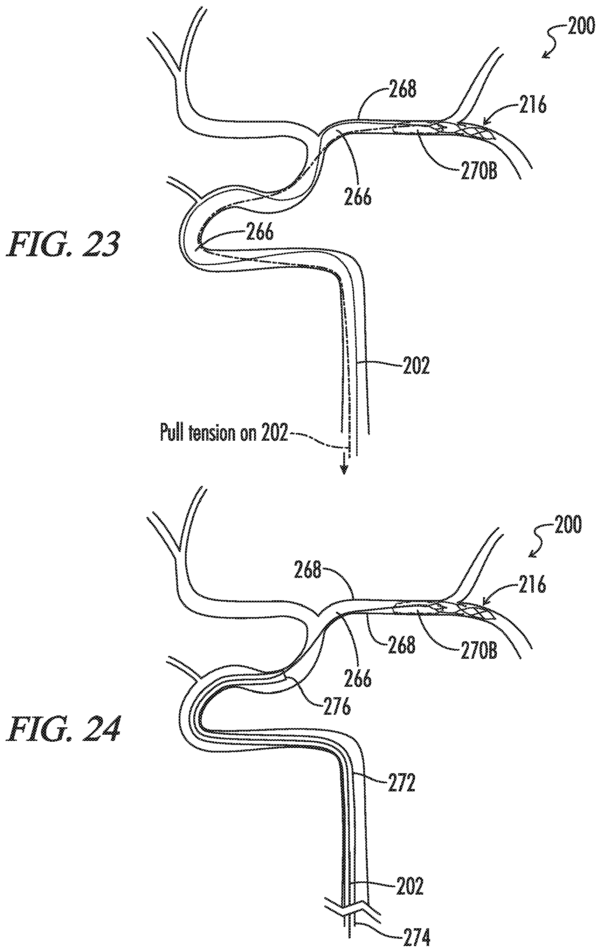

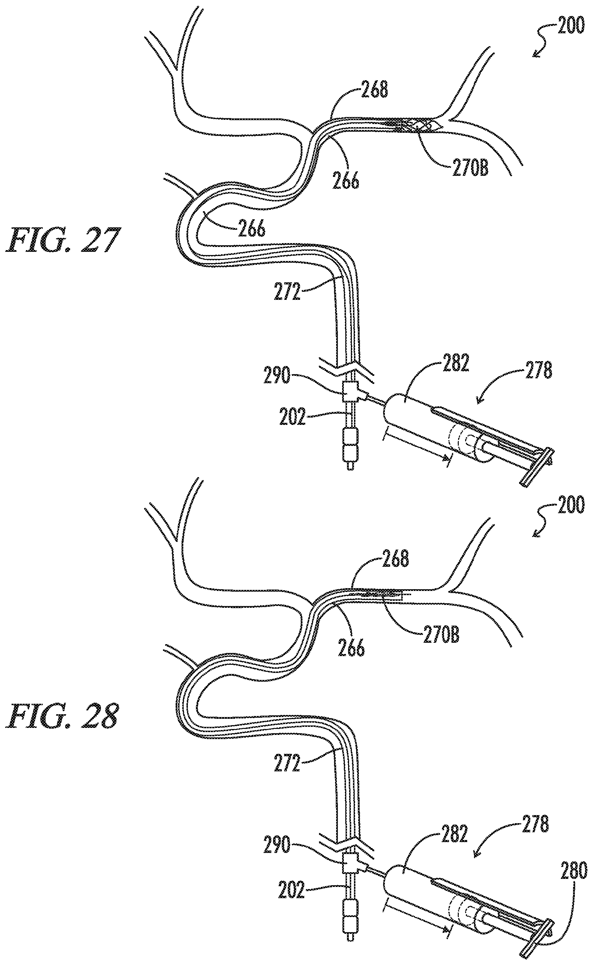

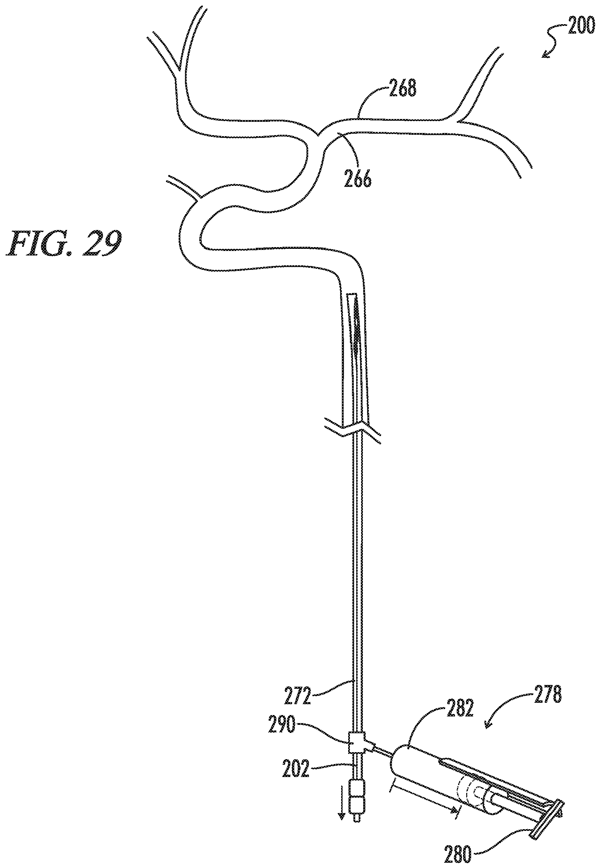

FIGs. 20A, 20B and 20C show a distal body **216** that is similar to the distal body **216** of FIGs. 14A, 14B and 14C except that the distal body **216** of FIGs. 20A, 20B and 20C is slightly shorter and its unattached, distal-pointing crowns **258A, 258B, 258C,** and **258D** are closer to the proximal tube **228.** The shortened distal body **216** of FIGs. 20A, 20B and 20C is particularly adapted for tortuous blood vessels **266.** FIG. 21-29 show stepwise deployment of the distal body **216** of FIGs. 20A, 20B and 20C in use with a manual (i.e., hand-operated), volume-dependent (i.e. volume locked) suction catheter **272** that is locked at between about 10 to about 60 cubic centimeters (cc). Optionally, the suction catheter **272** has an outer diameter of between about 0.05 inches and about 0.09 inches and its outer diameter is substantially larger than the outer diameter of the delivery catheter **208.** The clot **270** is located in the vessel **266** through the use of, for example, contrast dye injected proximal and distal to the clot **270.** As shown in FIG. 21, a delivery catheter **208** containing the distal body **216** of FIGs. 20A, 20B and 20C is positioned in the tortuous vessel **266** distal to the clot **270.** The delivery catheter **208** is withdrawn, deploying the distal body **216.** See FIG. 22. The distal body **216** is moved proximally relative to the clot **270** and tension is exerted on pull wire 202. See FIG. 23. While maintaining tension on the pull wire **202,** a suction catheter **272** having a proximal end **274** and a distal end **276** is delivered over the pull wire **202** that is attached to the distal body **216.** See FIG. 24. (The reason for exerting tension on the pull wire **202** is that the pull wire **202** serves as the guide/track for the movement of the suction catheter **272** and without tension, the suction catheter **272** and pull wire **202** could end up in the ophthalmic artery **288**). The distal end **276** of the suction catheter **272** is positioned against the clot **270.** A syringe **278** is attached to the suction catheter **272** using a rotating hemostatic valve **290,** which allows the surgeon to aspirate while a pull wire **202** is in the system. The surgeon aspirates the syringe **278** by pulling back on the lever **280** to a mark on the base **282** corresponding to between about 10 and about 60 cubic centimeters of fluid. The surgeon then locks the lever **280** (and attached plunger) into place, leaving the suction catheter **272** under suction. The surgeon captures the clot **270** in the distal body **216** using the techniques described in FIGs. 15-19. The distal body **216** and clot **270** become captured by the suction catheter **272.** See FIGs. 27 and 28. The surgeon then removes the suction catheter **272** and the distal body **216** and the clot **270,** captured by the suction catheter **272,** by moving the suction catheter **272** proximally out of the vessel **266.** See FIG. 29. It is believed that the suction catheter **272** would be helpful in the event that a small portion of the clot **270** breaks off when retrieving the clot **270** using the distal body **216.**

To examine effectiveness of the systems **200,** the systems **200** of FIGs. 11-20, without the use of a suction catheter **272,** were used to retrieve soft and hard clots **270A** and **270B** induced in a pig weighing between 30 to 50 kg. The weight of the pig was chosen so that the size of its vessels **266** would be approximate to the size of a human vessel. The pig was anesthetized. Several hard clots **270B** were prepared by mixing pig blood and barium and incubating the mixture for 2 hours. Several soft clots **270A** were prepared by mixing pig blood, thrombin and barium and incubating the mixture for 1 hour. The clots **270A** and **270B,** each of which had a width of 4 to 6 mm and a length of 10 to 40 mm, were then inserted into a vessel **266** having a diameter of 2 to 4 mm. (Only one clot **270A** and **270B** was located in the vessel **266** at a time). Angiograms were then performed to confirm occlusion. After waiting ten minutes after confirming occlusion, the distal bodies **216** of FIGs. 11-20 were then delivered distal to the clots **270A** and **270B** as described above and were used to retrieve the clots **270A** and **270B** as described in FIGs. 11-19. In each case, the distal bodies **216** were successful in retrieving the clots **270A** and **270B.**

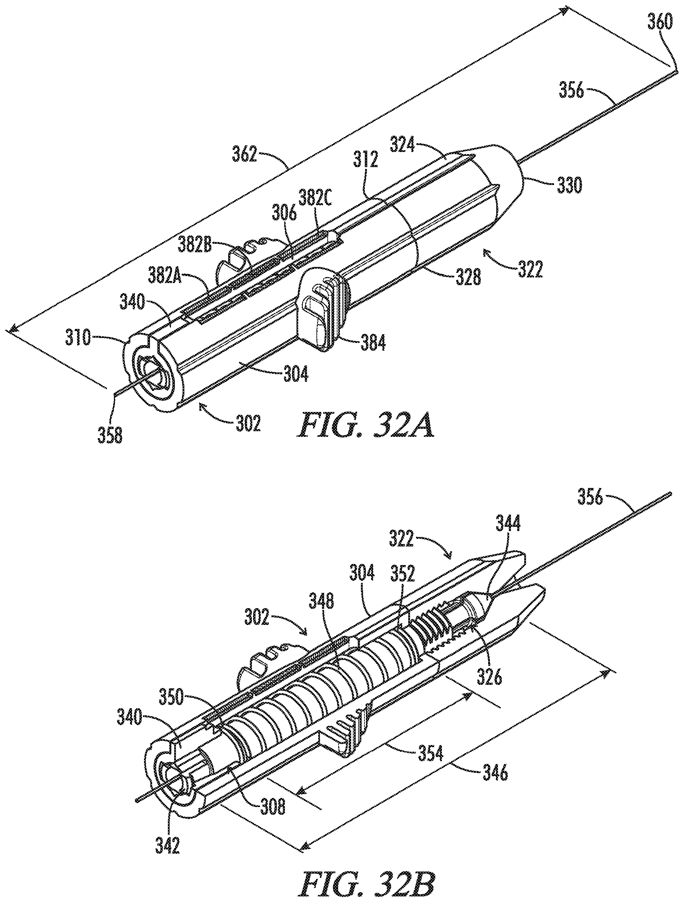

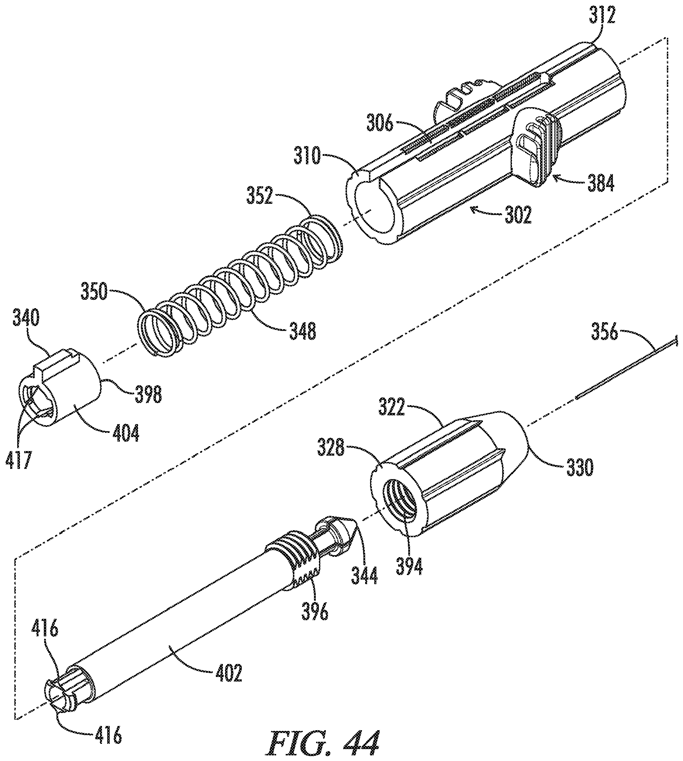

### The Embodiments of FIGs. 32-45

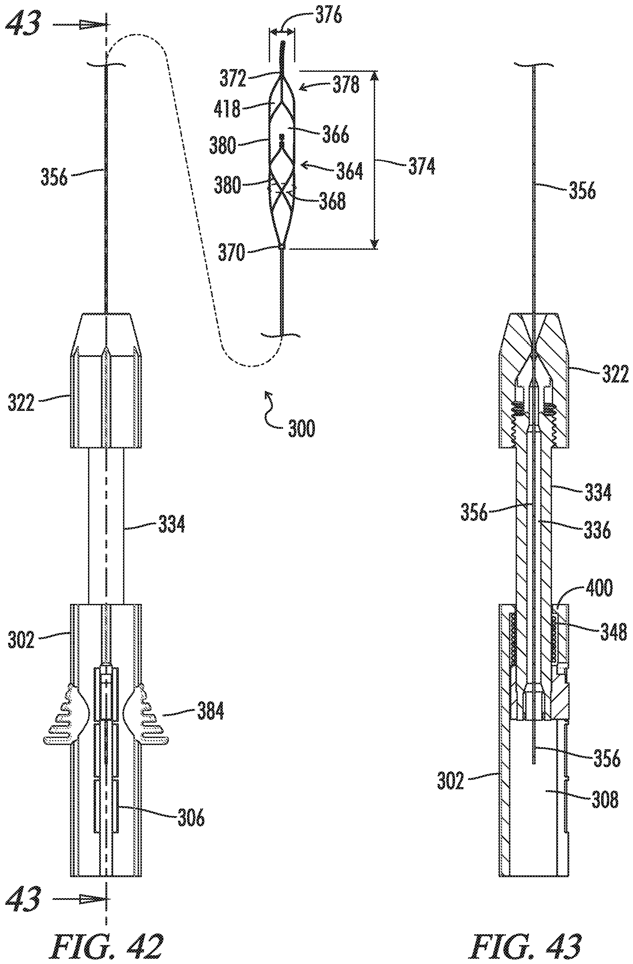

FIGs. 32-41 and 43-45 illustrate a strain gauge that may be used with any suitable stent retriever, such as (as is illustrated in FIG. 42) the distal body of FIGs. 11-20, which is currently available under the trade name NEVA from Legacy Ventures LLC dba Vesalio (Nashville, Tennessee). Alternatively, for example, the SOLITAIRE revascularization device (Medtronic, Minneapolis, Minnesota), or TREVO retriever (Stryker, Kalamazoo, Michigan) or other suitable stent retrievers may be used with the strain gauge. Additional stent retrievers are described in U.S. Patent Publication 2018/0296235. Optionally, the strain gauge includes a clamp **392** that releasably engages the pull wire **356,** which in turn is attached to a distal body **364,** as shown in FIGs. 32-45. As described more particularly below, the strain gauge illustrated in FIGs. 32-45 is comprised of a proximal outer tube **302,** a distal outer tube **322** and an inner tube **334.**

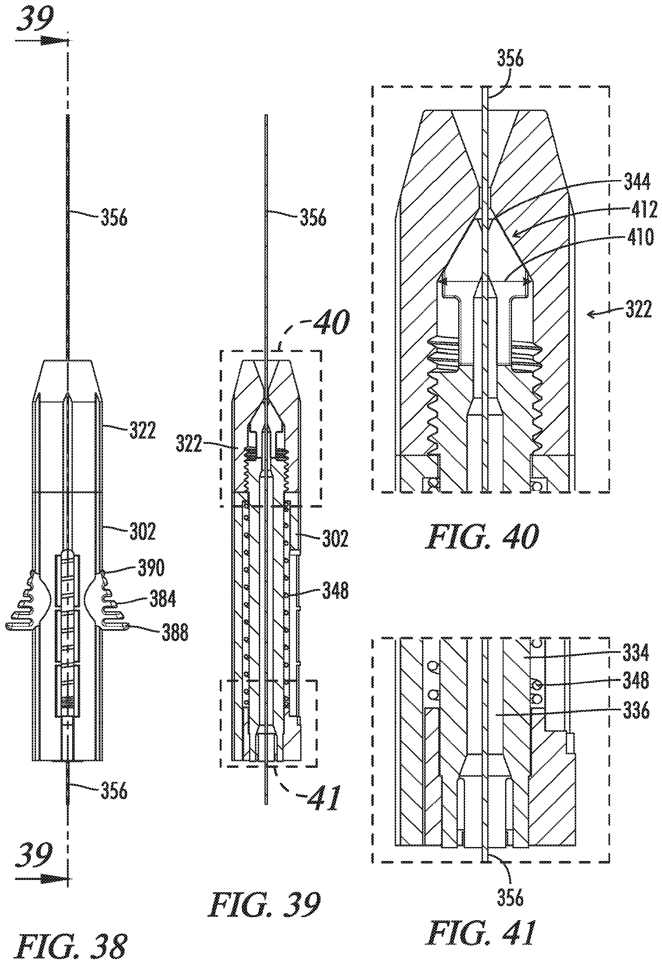

More particularly, in some embodiments, as shown in FIGs. 32-45, the present disclosure provides a system **300** for removing a blood clot from a human blood vessel that includes a proximal outer tube **302** comprising a proximal outer tube exterior **304** comprising a slot **306,** a proximal outer tube interior **308,** a proximal outer tube proximal end **310,** a proximal outer tube distal end **312,** and a proximal outer tube length **314** extending from the proximal outer tube proximal end **310** to the proximal outer tube distal end **312.** The proximal outer tube **302** serves as the handle and is held by the surgeon's hand and may include two handle flanges **384** comprising grooves **386** located on opposite sides of the proximal outer tube exterior **304** (e.g., located approximately 180 degrees apart). The surgeon's fingers may engage the handle flanges **384.** Optionally, the handle flanges **384** have a proximal end **388** and a distal end **390** and the handle flanges **384** extend laterally from the proximal outer tube **302** to a greater extent at the proximal end **388** of the handle flanges **384** as compared to the distal end **390** of the handle flanges **384** so that the handle flanges **384** are sloped, as best seen in FIG. 38.

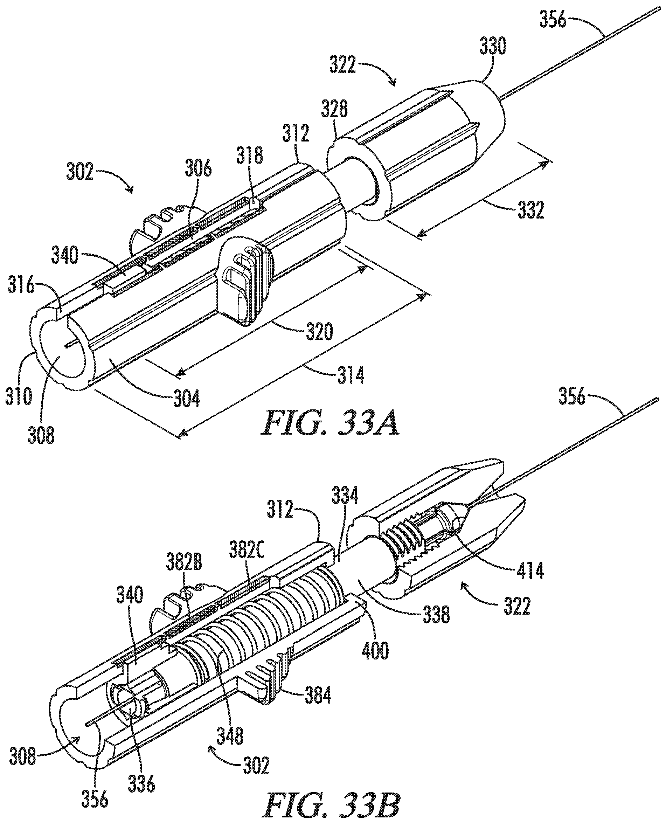

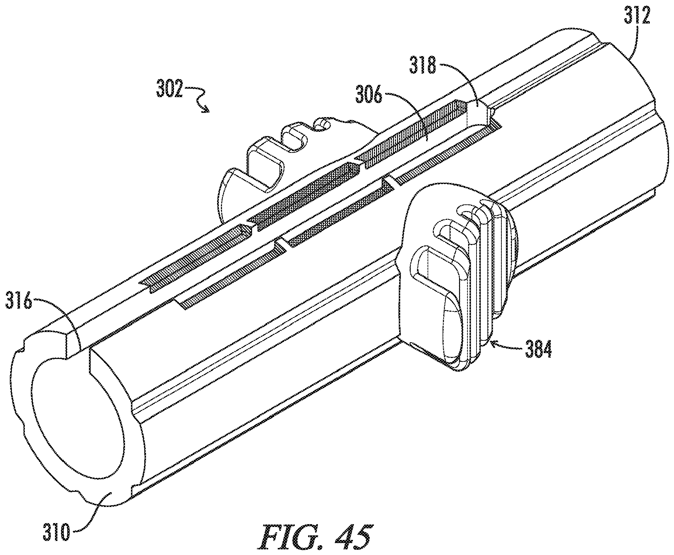

The slot **306** comprises a slot proximal end **316,** a slot distal end **318,** and a slot length **320** extending from the slot proximal end **316** to the slot distal end **318** and parallel to the proximal outer tube length **314.** Optionally, the slot proximal end **316** extends to the proximal end **310** of the proximal outer tube **302** and the slot distal end **318** is located proximal to the distal end **312** of the proximal outer tube **302,** as shown in FIG. 45 for example. In other words, the slot length **320** may be less than the proximal outer tube length **314,** as shown in FIG. 33A for example.

Optionally, the system **300** further includes a distal outer tube/tightening collar **322** comprising a distal outer tube exterior **324,** a distal outer tube interior **326,** a distal outer tube proximal end **328** located distal to the proximal outer tube distal end **312,** a distal outer tube distal end **330,** a distal outer tube length **332** extending from the distal outer tube proximal end **328** to the distal outer tube distal end **330.** Optionally, the distal outer tube proximal end **328** is not attached to the proximal outer tube distal end **312** so that the proximal outer tube **302** can move proximally relative to the distal outer tube **322,** as shown in FIGs. 33A, 33B, 34A, 34B, 35A, 35B, 42, 43, for example, as tension is placed on the pull wire **356,** as described below.

The system **300** further includes an inner tube/stem **334** extending from the proximal outer tube interior **308** to the distal outer tube interior **326,** the inner tube **334** comprising an inner tube interior **336,** an inner tube exterior **338** comprising a fin **340** (which in turn is positioned in the proximal outer tube slot **306**), an inner tube proximal end **342,** an inner tube distal end **344** and an inner tube length **346** extending from the inner tube proximal end **342** to the inner tube distal end **344.** The inner tube **334** has a smaller height and width than the height and width of the proximal outer tube **302** and the distal outer tube **322.**

The system **300** further includes a compressible spring **348** having a spring proximal end **350,** a spring distal end **352,** and a spring length **354** extending from the spring proximal end **350** to the spring distal end **352.**

The system **300** further includes a pull wire **356** having a pull wire proximal end **358** that optionally is located proximal to the proximal outer tube proximal end **310** and a pull wire distal end **360** that is optionally located distal to the distal outer tube distal end **330.**

Optionally, the proximal outer tube proximal end **310,** the distal outer tube distal end **330,** the inner tube proximal and distal ends **342** and **344** are open to allow the pull wire **356** to pass therethrough, the proximal outer tube distal end **312** and the distal outer tube proximal end **328** are open to allow the inner tube **334** (and the pull wire **356** contained therein) to pass therethrough, the inner tube interior **336** is hollow to accommodate the pull wire **356** and the proximal and distal outer tubes interiors **308** and **326** are hollow to accommodate the inner tube **334** and pull wire **356.**

Optionally, the pull wire **356** extends through the inner tube interior, proximal end and distal end **336, 342** and **344,** as well as the proximal outer tube interior, proximal end, and distal end **308, 310** and **312** and distal outer tube interior, proximal end and distal end **326, 328** and **330.**

The system **300** further includes a distal body **364** located distal to the distal outer tube **322** and that may include a distal body interior **366,** a distal body perimeter **368,** a distal body proximal end **370** connected to the pull wire **356,** a distal body distal end **372,** a distal body length **374** extending from the distal body proximal end **370** to the distal body distal end **372,** and a distal body height (not labelled) and width **376** perpendicular to the distal body length **374,** the distal body **364** comprising a framework **378** comprised of a plurality of cells **418** formed by a plurality of memory metal strips **380.** The distal body **364** has a relaxed state wherein the distal body **364** has a first height and a first width **376,** and a collapsed state wherein the distal body **364** has a second height and a second width **376,** the second height of the distal body **364** less than the first height of the distal body **364,** the second width of the distal body **364** less than the first width of the distal body **364.** As noted above, preferably, the distal body **364** is a stent retriever.

Optionally, the inner tube **334** (and attached fin **340**) is attached to the distal outer tube **322** and automatically moves along with the distal outer tube **322,** (as well as the pull wire **356,** and the distal body **364** when the clamp **392** mentioned below is in the closed position) in the lengthwise direction. In other words, when the clamp **392** is in the closed position, pulling the distal outer tube **322** proximally automatically moves the inner tube **334** (and attached fin **340**), pull wire **356** and the distal body **364** proximally, and similarly, pushing the distal outer tube **322** distally automatically moves the inner tube **334** (and attached fin **340**), pull wire **356,** and the distal body **364** distally.

Optionally, when the clamp **392** is in the closed position, the proximal outer tube **302** is configured to move proximally for a pre-determined distance relative to the inner tube **334,** distal outer tube **322,** the pull wire **356** and the distal body **364** in the lengthwise direction so the proximal outer tube **302** moves from a position in which the distal end of the proximal outer tube **312** abuts the proximal end of the distal outer tube **322,** as shown in FIGs. 32A, 32B and 38, for example, to a position in which the distal end of the proximal outer tube **312** does not abut the proximal end of the distal outer tube **328,** as shown in FIGs. 33A, 33B, 34A, 34B, 35A, 35B, 42, 43, for example.

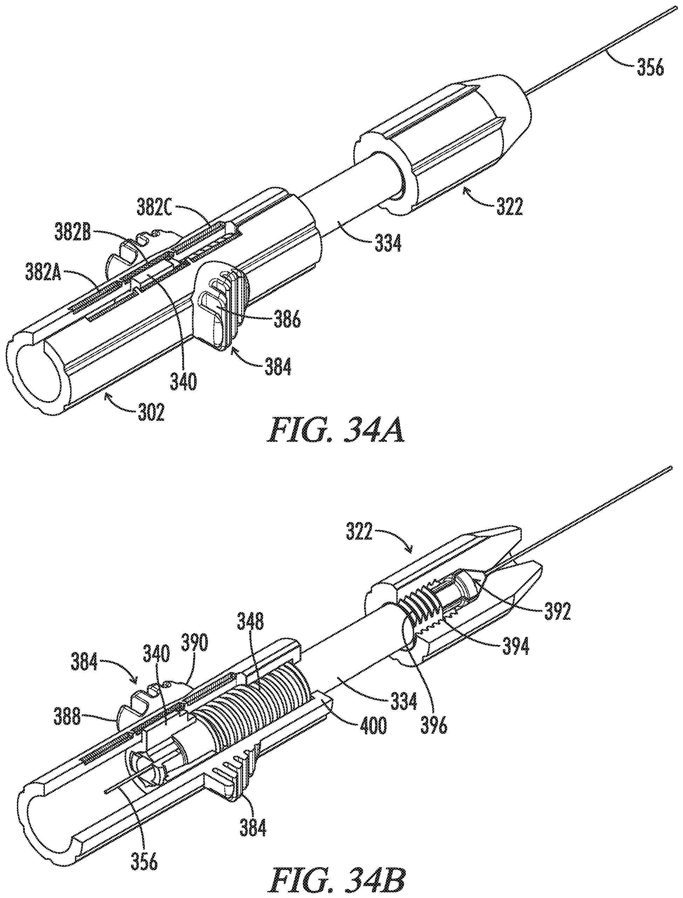

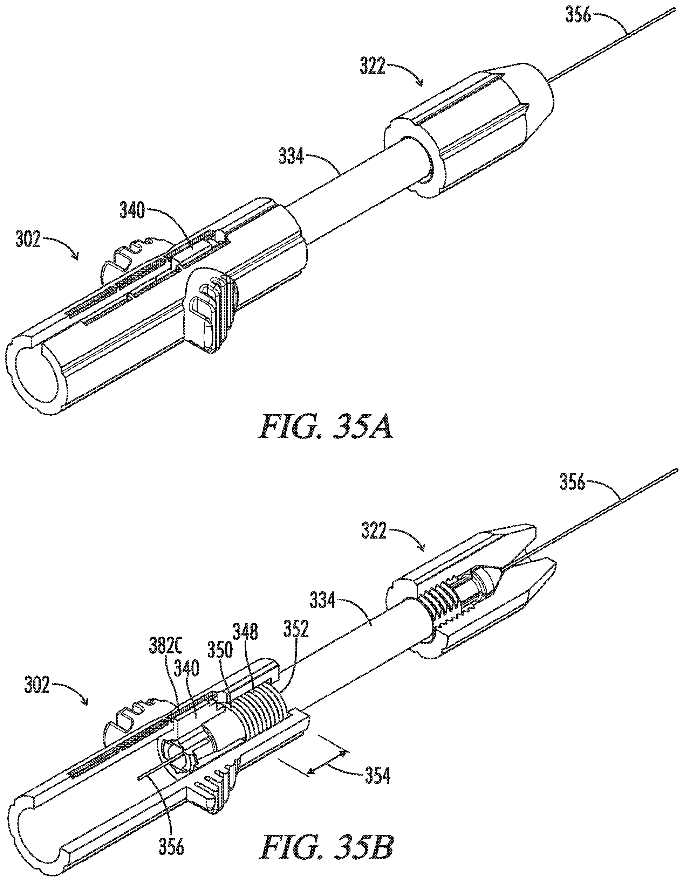

Optionally, moving the proximal outer tube **302** proximally relative to the distal outer tube **322,** the inner tube **334,** the pull wire **356** and the distal body **364** causes the proximal outer tube slot **306** to move proximally relative to the fin **340** and causes the spring **348** to move from a relaxed length to a compressed length. Such movement allows the fin **340** to move relative to proximal outer tube indicia **382A, 382B,** and **382C,** which are adjacent to the slot **306.** For example, the proximal-most indicia **382A** may represent the least resistance on the pull wire **356** and may be labelled green, indicating to the surgeon that when the fin **340** is adjacent or proximal to the proximal-most indicia **382A** (as shown in FIGs. 32A and 33A, for example) the surgeon is able to freely move the proximal outer tube **302** proximally with minimal caution (and thereby the system **300** proximally). Similarly, the middle indicia **382B** may represent a medium level of resistance on the pull wire **356** and may be labelled yellow, indicating to the surgeon that when the fin **340** is adjacent to the middle indicia **382B** (as shown in FIG. 34A, for example) the surgeon should exercise caution when moving the proximal outer tube **302** proximally (and thereby the system **300** proximally), because of the increased resistance on the pull wire **356.** Finally, the distal indicia **382C** may represent the most resistance on the pull wire **356** and may be labelled red, indicating to the surgeon that when the fin **340** is adjacent to the distal indicia **382C** (as shown in FIG. 35A, for example) the surgeon should exercise more caution when moving the proximal outer tube **302** proximally (and thereby the system **300** proximally), because of the increased resistance on the pull wire **356** may indicate that distal body **364** is caught on something in the human's vasculature system. Thus, a feature of the system **300** is to provide tensile feedback to the surgeon as the surgeon pulls the system **300** proximally so that the surgeon does not break the pull wire **356** or cause the pull wire **356** to separate from distal body **364** or damage the vessel and the indicia **382A-382C** may be in the form of colors to indicate various tension zones. One pound corresponds to 0.45 kilograms. The proximal indicia **382A** may be calibrated at, for example, 01.49 pounds of tension, the middle indicia **382B** may be calibrated at, for example, 1.5-2.99 pounds of tension and the distal indicia **382C** may be calibrated at, for example, over 3 pounds of tension.

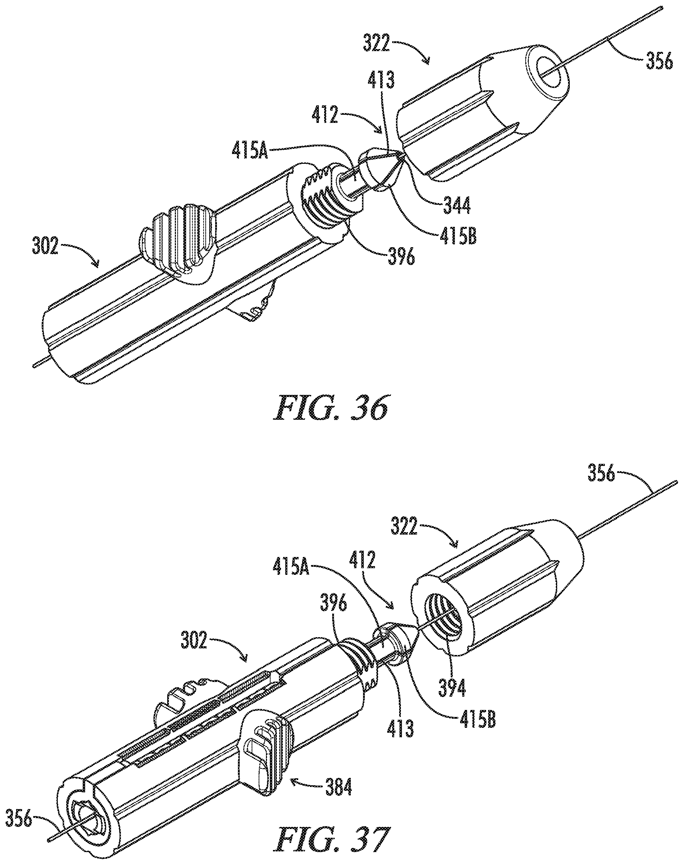

Optionally, at least one of the distal outer tube **322** and the inner tube distal end **344** comprises a clamp **392** configured to close around the pull wire **356** and releasably engage the pull wire **356.** For example, optionally the clamp **392** is comprised of two or more clamping segments **415A** and **415B** with the outer surface of the clamping segments **415A** and **415B** having two or more slits **413** extending therethrough and formed into an inner tube tapered portion **412.** Optionally the distal outer tube interior **326** comprises inner tube threads **396** and a conical tapered interior surface **414.** For example, when the conical tapered interior surface **414** of the distal outer tube **322** is drawn into contact with the inner tube tapered portion **412,** the clamping segments **415A** and **415B** are forced to collapse and clamp onto pull wire **356.** Optionally the inner tube **334** comprises an inner tube height (not labelled) and an inner tube width **410,** and the inner tube distal end **344** comprises a inner tube tapered portion **412** in which the inner tube height and width **410** decrease at the inner tube distal end **344,** as shown in FIGs. 36 and 40 for example. Additionally, optionally, the distal outer tube interior **326** and the inner tube **334** comprise mating threads, wherein moving the inner tube threads **396** along the distal outer tube interior threads **394** in a first direction causes the conical tapered interior surface/wall **414** of the distal outer tube **322** to contact and apply pressure to the inner tube tapered portion **412** which closes the clamp **392** around the pull wire **356** and further wherein moving the inner tube threads **396** along the distal outer tube interior threads **394** in a second direction causes the conical tapered interior surface/wall **414** to move away from and release pressure on the inner tube tapered portion **412** which opens the clamp **392** with respect to the pull wire **356.** For example, rotating the distal outer tube **322** clockwise while holding the proximal outer tube **302** stationary may tighten the clamping segments **415A** and **415B** around the pull wire **356** (a clamp closed position), whereas rotating the distal outer tube **322** counterclockwise while holding the proximal outer tube **302** stationary may loosen the clamping segments **415A** and **415B** around the pull wire **356** allowing the proximal outer tube **302** and distal outer tube **322** to be moved off the pull wire **356** (a clamp open position). In other words, in the open position, the proximal outer tube **302,** the distal outer tube **322** and the inner tube **334** may freely slide proximally and distally over the pull wire **356.** It will be appreciated that the slits **413** allow the clamping segments **415A** and **415B** to radially expand to create the clamp open position and to contract to create the clamp closed position. To use the system **300,** the surgeon may move the distal outer tube **322** and the proximal outer tube **302** (and attached inner tube **334**) distally over the pull wire **356** (from the pull wire proximal end **358**) so that the pull wire **356** is positioned in the distal outer tube and proximal outer tube interiors **326, 308** and then rotate the distal outer tube **322** clockwise while holding the proximal outer tube **302** stationary thereby forcing the clamping segments **415A** and **415B** to tighten and grip onto the pull wire **356** and thus fixing the proximal outer tube **302** and distal outer tube **322** on the pull wire **356.** The inner tube **334** preferably does not rotate relative to the proximal outer tube **302** due to the fact that the fin **340** is located in the slot **306** and the splines **416** described below engage the spline grooves **417** of collar **404.**

Another feature of the system **300** is that pull wires **356** are traditionally thin and thus difficult for the surgeon to grip and twist. Thus, the proximal outer tube **302,** which is wider and taller than the pull wire **356,** allows the user, when the clamp **392** is in the closed position, to more readily rotate the proximal outer tube **302** (and thereby the distal outer tube **322,** which is attached to the pull wire **356** through clamp **392,** which in turn is attached to the distal body **364**) about the proximal outer tube length/longitudinal axis **314** in a clockwise and counterclockwise manner, thereby allowing the distal body **364** to rotate in the blood vessel if needed to capture the clot.

Optionally, the spring proximal end **350** rests against the collar distal end **398** located in the proximal outer tube interior **308** and connected to the fin **340** and the spring distal end **352** rests against a distal ledge 400 located distal to the fin **340** in the proximal outer tube interior **308.**

Optionally, as shown in FIGs. 32B, 33B, 34B, 35B and 44, for example, the inner tube **334** is comprised of a rod **402** and a collar **404** enveloping the rod **402** and the collar **404** comprises the fin **340.** Additionally, optionally, the spine grooves **417** of the collar **404** engage the spines **416** of the rod **402** thus preventing the collar **404** from rotating relative to the rod **402.**

Optionally, the fin **340** is engaged in the slot **306,** thus preventing the collar **404** from rotating relative to the proximal outer tube **302.** Furthermore, as rod **402** and clamp **392** are parts of inner tube **334,** they are connected and as such rotate together. Additionally, if the clamp **392** is collapsed onto/engages pull wire **356** (the clamp **392** is in the closed position), then pull wire **356** 5 cannot rotate relative to clamp **392.** Therefore, through the engagement of the slot **306** with the fin **340** and the engagement of the splines **416** with the spline grooves **417** and the engagement of the clamp **392** with the pull wire **356,** rotation of the proximal outer tube **302** causes rotation of the pull wire **356.**

Optionally, the proximal outer tube **302,** inner tube **334** and distal outer tube **322** 10 are rigid, e.g., comprised of an injection molded plastic.

The system **300** may be used in a method of removing a blood clot from a blood vessel of a human the method comprising the steps of a) providing the system **300;** b) positioning a catheter (not shown but previously described) comprising the distal body **364** in the blood vessel; c) deploying the distal body **364** from the catheter and allowing the height and 15 width **376** of the distal body **364** to increase; d) capturing the blood clot with the distal body **364;**
and e) moving the distal body **364** proximally out of the blood vessel by pulling proximally on the proximal outer tube **302.**

### Part List for FIGs. 32-45

| | |
|---|---|
| system | 300 |
| proximal outer tube | 302 |
| proximal outer tube exterior | 304 |
| slot | 306 |
| proximal outer tube interior | 308 |
| proximal outer tube proximal end | 310 |
| proximal outer tube distal end | 312 |
| proximal outer tube length | 314 |
| slot proximal end | 316 |
| slot distal end | 318 |
| slot length | 320 |
| distal outer tube | 322 |
| distal outer tube exterior | 324 |
| distal outer tube interior | 326 |
| distal outer tube proximal end | 328 |
| distal outer tube distal end | 330 |
| distal outer tube length | 332 |
| inner tube | 334 |
| inner tube interior | 336 |
| inner tube exterior | 338 |
| fin | 340 |
| inner tube proximal end | 342 |
| inner tube distal end | 344 |
| inner tube length | 346 |
| spring | 348 |
| spring proximal end | 350 |
| spring distal end | 352 |
| spring length | 354 |
| pull wire | 356 |
| pull wire proximal end | 358 |
| pull wire distal end | 360 |
| pull wire length | 362 |
| distal body | 364 |
| distal body interior | 366 |
| distal body perimeter | 368 |
| distal body proximal end | 370 |
| distal body distal end | 372 |
| distal body length | 374 |
| distal body width | 376 |
| distal body framework | 378 |
| distal body memory metal strips | 380 |
| indicia | 382A, 382B, 382C |
| handle flange | 384 |
| flange groove | 386 |
| flange proximal end | 388 |
| flange distal end | 390 |
| clamp | 392 |
| distal outer tube threads | 394 |
| inner tube threads | 396 |
| collar distal end | 398 |
| distal ledge | 400 |
| rod | 402 |
| collar | 404 |
| inner tube width | 410 |
| inner tube tapered portion | 412 |
| slits | 413 |
| conical tapered interior surface/wall | 414 |
| clamping segments | 415A and 415B |
| splines | 416 |
| spline grooves | 417 |
| distal body cells | 418 |

Having now described the invention in accordance with the requirements of the patent statutes, those skilled in the art will understand how to make changes and modifications to the disclosed embodiments to meet their specific requirements or conditions. Changes and modifications may be made without departing from the scope of the invention, as defined and limited solely by the following claims. In particular, although the system has been exemplified for use in retrieving blood clots, the system may be used to retrieve other objects from animal lumens. In addition, the steps of any method described herein may be performed in any suitable order and steps may be performed simultaneously if needed.

Terms of degree such as "substantially", "about" and "approximately" as used herein mean a reasonable amount of deviation of the modified term such that the end result is not significantly changed. For example, these terms can be construed as including a deviation of at least ± 5% of the modified term if this deviation would not negate the meaning of the word it modifies. 15

## Claims

1. A system (10; 200; 300) for removing a blood clot from a human blood vessel, the system comprising:
a proximal outer tube (302) comprising a proximal outer tube exterior (304) comprising a slot (306, a proximal outer tube interior (308), a proximal outer tube proximal end (310), a proximal outer tube distal end (312), and a proximal outer tube length (314) extending from the proximal outer tube proximal end to the proximal outer tube distal end, wherein the slot comprises a slot proximal end (316), a slot distal end (318), and a slot length (320) extending from the slot proximal end to the slot distal end and parallel to the proximal outer tube length;
a distal outer tube (322) comprising a distal outer tube exterior (324), a distal outer tube interior (326), a distal outer tube proximal end (328) located distal to the proximal outer tube distal end, a distal outer tube distal end (330), a distal outer tube length (332) extending from the distal outer tube proximal end to the distal outer tube distal end, the distal outer tube proximal end not attached to the proximal outer tube distal end;
an inner tube (334) comprising an inner tube interior (336), an inner tube exterior (338) comprising a fin (340) positioned in the proximal outer tube slot, an inner tube proximal end (342) , an inner tube distal end (344) and an inner tube length (346) extending from the inner tube proximal end to the inner tube distal end;
a pull wire (356) having a pull wire proximal end (358) and a pull wire distal end (360) located distal to the distal outer tube distal end,
a distal body (364) located distal to the distal outer tube and comprising a distal body interior (366), a distal body perimeter (368), a distal body proximal end (370) connected to the pull wire, a distal body distal end (372), a distal body length (374) extending from the distal body proximal end to the distal body distal end, and a distal body height and width (376) perpendicular to the distal body length, the distal body comprising a framework (378) formed by a plurality of memory metal strips (380), wherein the distal body has a relaxed state wherein the distal body has a first height and a first width, and a collapsed state wherein the distal body has a second height and a second width, the second height of the distal body less than the first height of the distal body, the second width of the distal body less than the first width of the distal body,
**characterised in that**:
the inner tube extends from the proximal outer tube interior to the distal outer tube interior;
the system comprises a compressible spring (348) having a spring proximal end (350), a spring distal end (352), and a spring length (354) extending from the spring proximal end (350) to the spring distal end; and
wherein at least one of the distal outer tube and the inner tube comprises a clamp (392) having a closed position in which the clamp closes around the pull wire and an open position in which the clamp is open with respect to the pull wire,
wherein, when the clamp is in the open and closed position, the inner tube is attached to the distal outer tube and moves along with the distal outer tube in the lengthwise direction, wherein, at least when the clamp is in the closed position, the proximal outer tube is configured to move proximally for a pre-determined distance relative to the inner tube, distal outer tube, the pull wire and the distal body in the lengthwise direction,
wherein, at least when the clamp is in the closed position, moving the proximal outer tube proximally relative to the distal outer tube, the inner tube, the pull wire and the distal body causes the proximal outer tube slot to move proximally relative to the fin and causes the spring to move from a relaxed length to a compressed length.

2. The system of claim 1 wherein, when the clamp (392) is in the closed position, rotating the proximal outer tube (302) about the proximal outer tube length (314) in a clockwise and counterclockwise direction is configured to rotate the distal outer tube (322), the pull wire (356) and the distal body (364).

3. The system of claim 2 wherein, when the clamp (392) is in the open position, rotating the proximal outer tube (302) about the proximal outer tube length (314) in a clockwise and counterclockwise direction is configured to rotate the distal outer tube (322) but neither the pull wire (356) nor the distal body (364).

4. The system of claim 1 wherein, when the clamp (392) is in the open position, the proximal outer tube (302) is configured to move proximally for a pre-determined distance relative to at least the inner tube (334) and the distal outer tube (322) in the lengthwise direction.

5. The system of claim 1 wherein, when the clamp (392) is in the open position, the proximal outer tube (302), the inner tube (334), and the distal outer tube (322) are freely slideable over the pull wire (356) in the proximal and distal directions.

6. The system of claim 1 wherein the system comprises a relaxed position in which the proximal outer tube distal end (312) abuts against the distal outer tube proximal end and in which the spring is the relaxed length and a second position in which the distal end of the proximal outer tube (302) does not abut the proximal end (328) of the distal outer tube and in which the spring (348) is the compressed length.

7. The system of claim 1 wherein the proximal outer tube exterior (304) comprises indicia (382A, 382B, 382C) adjacent to the slot (306).

8. The system of claim 1 wherein the proximal outer tube exterior (304) comprises two handle flanges (384) comprising grooves (386) located on opposite sides of the proximal outer tube exterior.

9. The system of claim 8 wherein the handle flanges (384) have a proximal end and a distal end and further wherein the handle flanges extend laterally from the proximal outer tube (302) to a greater extent at the proximal end of the handle flanges as compared to the distal end of the handle flanges.

10. The system of claim 1 wherein the inner tube (334) comprises an inner tube height and an inner tube width (410), and further wherein the inner tube distal end (344) comprises a tapered portion (412) in which the inner tube height and width decrease at the inner tube distal end.

11. The system of claim 10 wherein the distal outer tube interior (326) and the inner tube (334) comprise mating threads, wherein moving the inner tube threads (396) along the distal outer tube interior threads (394) in a first direction moves the clamp (392) to the closed position and further wherein moving the inner tube threads along the distal outer tube interior threads in a second direction moves the clamp (392) to the open position.

12. The system of claim 1 wherein the slot proximal end (316) is at the proximal end of the proximal outer tube (302).

13. The system of claim 1 wherein the inner tube is comprised of a rod (402) and a collar (404) enveloping the rod and further wherein the collar comprises the fin (340).

14. The system of claim 1 wherein the slot length (320) is less than the proximal outer tube length (314).

15. The system of claim 1 wherein the proximal outer tube distal end (312), the distal outer tube proximal end (328), and the inner tube distal end (344) are open and the pull wire (356) extends through at least the inner tube open distal end.

16. The system of claim 15 wherein the proximal outer tube interior (308) is hollow, the proximal outer tube proximal end (310) is open, the distal outer tube interior (326) is hollow, the inner tube interior (336) is hollow, the distal outer tube distal end (330) is open and the inner tube proximal end (342) is open.

17. The system of claim 16 wherein the pull wire proximal end (358) is proximal to the proximal outer tube open proximal end, and further wherein the pull wire extends through the inner tube hollow interior, the inner tube open proximal end, the distal outer tube open proximal end, the distal outer tube open distal end, the distal outer tube hollow interior, the proximal outer tube open distal end, the proximal outer tube hollow interior, and the proximal outer tube open proximal end.

## Patentansprüche

1. System (10; 200; 300) zum Entfernen eines Blutgerinnsels aus einem menschlichen Blutgefäß, wobei das System umfasst: ein proximales Außenrohr (302), das ein proximales Außenrohräußere (304), das einen Schlitz (306) umfasst, ein proximales Außenrohrinnere (308), ein proximales Ende (310) des proximalen Außenrohrs, ein distales Ende (312) des proximalen Außenrohrs und eine proximale Außenrohrlänge (314), die sich von dem proximalen Ende des proximalen Außenrohrs zu dem distalen Ende des proximalen Außenrohrs erstreckt, umfasst, wobei der Schlitz ein proximales Schlitzende (316), ein distales Schlitzende (318) und eine Schlitzlänge (320), die sich von dem proximalen Schlitzende zu dem distalen Schlitzende und parallel zu der proximalen Außenrohrlänge erstreckt, umfasst;
ein distales Außenrohr (322), das ein distales Außenrohräußeres (324), ein distales Außenrohrinneres (326), ein proximales Ende (328) des distalen Außenrohrs, das sich distal zu dem distalen Ende des proximalen Außenrohrs befindet, ein distales Ende (330) des distalen Außenrohrs, eine distale Außenrohrlänge (332), die sich von dem proximalen Ende des distalen Außenrohrs zu dem distalen Ende des distalen Außenrohrs erstreckt, umfasst, wobei das proximale Ende des distalen Außenrohrs nicht an dem distalen Ende des proximalen Außenrohrs befestigt ist;
ein Innenrohr (334), das ein Innenrohrinnere (336), ein Innenrohräußere (338), umfassend einen Steg (340), der in dem proximalen Außenrohrschlitz positioniert ist, ein proximales Innenrohrende (342), ein distales Innenrohrende (344) und eine Innenrohrlänge (346), die sich von dem proximalen Innenrohrende zu dem distalen Innenrohrende erstreckt, umfasst;
einen Zugdraht (356), der ein proximales Zugdrahtende (358) und ein distales Zugdrahtende (360), das sich distal zu dem distalen Ende des distalen Außenrohrs befindet, aufweist;
einen distalen Körper (364), der sich distal zu dem distalen Außenrohr befindet und ein distales Körperinneres (366), einen distalen Körperumfang (368), ein proximales Ende (370) des distalen Körpers, das mit dem Zugdraht verbunden ist, ein distales Ende (372) des distalen Körpers, eine distale Körperlänge (374), die sich von dem proximalen Ende des distalen Körpers zu dem distalen Ende des distalen Körpers erstreckt, und eine distale Körperhöhe und -breite (376), senkrecht zu der distalen Körperlänge, umfasst, wobei der distale Körper einen Rahmen (378) umfasst, der von einer Vielzahl von Formgedächtnismetallstreifen (380) gebildet wird, wobei der distale Körper einen entspannten Zustand, wobei der distale Körper eine erste Höhe und eine erste Breite aufweist, und einen eingeklappten Zustand, wobei der distale Körper eine zweite Höhe und eine zweite Breite aufweist, aufweist, wobei die zweite Höhe des distalen Körpers geringer als die erste Höhe des distalen Körpers ist, wobei die zweite Breite des distalen Körpers geringer als die erste Breite des distalen Körpers ist,
**dadurch gekennzeichnet, dass**:
das Innenrohr sich von dem proximalen Außenrohrinneren zu dem distalen Außenrohrinneren erstreckt;
das System eine komprimierbare Feder (348) umfasst, die ein proximales Federende (350), ein distales Federende (352) und eine Federlänge (354), die sich von dem proximalen Federende (350) zu dem distalen Federende erstreckt, aufweist; und
wobei mindestens eines des distalen Außenrohrs und des Innenrohrs eine Klemme (392) umfasst, die eine geschlossene Position, in der die Klemme um den Zugdraht geschlossen ist, und eine offene Position, in der die Klemme in Bezug auf den Zugdraht offen ist, aufweist,
wobei, wenn die Klemme in der offenen und geschlossenen Position ist, das Innenrohr an dem distalen Außenrohr befestigt ist und sich gemeinsam mit dem distalen Außenrohr in der Längsrichtung bewegt, wobei, zumindest wenn die Klemme in der geschlossenen Position ist, das proximale Außenrohr dazu konfiguriert ist, sich für eine vorgegebene Distanz relativ zu dem Innenrohr, dem distalen Außenrohr, dem Zugdraht und dem distalen Körper in der Längsrichtung proximal zu bewegen,
wobei, zumindest wenn die Klemme in der geschlossenen Position ist, das proximale Außenrohr relativ zu dem distalen Außenrohr, dem Innenrohr, dem Zugdraht und dem distalen Körper proximal zu bewegen, den proximalen Außenrohrschlitz veranlasst, sich relativ zu dem Steg proximal zu bewegen, und die Feder veranlasst, sich von einer entspannten Länge zu einer komprimierten Länge zu bewegen.

2. System nach Anspruch 1, wobei, wenn die Klemme (392) in der geschlossenen Position ist, das proximale Außenrohr (302) in einer Richtung im Uhrzeigersinn und gegen den Uhrzeigersinn um die proximale Außenrohrlänge (314) zu drehen, dazu konfiguriert ist, das distale Außenrohr (322), den Zugdraht (356) und den distalen Körper (364) zu drehen.

3. System nach Anspruch 2, wobei, wenn die Klemme (392) in der offenen Position ist, das proximale Außenrohr (302) in einer Richtung im Uhrzeigersinn und gegen den Uhrzeigersinn um die proximale Außenrohrlänge (314) zu drehen, dazu konfiguriert ist, das distale Außenrohr (322), aber weder den Zugdraht (356) noch den distalen Körper (364) zu drehen.

4. System nach Anspruch 1, wobei, wenn die Klemme (392) in der offenen Position ist, das proximale Außenrohr (302) dazu konfiguriert ist, sich für eine vorgegebene Distanz relativ zu zumindest dem Innenrohr (334) und dem distalen Außenrohr (322) in der Längsrichtung proximal zu bewegen.

5. System nach Anspruch 1, wobei, wenn die Klemme (392) in der offenen Position ist, das proximale Außenrohr (302), das Innenrohr (334) und das distale Außenrohr (322) in der proximalen und der distalen Richtung frei über den Zugdraht (356) schiebbar sind.

6. System nach Anspruch 1, wobei das System eine entspannte Position, in der das distale Ende (312) des proximalen Außenrohrs an dem proximalen Ende des distalen Außenrohrs anliegt und in der die Feder die entspannte Länge ist, und eine zweite Position, in der das distale Ende des proximalen Außenrohrs (302) nicht an dem proximalen Ende (328) des distalen Außenrohrs anliegt und in der die Feder (348) die komprimierte Länge ist, umfasst.

7. System nach Anspruch 1, wobei das proximale Außenrohräußere (304) Indizes (382A, 382B, 382C) umfasst, die an den Schlitz (306) angrenzen.

8. System nach Anspruch 1, wobei das proximale Außenrohräußere (304) zwei Griffflansche (384) umfasst, die Kerben (386) umfassen, die sich an entgegengesetzten Seiten des proximalen Außenrohräußeren befinden.

9. System nach Anspruch 8, wobei die Griffflansche (384) ein proximales Ende und ein distales Ende aufweisen und wobei weiter die Griffflansche sich seitlich von dem proximalen Außenrohr (302) zu einem größeren Ausmaß an dem proximalen Ende der Griffflansche verglichen zu dem distalen Ende der Griffflansche erstrecken.

10. System nach Anspruch 1, wobei das Innenrohr (334) eine Innenrohrhöhe und eine Innenrohrbreite (410) umfasst und wobei weiter das distale Innenrohrende (344) einen verjüngten Abschnitt (412) umfasst, in dem die Innenrohrhöhe und -breite bei dem distalen Innenrohrende abnehmen.

11. System nach Anspruch 10, wobei das distale Außenrohrinnere (326) und das Innenrohr (334) zusammenpassende Gewinde umfassen, wobei die Innenrohrgewinde (396) entlang der distalen Außenrohrinnengewinde (394) in einer ersten Richtung zu bewegen, die Klemme (392) zu der geschlossenen Position bewegt, und wobei weiter die Innenrohrgewinde entlang der distalen Außenrohrinnengewinde in einer zweiten Richtung zu bewegen, die Klemme (392) zu der offenen Position bewegt.

12. System nach Anspruch 1, wobei das proximale Schlitzende (316) an dem proximalen Ende des proximalen Außenrohrs (302) ist.

13. System nach Anspruch 1, wobei das Innenrohr einen Stab (402) und einen Kranz (404), der den Stab umschließt, umfasst, und wobei weiter der Kranz den Steg (340) umfasst.

14. System nach Anspruch 1, wobei die Schlitzlänge (320) geringer als die proximale Außenrohrlänge (314) ist.

15. System nach Anspruch 1, wobei das distale Ende (312) des proximalen Außenrohrs, das proximale Ende (328) des distalen Außenrohrs und das distale Innenrohrende (344) offen sind und der Zugdraht (356) sich durch zumindest das offene distale Innenrohrende erstreckt.

16. System nach Anspruch 15, wobei das proximale Außenrohrinnere (308) hohl ist, das proximale Ende (310) des proximalen Außenrohrs offen ist, das distale Außenrohrinnere (326) hohl ist, das Innenrohrinnere (336) hohl ist, das distale Ende (330) des distalen Außenrohrs offen ist und das proximale Innenrohrende (342) offen ist.

17. System nach Anspruch 16, wobei das proximale Zugdrahtende (358) proximal zu dem proximalen Ende des proximalen Außenrohrs ist und wobei weiter der Zugdraht sich durch das hohle Innenrohrinnere, das offene proximale Innenrohrende, das offene proximale Ende des distalen Außenrohrs, das offene distale Ende des distalen Außenrohrs, das hohle distale Außenrohrinnere, das offene distale Ende des proximalen Außenrohrs, das hohle proximale Außenrohrinnere und das offene proximale Ende des proximalen Außenrohrs erstreckt.

## Revendications

1. Système (10 ;200 : 300) pour retirer un caillot de sang d'un vaisseau sanguin humain, le système comprenant : un tube extérieur proximal (302) comprenant un extérieur de tube extérieur proximal (304) comprenant une fente (306), un intérieur de tube extérieur proximal (308), une extrémité proximale de tube extérieur proximal (310), une extrémité distale de tube extérieur proximal (312), et une longueur de tube extérieur proximal (314) s'étendant de l'extrémité proximale du tube extérieur proximal à l'extrémité distale du tube extérieur proximal, dans laquelle la fente comprend une extrémité proximale de la fente (316), une extrémité distale de la fente (318) et une longueur de la fente (320) s'étendant de l'extrémité proximale de la fente à l'extrémité distale de la fente et parallèle à la longueur du tube extérieur proximal ;
un tube extérieur distal (322) comprenant un extérieur de tube extérieur distal (324), un intérieur de tube extérieur distal (326), une extrémité proximale de tube extérieur distal (328) situé de façon distale à l'extrémité distale du tube extérieur proximal, une extrémité distale du tube extérieur distal (330), une longueur de tube extérieur distal (332) s'étendant de l'extrémité proximale du tube extérieur distal à l'extrémité distale du tube extérieur distal, l'extrémité proximale du tube extérieur distal n'étant pas fixée à l'extrémité distale du tube extérieur proximal ;
un tube intérieur (334) comprenant un intérieur de tube intérieur (336), un extérieur de tube intérieur (338) comprenant une ailette (340) positionnée dans la fente du tube extérieur proximal, une extrémité proximale de tube intérieur (342), une extrémité distale de tube intérieur (344) et une longueur de tube intérieur (346) s'étendant de l'extrémité proximale du tube intérieur à l'extrémité distale du tube intérieur ;
un fil de traction (356) présentant une extrémité proximale de fil de traction (358) et une extrémité distale de fil de traction (360) située de façon distale par rapport à l'extrémité distale du tube extérieur,
un corps distal (364) situé de façon distale par rapport au tube extérieur distal et comprenant un intérieur de corps distal (366), un périmètre de corps distal (368), une extrémité proximale de corps distal (370) reliée au fil de traction, une extrémité distale de corps distal (372), une longueur de corps distal (374) s'étendant de l'extrémité proximale de corps distal à l'extrémité distale de corps distal, et une hauteur et une largeur de corps distal (376) perpendiculaires à la longueur de corps distal, le corps distal comprenant une armature (378) formée par une pluralité de bandes de métal à mémoire (380), dans laquelle le corps distal présente un état détendu dans lequel le corps distal présente une première hauteur et une première largeur, et un état replié dans lequel le corps distal présente une seconde hauteur et une seconde largeur, la seconde hauteur du corps distal étant inférieure à la première hauteur du corps distal, la seconde largeur du corps distal étant inférieure à la première largeur du corps distal,
**caractérisé en ce que** :
le tube intérieur s'étend d'un intérieur de tube extérieur proximal à l'intérieur du tube extérieur distal ;
le système comprend un ressort compressible (348) présentant une extrémité proximale de ressort (350), une extrémité distale de ressort (352) et une longueur de ressort (354) s'étendant de l'extrémité proximale de ressort (350) à l'extrémité distale de ressort ; et
dans lequel au moins l'un du tube extérieur distal et du tube intérieur comprend une pince (392) présentant une position fermée dans laquelle la pince se referme autour du fil de traction et une position ouverte dans laquelle la pince est ouverte par rapport au fil de traction,
dans lequel, lorsque la pince est en position ouverte et fermée, le tube intérieur est fixé au tube extérieur distal et se déplace avec le tube extérieur distal dans le sens de la longueur, dans lequel, au moins lorsque la pince est en position fermée, le tube extérieur proximal est configuré pour se déplacer de façon proximale sur une distance prédéterminée par rapport au tube intérieur, au tube extérieur distal, au fil de traction et au corps distal dans le sens de la longueur,
dans lequel, au moins lorsque la pince est en position fermée, le déplacement de façon proximale du tube extérieur proximal par rapport au tube extérieur distal, au tube intérieur, au fil de traction et au corps distal entraîne le déplacement de façon proximale de la fente du tube extérieur proximal par rapport à l'ailette et fait passer le ressort d'une longueur détendue à une longueur comprimée.

2. Système selon la revendication 1, dans lequel, lorsque la pince (392) est en position fermée, la rotation du tube extérieur proximal (302) autour de la longueur du tube extérieur proximal (314) dans le sens horaire et dans le sens anti-horaire est configurée pour faire tourner le tube extérieur distal (322), le fil de traction (356) et le corps distal (364).

3. Système selon la revendication 2, dans lequel, lorsque la pince (392) est en position ouverte, la rotation du tube extérieur proximal (302) autour de la longueur du tube extérieur proximal (314) dans le sens horaire et dans le sens anti-horaire est configurée pour faire tourner le tube extérieur distal (322), mais ni le fil de traction (356), ni le corps distal (364).

4. Système selon la revendication 1, dans lequel, lorsque la pince (392) est en position ouverte, le tube extérieur proximal (302) est configuré pour se déplacer de façon proximale sur une distance prédéterminée par rapport à au moins le tube intérieur (334) et le tube extérieur distal (322) dans le sens de la longueur.

5. Système selon la revendication 1, dans lequel, lorsque la pince (392) est en position ouverte, le tube extérieur proximal (302), le tube intérieur (334), et le tube extérieur distal (322) peuvent coulisser librement sur le fil de traction (356) dans les directions proximale et distale.

6. Système selon la revendication 1, dans lequel le système comprend une position détendue dans laquelle l'extrémité distale du tube extérieur proximal (312) est en butée contre l'extrémité proximale du tube extérieur distal et dans laquelle le ressort est de longueur détendue et une seconde position dans laquelle l'extrémité distale du tube extérieur proximal (302) n'est pas en butée contre l'extrémité proximale (328) du tube extérieur distal et dans laquelle le ressort (348) est de la longueur comprimée.

7. Système selon la revendication 1 dans lequel l'extérieur du tube externe proximal (304) comprend des repères (382A, 382B, 382C) adjacents à la fente (306).

8. Système selon la revendication 1 dans lequel l'extérieur du tube extérieur proximal (304) comprend deux brides de poignée (384) comprenant des rainures (386) situées sur les côtés opposés de l'extérieur du tube extérieur proximal.

9. Système selon la revendication 8, dans lequel les brides de poignée (384) présentent une extrémité proximale et une extrémité distale et dans lequel en outre les brides de poignée s'étendent latéralement à partir du tube extérieur proximal (302) dans une plus grande mesure à l'extrémité proximale des brides de poignée par rapport à l'extrémité distale des brides de poignée.

10. Système selon la revendication 1, dans lequel le tube intérieur (334) comprend une hauteur de tube intérieur et une largeur de tube intérieur (410), et dans lequel en outre l'extrémité distale du tube intérieur (344) comprend une partie conique (412) dans laquelle la hauteur et la largeur du tube intérieur diminuent à l'extrémité distale du tube intérieur.

11. Système selon la revendication 10 dans lequel l'intérieur du tube extérieur distal (326) et le tube intérieur (334) comprennent des filets de raccordement, dans lequel le déplacement des filets du tube intérieur (396) le long des filets intérieurs du tube extérieur distal (394) dans une première direction déplace la pince (392) en position fermée et dans lequel en outre le déplacement des filets du tube intérieur le long des filets intérieurs du tube extérieur distal dans une seconde direction déplace la pince (392) en position ouverte.

12. Système selon la revendication 1, dans lequel l'extrémité proximale de la fente (316) se trouve à l'extrémité proximale du tube extérieur proximal (302).

13. Système selon la revendication 1, dans lequel le tube intérieur est composé d'une tige (402) et d'un collier (404) enveloppant la tige et dans lequel le collier comprend l'ailette (340).

14. Système selon la revendication 1, dans lequel la longueur de la fente (320) est inférieure à la longueur du tube extérieur proximal (314).

15. Système selon la revendication 1, dans lequel l'extrémité distale du tube extérieur proximal (312), l'extrémité proximale du tube extérieur distal (328) et l'extrémité distale du tube intérieur (344) sont ouvertes et le fil de traction (356) s'étend à travers au moins l'extrémité distale ouverte du tube intérieur.

16. Système selon la revendication 15, dans lequel l'intérieur du tube extérieur proximal (308) est creux, l'extrémité proximale du tube extérieur proximal (310) est ouverte, l'intérieur du tube extérieur distal (326) est creux, l'intérieur du tube intérieur (336) est creux, l'extrémité distale du tube extérieur distal (330) est ouverte et l'extrémité proximale du tube intérieur (342) est ouverte.

17. Système selon la revendication 16, dans lequel l'extrémité proximale du fil de traction (358) est proximale à l'extrémité proximale ouverte du tube extérieur, et dans lequel en outre le fil de traction s'étend à travers l'intérieur creux du tube intérieur, l'extrémité proximale ouverte du tube intérieur, l'extrémité proximale ouverte du tube extérieur distal, l'extrémité distale ouverte du tube extérieur distal, l'intérieur creux du tube extérieur distal, l'extrémité distale ouverte du tube extérieur proximal, l'intérieur creux du tube extérieur proximal, et l'extrémité proximale ouverte du tube extérieur proximal.
